# EUROPÄISCHE PATENTANMELDUNG

(11) **EP 2 168 979 A1**
(43) Veröffentlichungstag der Anmeldung: **31.03.2010**
(21) Anmeldenummer: 08164921.2
(22) Anmeldetag: 23.09.2008
(51) Int. Cl.: C07K 14/435

(54) **Verfahren zur Isolierung neuraler Zellen mit Tenascin-R-Verbindungen**

(71) Anmelder: Rheinische Friedrich-Wilhelms-Universität Bonn, 53113 Bonn (DE)
(72) Erfinder: Pesheva, Penka Prof. Dr., 53227 Bonn (DE); Probstmeier, Rainer Dr., 53227 Bonn (DE)
(74) Vertreter: Helbing, Jörg

(57) **Zusammenfassung**

Die Erfindung betrifft ein Verfahren zur Isolierung von neuralen Zellen unter Verwendung von Tenascin-R-Verbindungen, besonders für dieses Verfahren geeignete Tenascin-R-Fragmente und Tenascin-R-Fusionsproteine, die rekombinante Herstellung dieser Tenascin-R-Verbindungen, sowie einen Kit zur Durchführung dieses Verfahrens und die Verwendung des Verfahrens zur Herstellung hochreiner neuraler Zellpopulationen. Die Erfindung betrifft weiterhin Antikörper, die für die Detektion und Isolation von Tenascin-R-Verbindungen geeignet sind.

## Beschreibung

Die Erfindung betrifft ein Verfahren zur Isolierung von neuralen Zellen unter Verwendung von Tenascin-R-Verbindungen, besonders für dieses Verfahren geeignete Tenascin-R-Fragmente und Tenascin-R-Fusionsproteine, die rekombinante Herstellung dieser Tenascin-R-Verbindungen, sowie einen Kit zur Durchführung dieses Verfahrens und die Verwendung des Verfahrens zur Herstellung hochreiner neuraler Zellpopulationen. Die Erfindung betrifft weiterhin Antikörper, die für die Detektion und Isolation von Tenascin-R-Verbindungen geeignet sind.

### Hintergrund der Erfindung

Bei der Kontaktaufnahme von Zellen mit dem sie umgebenden extrazellulären Milieu kann es zu vielfältigen Antworten der Zelle kommen, die von Abstoßung und Vermeidung des Milieus auf der einen bis zur stabilen Zelladhäsion auf der anderen Seite reichen können. Das Wechselspiel zwischen Komponenten der extrazellulären Umgebung, der Extrazellulärmatrix, einerseits, und auf der Zelloberfläche vorhandenen Rezeptoren für solche Komponenten andererseits, bildet die Grundlage für viele entwicklungsbiologische Prozesse, die für eine Zelle unter anderem durch Proliferations-, Migrations- oder Differenzierungsverhalten gekennzeichnet sind. Solch zelluläres Verhalten führt auf Organismenebene zur Musterbildung oder auf Gewebe- oder Organebene zur Neubildung als Folge einer Verletzung (Boudreau, N. J., Jones, P. L., Biochem. J. 339:481-488 (1999); Sobeih, M. M., Corfas, G., Int. J. Dev. Neurosci. 148:971-84 (2002); Schmid, R. S., Anton, E. S., Cereb. Cortex. 13:219-24 (2003)). Im Zentralnervensystem (ZNS) korreliert die regulierte Expression von Extrazellulärmatrixkomponenten wie Chondroitinsulfat-Proteoglykanen oder Proteinen der Tenascin-Familie mit biologischen Prozessen, welche sowohl die Adhäsion und Migration von Neuronen, die Navigation von Axonen, die Synapsenbildung und -plastizität, die Wirkung von Wachstumsfaktoren und Cytokinen als auch das Überleben von Neuronen und die strukturelle Organisation der Extrazellulärmatrix umfassen (Dow, K. E., Wang, W., Cell Mol. Life Sci. 54:567-81 (1998); Wright, J. W. et al., Peptides 23:221-46 (2002); Grimpe, B., Silver, J., Prog. Brain Res. 137:333-49 (2002); Bosman, F. T., Stamenkovic, I., J. Pathol. 200:423-8 (2003)).

Tenascin-R (TN-R) (ehemals als J1-160/180, Janusin oder Restrictin bezeichnet) ist ein Mitglied der Tenascin-Familie von Extrazellulärmatrixproteinen, das exklusiv im ZNS von Vertebraten auftritt und dort von Oligodendrozyten und manchen Gruppen von Neuronen, i.e. Motoneurone und Interneurone, während späterer Entwicklungsstadien und im erwachsenen Zustand exprimiert wird (Pesheva, P., Probstmeier, R., Prog. Neurobiol. 61:465-93 (2000); Scherberich, A. et al., J. Cell Sci. 117-571-81 (2004)). Das Protein tritt in zwei molekularen Formen auf, mit Molekulargewichten von 160 kD (TN-R 160) oder 180 kD (TN-R 180). TN-R findet sich im Gewebe primär in Assoziation mit Oligodendrozyten, myelinisierten Axonen, perineuronalen Netzen von Moto- und Interneuronen, sowie dendriten- und synapsenreichen Regionen.

TN-R ist aus vier unterschiedlichen Domänenstrukturen aufgebaut. Der N-Terminus, dessen Sequenz nur in Tenascin-Proteinen vorkommt, enthält ein Cystein-reiches Segment (Cys-rich), und wird gefolgt von viereinhalb EGF-ähnlichen Segmenten (EGF-like) sowie 9 Fibronektin Typ III (FN III)-ähnlichen Domänen (von denen die 6. Domäne alternativ gespleisst werden kann). Den C-Terminus des TN-R Proteins bildet eine globuläre, Fibrinogen-ähnliche Domäne (FNG). Einzelne TN-R Polypeptidketten sind an ihren N-Termini über Disulfidbrücken miteinander verbunden und bilden so Homotrimere (TN-R 180) oder -dimere (TN-R 160), wobei letztere durch proteolytische Spaltung von TN-R 180 nahe des N-Terminus entstehen (Woodworth, A. et al., J. Biol. Chem. 279:10413-21 (2004)).

Die Funktionsweite von TN-R umfasst die molekulare Kontrolle neuraler Zelladhäsion, Migration und Differenzierung (von der Axonnavigation Fortsätze bildender Neuronen bis zur Reifung myelinbildender Oligodendrozyten) während normaler entwicklungsbiologischer Vorgänge sowie regenerativer Prozesse nach Verletzung im erwachsenen Gehirn (Pesheva, P., Probstmeier, R., Prog. Neurobiol. 61:465-93 (2000); Chiquet-Ehrismann, R., Int. J. Biochem. Cell. Biol. 36:986-90 (2004)). TN-R wirkt u.a. als adhäsives oder antiadhäsives Molekül, als Differenzierungsfaktor für Oligodendrozyten, oder als "Stopmolekül" für wachsende Axone. Diese Eigenschaften sind von den entsprechenden Rahmenbedingungen abhängig: dem jeweiligen Zelltyp, dem Vorhandensein zellulärer Rezeptoren und Signalkaskaden, der räumlichen Verteilung und der posttranslationalen Modifikation des TN-R Glykoproteins. Einige der zellulären Rezeptoren von TN-R, die identifiziert wurden (F3/F11, Disialoganglioside, Sulfatide), induzieren unterschiedliche zelluläre Wirkmechanismen, die einerseits bei der Musterbildung während der Ontogenese, andererseits bei regenerativen Prozessen von Bedeutung sind (Angelov, D. et al., J. Neurosci. 18:6218-29 (1998); Probstmeier, R. et al., J. Neurosci. Res. 60:21-36 (2000); Montag-Sallaz, M., Montag, D., Genes Brain Behav. 2:20-31 (2003); Saghatelyan, A. et al., Nat. Neurosci. 7:347-56 (2004); Brenneke, F. et al., Epilepsy Res. 58:133-43 (2004)). Die beiden wesentlichen Effekte von TN-R auf das Verhalten neuraler Zellen sind die Inhibition der Zelladhäsion und des Neuritenwachstums einerseits und die Förderung der Oligodendrozytenadhäsion und -differenzierung andererseits. Letztere erfolgt vermittelt durch Sulfatide und bewirkt letztendlich Oligodendrozytenmigration und Myelinisierung/Remyelinisierung. Erstere kann entweder substratunabhängig erfolgen (vermittelt durch F3/F11 und andere, noch unbekannte Faktoren), oder Substrat/Integrin-abhängig (vermittelt durch Fibronektin und GD2/GD3). Die inhibitorische Wirkung von TN-R hat letztlich einen Einfluss auf die neurale Zellmigration, das räumlich koordinierte Axonwachstum und die Synaptogenese, oder sie trägt unter Verletzungsbedingungen zur Verhinderung der Axon-Regeneration und der Adhäsion aktivierter Mikroglia in einer TN-R-reichen Umgebung bei.

Einige neurale Rezeptoren und intrazelluläre Signalwege, die die Wirkung von TN-R in neuronalen und glialen Zellen vermitteln, sind bekannt, ebenso konnten molekulare Komponenten identifiziert werden, die an der Expression von TN-R durch Oligodendrozyten und Motoneurone beteiligt sind (Tab. 1; Pesheva, P. et al., Prog. Brain Res. 132:103-14 (2001)).

**Tab.1: Für TN-R identifizierte zelluläre Rezeptoren und Liganden der Extrazellulärmatrix. In der rechten Tabellenhälfte sind die für die jeweilige Interaktion relevanten Domänen von TN-R angegeben; CS GAG: Chondroitinsulfat-Glykosaminoglykane; EGF-L: EGF-ähnliche Segmente und Cystein-reiches Segment.**

| Zelluläre Rezeptoren | Tenascin-R Bindungsdomäne | | Liganden der Extrazellulärmatrix | Tenascin-R Bindungsdomäne |
|---|---|---|---|---|
| F3/F11 | EGF-L, FN2-3 | | Fibronektin | CS GAG, unbekannt |
| β2 Untereinheit von Na-Kanälen | FN1-2, FN6-8 | | Collagene | Unbekannt |
| Neurofascin | FN2-5 | | Tenascin-C | CS GAG, Ca²⁺-abhängig |
| CALEB | FNG | | Tenascin-R | Unbekannt, Kation²⁺-abhängig |
| MAG | EGF-L, FNG | | Lecticane | FN3-5, Ca²⁺-abhängig |
| Sulfatide | Unbekannt | | Phosphacan | EGF-L, Ca²⁺-abhängig |
| Ganglioside | Unbekannt | | | |

Als aus mehreren Domänen aufgebautes Extrazellulärmatrixprotein erscheint eine Aufteilung der distinkten biologischen Funktionen auf distinkte Domänenbereiche und/oder distinkte Glykostrukturen von TN-R nahe liegend. Ähnlich wie die anderen bekannten TN-R Proteine, setzt sich das humane TN-R Protein aus verschiedenen distinkten Domänen zusammen (Carnemolla et al., 1996). Beginnend mit einer Cystein-reichen Region am N-Terminus, gefolgt von 4.5 EGF-ähnlichen Domänen und 9 FN III-ähnlichen Domänen (von denen die 6. Domäne alternativ gespleisst werden kann), endet das Molekül am C-Terminus mit einer Fibrinogen-ähnlichen Domäne. Die publizierte humane TN-R mRNA-Sequenz umfasst mit 9 FN III-ähnlichen Domänen 4716 Basen. Hiervon entfällt auf den kodierenden Bereich der Abschnitt zwischen den Basen 82 bis 4158, auf das Signalpeptid (zur Sekretion des TN-R Proteins) der Basenbereich 82 bis 150 (SEQ ID NO:2).

Es ist bekannt, dass aus adultem Nagergehirn aufgereinigte TN-R Proteine die Adhäsion und Fortsatzbildung von aus früh postnatalem Gehirn isolierten O4/Sulfatid-positiven Oligodendrozyten fördern (Pesheva, P. et al., J. Neurosci. 17:4642-51 (1997)). Diese Vorgänge werden durch Sulfatide vermittelt, einer Gruppe von in der Zellmembran von Oligodendrozyten vorkommenden Glykolipiden. Eine wichtige Konsequenz der Interaktion von TN-R mit Sulfatid-exprimierenden Oligodendrozyten ist eine Stimulierung der Reifung dieser Zellen; i.e. die vermehrte Expression myelinspezifischer Proteine und Glykolipide, was eine Sulfatid-vermittelte Wirkungsweise des Differenzierungspotentials von TN-R auf Oligodendrozyten nahe legt (Pesheva, P. et al., J. Neurosci. 17:4642-51 (1997)).

EP 0759987, US 5,635,360, US 5,681,931 und US 5,591,583 beschreiben humanes TN-R und dessen immunologischen Nachweis unter Verwendung von Antikörpern gegen ein Proteinfragment, welches den Nukleotiden 2686-3165 der cDNA-Sequenz und somit den FN III-Domänen 6 und 7 des humanen TN-R entspricht.

Die frühesten Stadien sich entwickelnder Gliazellen finden sich in Säugern im Rückenmark in ventralen Bereichen des Neuralrohrs, im Gehirn in den ventrikulären Zonen des Vorderhirns. In diesen Regionen proliferieren erste Vorläuferzellen von Oligodendrozyten, die durch eine einfache Morphologie und die Expression des Disialogangliosids GD3 und/oder von 04 Antigenen gekennzeichnet sind, und wandern in der Folgezeit in Bereiche der später entstehenden weißen Substanz ein (Miller, R. H. Prog. Neurobiol. 67:451-67 (2002); Noble, M. et al., Dev. Biol. 265:33-52 (2004); Liu, Y. Rao, M. S., Biol. Cell. 96:279-90 (2004)). Dort werden die Zellen postmitotisch und differenzieren in reife Oligodendrozyten mit einer komplexen Morphologie. Dieser Reifungsprozess korreliert mit der Expression myelinspezifischer Lipide (Sulfatide und Galactocerebroside) und Proteine (MBP, MAG, und PLP). Die Entstehung, das Überleben und die Differenzierung von Oligodendrozyten in myelinbildende Zellen werden über verschiedene Wachstumsfaktoren (bFGF, PDGF, CNTF, IGF-1, NT-3), Hormone und Extrazellulärmatrixmoleküle (Thyroidhormone, Retinolsäure, TN-R) reguliert (Dubois-Dalcq, M., Murray, K., Pathol. Biol. (Paris) 48:80-6 (2000); Kagawa, T. et al., Microsc. Res. Tech. 52:740-5 (2001); Noble, M. et al., Dev. Neurosci. 25:217-33 (2003)).

Da insbesondere für gliale Zellen (wie Oligodendrozyten und neurale Stammzellen) keine adäquaten Modellsysteme in Gestalt von Zelllinien vorhanden sind, muss zu ihrer Gewinnung sowohl in der Grundlagenforschung als auch im Rahmen potentieller diagnostischer/therapeutischer Anwendungsbereiche bisher auf relativ zeitaufwendige und niedrig effiziente Anreicherungsverfahren von Primärzellen zurückgegriffen werden. Diese Verfahren ermöglichen außerdem oftmals nur die Herstellung angereicherter Mischzellpopulationen. Die bisherigen Verfahren zur Isolierung definierter Zellpopulationen nutzen unterschiedliche Techniken wie Dichtegradientenzentrifugationen oder immunologische Verfahren (Fluorescenceactivated cell sorting, biomagnetic cell sorting, Antikörper- und Komplementvermittelte Zelltötung, Antikörper-Panning) (Luxembourg, A. T. et al., Nat. Biotechnol. 16:281-5 (1998); Uchida, N. et al., Proc. Natl. Acad. Sci. 97:14720-5 (2000); Nistri, S. et al., Biol. Proced. Online 4:32-37 (2002); Nunes, M. C. et al., Nat. Med. 9:439-47 (2003); Vroemen, M., Weidner, N., J. Neurosci. Methods 124:135-43 (2003)). Solche Verfahren sind nicht sehr effizient und ergeben nur eine unvollständige Anreicherung (durch Dichtegradientenzentrifugationen sind z. B. nur Anreicherungen distinkter Zellpopulationen möglich), oder sie sind zeitintensiv und/oder von hohen Zellverlusten begleitet (wie bei immunologischen Verfahren). Dies gilt insbesondere für Oligodendrozyten, die bisher durch eine mehrwöchige Kultivierung gemischter glialer Kulturen (McCarthy, K. D., DeVellis, J., J. Cell Biol. 85:890-902 (1980); Kramer, E.M. et al., J. Biol. Chem. 274:29042-9 (1999); Testai, F. D. et al., J. Neurosci. Res. 75:66-74 (2004)) oder über mehrere Selektionsschritte mit Hilfe von Fluorescence-activated/biomagnetic cell sorting oder Antikörper-Panning gewonnen werden (Scarlato, M. et al., J. Neurosci. Res. 59:430-5 (2000); Tang, D. G. et al., J. Cell Biol. 148:971-84 (2000); Diers-Fenger, M. et al., Glia 34:213-28 (2001); Crang, A. J. et al., Eur. J. Neurosci. 20:1445-60 (2004)).

In WO2006/067094 sind gereinigte TN-R Proteine die stabile Adhäsion von Oligodendrozyten unterschiedlicher Reifestadien unterstützen und auf neuronale und mikrogliale Zellen antiadhäsiv wirken beschrieben. Dieses ermöglicht die Isolierung und Reinigung definierter Zellpopulationen aus neuralem Primärgewebe, insbesondere zur direkten selektiven Aufreinigung von Oligodendrozyten aus gemischten neuralen Zellpopulationen. Es wird hierin ein Verfahren zur Verfügung gestellt, das es ermöglicht, in einem einzigen Reinigungsschritt eine hochreine definierte Zellpopulation, insbesondere eine Oligodendrozytenpopulation, aus Primärgewebe neuralen Ursprungs zu isolieren. Die Aufreinigung der Zellen erfolgt dabei über einen Selektionsschritt, in dem aus einer Einzelzellsuspension mittels einer Tenascin-R-haltigen Sonde, welche Tenascin-R-Verbindungen ausgewählt aus nativem Tenascin-R (nachfolgen kurz "TN-R") sowie Homologen und Fragmenten desselben und Fusionsproteine derartiger Verbindungen umfasst. Ein bevorzugtes Tenascin-R-Fragment enthält dabei den C-Terminus von nativem Tenascin-R oder eine Substitutions-, Deletions- und/oder Additionsmutante desselben, besonders bevorzugt denjenigen Bereich, der durch die Nukleotide 3439-4155 der SEQ ID NO:1 kodiert wird.

Es wurde nunmehr gefunden, dass ein Fragment, das die Aminosäurereste 24 bis 189 der SEQ ID NO:2 des humanen TN-R umfasst, oder ein Homolog oder Fragment desselben wie z. B. das Fragment H-TNR-Cys, das Aminosäurereste 24 bis 189 der SEQ ID NO:2 aufweist, ebenso gut wie das in der WO WO2006/067094 beschriebene H-TNR-S3 Fragment mit den Aminosäureresten 1120 bis 1358 von SEQ ID NO:2 zur Isolierung hochreiner glialer, insbesondere oligodendroglialer, Zellpopulationen in einem Einstufenverfahren angewendet werden. Im Gegensatz zu H-TNR-S3, bindet das H-TNR-Cys Fragment zusätzlich Sphingomyelin (ein in der Zellmembran verschiedener Zelltypen vorkommendes und an zellulären Signalmechanismen beteiligtes Phospholipid) und unterstützt das Überleben und die Transdifferenzierung adulter humaner mesenchymaler Stammzellen in neuronale oder oligodendrogliale Zellen.

Die vorliegende Erfindung betrifft somit
(1) ein Verfahren zur Isolierung und Reinigung von neuralen Zellen aus neuralem Primärgewebe von Vertebraten, umfassend die Selektion der Zellen aus einer Einzelzellsuspension mittels einer Tenascin-R-haltigen Sonde ("Tenascin-R-Sonde"), welche ein N-terminales Fragment von nativem Tenascin-R (TN-R) sowie Homologen und Fragmenten desselben und Fusionsproteine derartiger Verbindungen umfasst;
(2) eine bevorzugte Ausführungsform von (1), wobei das Tenascin-R-Fragment ein Peptid mit der Sequenz der Aminosäurereste 51-216 von SEQ ID NO:2 ist und das Überleben und/oder die Transdifferenzierung adulter humaner mesenchymaler Stammzellen in neuronale oder oligodendrogliale Zellen, insbesondere unter definierten serumfreien Kulturbedingungen ermöglicht;
(3) ein Tenascin-R-Fragment oder ein Tenascin-R-Fusionsprotein wie in (1) oder (2) definiert;
(4) eine DNA, die ein Tenascin-R-Fragment oder ein Tenascin-R-Fusionsprotein von (3) kodiert;
(5) einen Vektor, der eine DNA nach (4) umfasst;
(6) einen Wirtsorganismus, der mit einem Vektor nach (5) transformiert/transfiziert ist und/oder eine DNA nach (4) aufweist;
(7) ein Verfahren zur Herstellung eines Tenascin-R-Fragments oder Tenascin-R-Fusionsproteins nach (3), umfassend das Kultivieren des Wirtsorganismus nach (6);
(8) einen Antikörper, insbesondere ein monoklonaler Antikörper, der durch Immunisierung eines geeigneten Wirtsorganismus mit einem Tenascin-R-Fragment nach (3) erhältlich ist;
(9) eine Zelllinie oder Hybridomzelllinie, die einen monoklonalen Antikörper nach (8) produziert;
(10) ein Kit zur Isolierung und Reinigung von neuralen Zellen, insbesondere von Oligodendrozyten, nach dem Verfahren von (1) oder (2), insbesondere enthaltend
   (i) eine wie in (1) oder (2) definierte Tenascin-R-Sonde, und/oder
   (ii) einen Vektor, der für die in (i) definierte Tenascin-R-Sonde kodiert, und/oder
   (iii) eine Stammkultur einer Zelllinie, die dazu geeignet ist, die wie in (1) oder (2) definierte Tenascin-R-Sonde zu exprimieren;
(11) die Verwendung eines Tenascin-R-Fragments oder eines Tenascin-R-Fusionsproteins wie in (3) definiert, zur Gewinnung von neuralen Zellen, insbesondere von Oligodendrozyten, für die Anzucht differenzierter Zellen, insbesondere neuraler Zellen, in neurobiologischen und zellphysiologischen Untersuchungen, in der biologischen und klinischen Forschung und für diagnostische und therapeutische Verfahren *in vitro* und *in vivo,* insbesondere für die Herstellung eines Medikaments zur Zelltherapie und zur Therapie von neurodegenerativen Krankheiten, die mit einem Verlust von Oligodendrozyten oder Myelin einhergehen, insbesondere Multipler Sklerose und periventrikulärer Leukomalazie (PVL);
(12) ein Verfahren zur Herstellung von Oligodendrozyten aus isolierten Stammzellen *in vitro* durch Inkubation der Stammzellen in Anwesenheit eines Tenascin-R-Fragments oder eines Tenascin-R-Fusionsproteins wie in (3) definiert;
(13) ein Verfahren zur Zelltherapie oder zur Therapie von neurodegenerativen Krankheiten, die mit einem Verlust von Oligodendrozyten oder Myelin einhergehen, insbesondere von Multipler Sklerose und periventrikulärer Leukomalazie (PVL), umfassend die Gabe eines Tenascin-R-Fragments oder eines Tenascin-R-Fusionsproteins wie in (3) definiert, an einen menschlichen oder tierischen Patienten;
(14) ein Verfahren zur Therapie und Prophylaxe traumatischer Nervenläsionen oder zur gezielten Beeinflussung der Neuralentwicklung, umfassend die Verabreichung einer pharmakologisch ausreichenden Menge des Antikörpers nach (8) an einen menschlichen oder tierischen Patienten, der einer solchen Behandlung bedarf.

### Kurzbeschreibung der Figuren

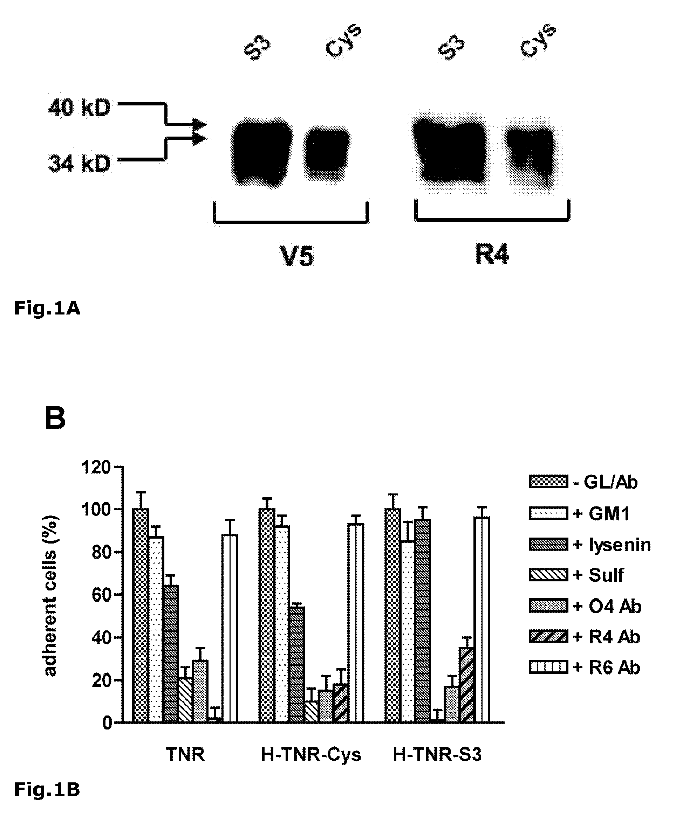

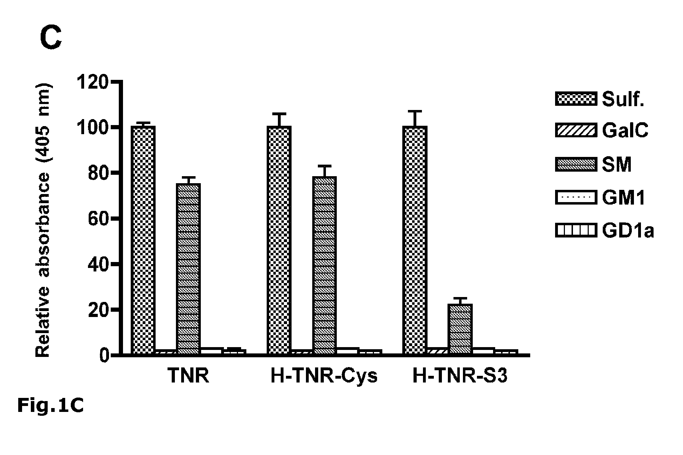

Fig. 1: Charakterisierung der Spezifität und funktionellen Aktivität des monoklonalen Antikörpers R4.
A) Westernblot-Analyse affinitätsgereinigter H-TNR-S3 und H-TNR-Cys Fragmente mit Tag-spezifischen (V5) und TN-R-spezifischen (R4) monoklonalen Antikörpern. Die über Nickel-Agarose gereinigten rekombinanten Fragmente (20 ng/Tasche) H-TNR-S3 (S3) und H-TNR-Cys (Cys) wurden gelelektrophoretisch aufgetrennt, auf Nitrozellulosefilter transferiert und mit V5 oder R4 Antikörper inkubiert. Immunreaktive Proteinbanden wurden durch Inkubation mit Peroxidase-gekoppelten anti-Maus IgG Antikörpern nachgewiesen. Pfeile zeigen das Molekulargewicht von H-TNR-S3 (40 kD), bzw. von H-TNR-Cys (34 kD).
B) Einfluss von polaren Glykolipiden, Lysenin (Sphingomyelin-bindenden Protein), 04- und TN-R-spezifischen Antikörpern auf die TN-R-vermittelte Zelladhäsion. TN-R Substrate (TNR, H-TNR-Cys, H-TNR-S3) wurden in der Abwesenheit (- GL/Ab) oder Anwesenheit von Monosialogangliosiden (+ GM1), Sulfatiden (+ Sulf) und TN-R-spezifischen Antikörpern (R4, R6) vorinkubiert. Erythrozyten wurden in Ab- oder Anwesenheit von Lysenin (100 µg/ml) und 04 Antikörpern (+ 04 Ab) auf die entsprechende Substrate ausgesät. Die Zahl adhärenter Zellen nach einstündiger Inkubationszeit auf unbehandelten TN-R Substraten (- GL/Ab) wurde als 100% gesetzt.
C) Interaktion R4 Epitop-tragender TN-R/TN-R Fragmente mit polaren Glykolipiden im Festphasen-Bindungsassay. Mikrotiterplatten wurden mit Sulfatiden (Sulf), Galactocerebrosiden (GalC), Sphingomyelin (SM) oder Sialogangliosiden (GM1, GD1a; alle bei 100 pmol/Vertiefung) beschichtet und mit TN-R oder rekombinanten TN-R Fragmenten (H-TNR-Cys, H-TNR-S3) für 2 h bei RT inkubiert. Die Bindung von TN-R/TN-R Fragmenten wurde mit R6 (für TN-R) oder V5 Antikörper (nach 1,5 Stunden Inkubation bei RT) mit Peroxidase-gekoppelten anti-Maus IgG Antikörpern nachgewiesen. Die maximale Bindung auf Sulfatide wurde für das jeweilige Protein als 100% gesetzt.

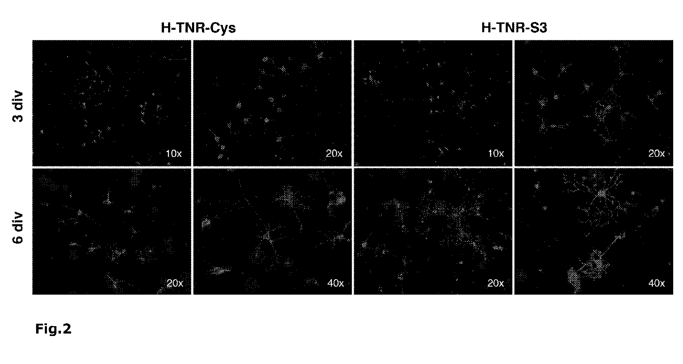

Fig. 2: Selektion von Oligodendrozyten aus Einzelzellsuspensionen (aus Gehirngewebe von 2 Tage alten Mäusen) auf substratgebundenen TN-R Fragmenten (H-TNR-Cys, H-TNR-S3). Auf diesen Substraten wachsende Zellen wurden für 3 Tage in serumfreiem Wachstumsmedium (3 div, obere Abbildungen) und für weitere 3 Tage (6 div, untere Abbildungen) in serumfreiem Differenzierungsmedium kultiviert. Adhärente Zellen konnten mit Hilfe indirekter Immunfluoreszenzfärbung mit Sulfatid-spezifischem Antikörper (04) als Oligodendrozyten identifiziert werden. 99±1% der isolierten Zellen waren 04-positiv. Übersichts- (10x) und Detailaufnahmen (20x und 40x) zeigen O4-positive Zellen auf den verschiedenen Substraten nach unterschiedlichen Kulturzeiten.

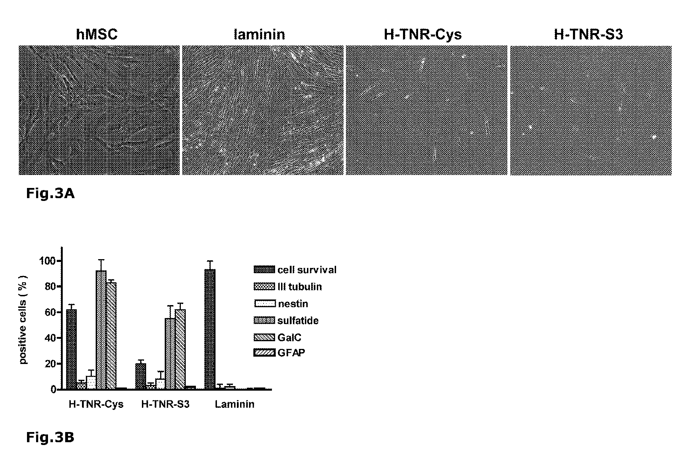

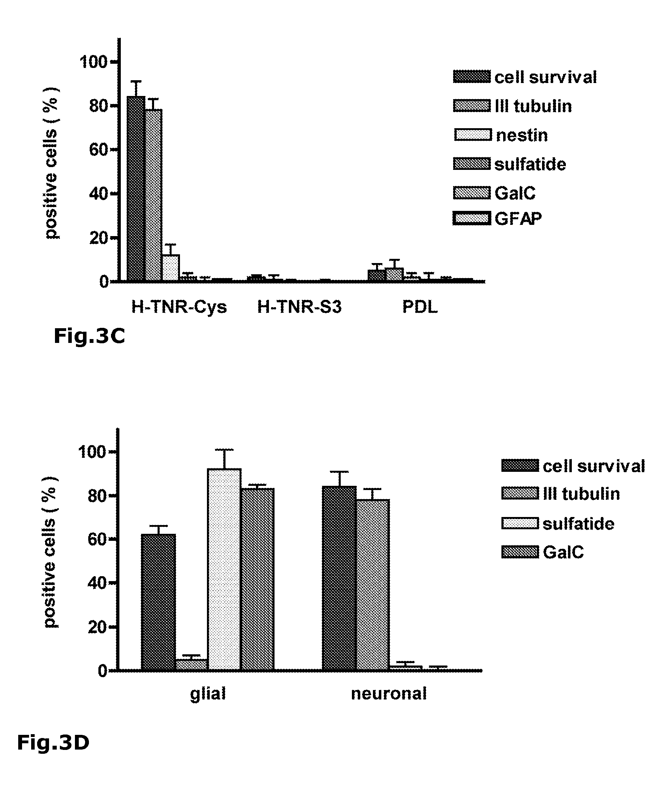

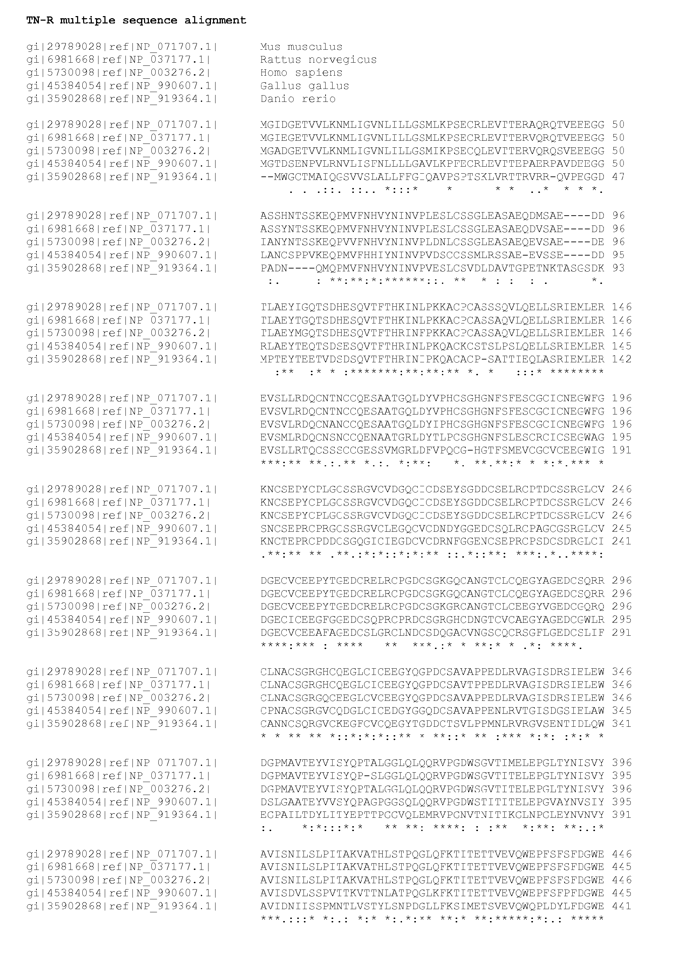

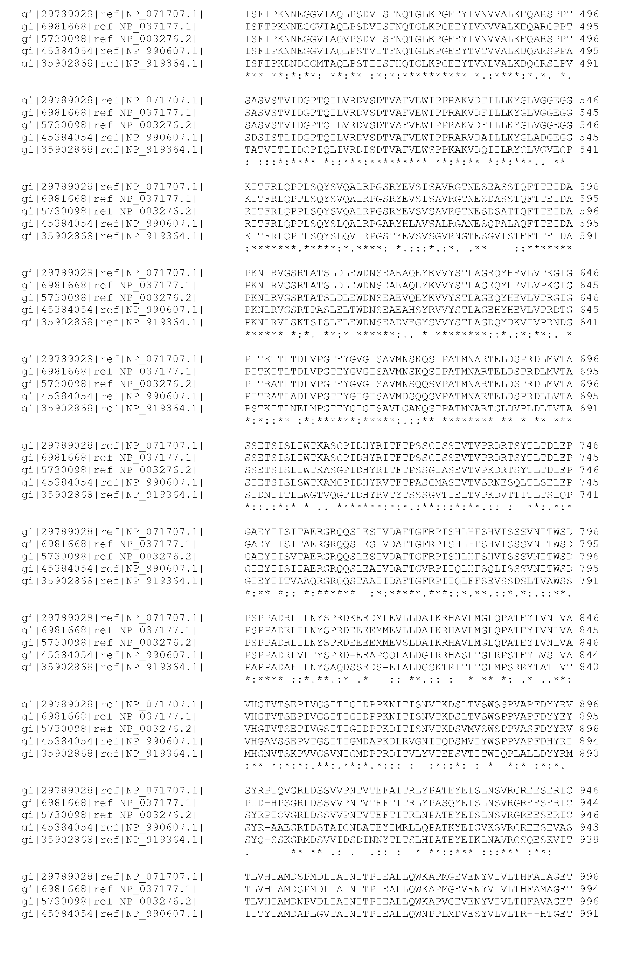

Fig. 3: H-TNR-Cys-vermittelte neurale Differenzierung humaner mesenchymaler Stammzellen (hMSC) aus adultem Knochenmark. Auf den verschiedenen Substraten wachsende Zellen wurden nach den angegebenen Kultivierungszeiten mit Antikörpern gegen Markermoleküle für Neurone (βIII tubulin), neurale/gliale Vorläuferzellen (nestin), Oligodendrozyten (sulfatide, GaIC) oder Astrozyten (GFAP) mittels indirekter Immunfluoreszenz angefärbt. Zur Bestimmung der Überlebensrate (cell survival) wurden adhärente Zellen auf den verschiedenen Substraten am Anfang und am Ende der Kultivierungsperiode mit DAPI angefärbt und die entsprechende Gesamtzellzahl zueinander in Relation gesetzt.
A) hMSC (linkes Bild) wurden auf substratgebundenen Laminin und TN-R Fragmenten (H-TNR-Cys, H-TNR-S3) für 5 Tage in serumfreiem Wachstumsmedium kultiviert.
B) Gliale Differenzierung von hMSC auf substratgebundenen TN-R Fragmenten (H-TNR-Cys, H-TNR-S3). Auf TN-R Fragmenten oder Laminin ausplattierte Zellen wurden für 2 Tage in serumfreiem Wachstumsmedium und für weitere 3 Tage in serumfreiem glialem Differenzierungsmedium kultiviert. Die Zahl der mit Antikörpern markierten Zellen auf dem jeweiligen Substrat wurde zur Gesamtzellzahl (100%) in Relation gesetzt. Über 80% der Zellen auf H-TNR-Cys Substraten waren Sulfatid-/GaIC-positiv und konnten als Oligodendrozyten identifiziert werden.
C) Neuronale Differenzierung von hMSC auf substratgebundenen TN-R Fragmenten (H-TNR-Cys, H-TNR-S3). Auf TN-R Fragmenten oder poly-D-Lysin (PDL) ausplattierte, aus Neurosphären stammende Zellen wurden für 10 Tage in serumfreiem neuronalem Differenzierungsmedium kultiviert. Die Zahl der mit Antikörpern markierten Zellen auf dem jeweiligen Substrat wurde zur Gesamtzellzahl (100%) in Relation gesetzt. 80% der Zellen auf H-TNR-Cys Substraten waren βIII Tubulin-positiv und konnten als Neurone identifiziert werden. D) Zusammenfassung über den Einfluss von H-TNR-Cys auf die neurale Transdifferenzierung von hMSC unter definierten serumfreien Kulturbedingungen. Überlebensrate und Expression Neuron- (βIII Tubulin) und Oligodendrozytenspezifischer Moleküle (Sulfatid/GaIC) von auf H-TNR-Cys Substraten wachsenden Zellen kultiviert in glialem (glial) oder neuronalem (neuronal) Differenzierungsmedium.

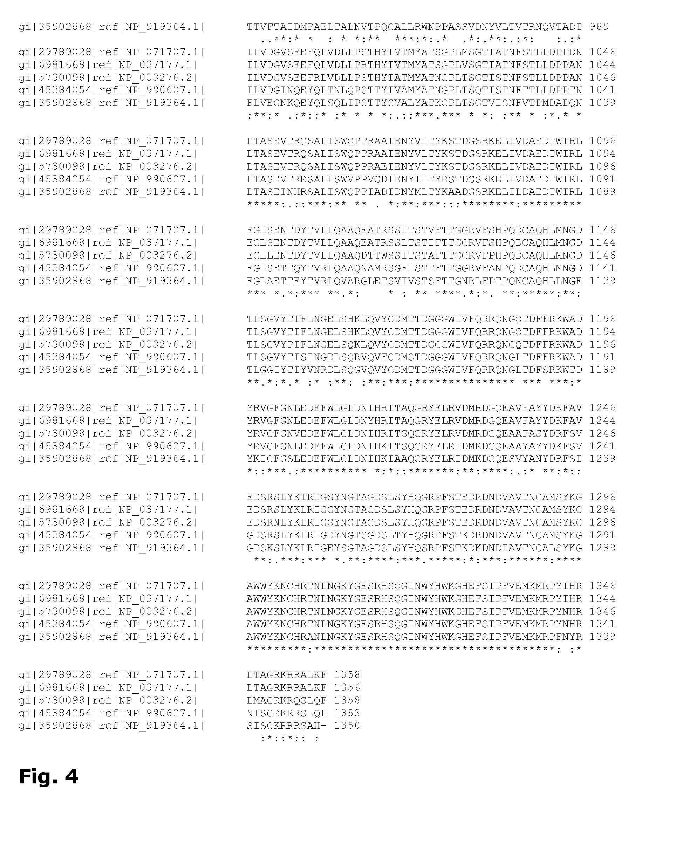

Fig. 4: CLUSTAL W (1.82)-Alignment der bekannten Aminosäuresequenzen von Tenascin-R aus verschiedenen Vertebraten, nämlich Hausmaus (SEQ ID NO:6), Ratte (SEQ ID NO:7), Mensch (SEQ ID NO:8), Huhn (SEQ ID NO:9) und Zebrafisch (SEQ ID NO:10), "*" kennzeichnet identische Aminosäuren, ":" kennzeichnet konservativen Aminosäureaustausch, "." kennzeichnet semi-konservativen Aminosäureaustausch.

| **Sequenzprotokoll - freier Text** | |
|---|---|
| SEQ ID NO: | Beschreibung |
| 1 & 2 | TN-R Nukleinsäuresequenz und Protein |
| 3-4 | Primer |
| 5 | Vektor pIN-II-omp A2 |
| 6-10 | TNR Protein von Hausmaus, Ratte, Mensch, Huhn und Zebrafisch |

### Detaillierte Beschreibung der Erfindung

Die Erfindung betrifft eine Methode zur Isolierung hochreiner Zellpopulationen aus neuralem Primärgewebe in einem Einstufenverfahren mit Hilfe nativer Tenascin-R-Proteine oder TN-R-Fragmente von Vertebraten, bevorzugt von Fischen, Amphibien, Reptilien, Vögeln und Säugetieren, besonders bevorzugt von Hai, Karpfen, Huhn, Nagern einschließlich Maus und Ratte, Rind, Schwein und Mensch, ganz besonders bevorzugt aus Nagern und Mensch, durch selektive Substratadhäsion.

Phylogenetische Studien zur Funktion von Tenascin-R im Nervensystem von Vertebraten zeigen als stammesgeschichtlich hoch konservierte Funktion von Tenascin-R-Proteinen die Unterstützung der Adhäsion und Fortsatzbildung von Oligodendrozyten. Die vorliegende Erfindung zeigt, dass diese Eigenschaft des gesamten Tenascin-R-Proteins auch auf distinkten rekombinanten Fragmenten des humanen Tenascin-R Proteins lokalisierbar ist.

"Tenascin-R-Sonde" bedeutet im Kontext der vorliegenden Erfindung ein N-terminales Fragment von nativem Tenascin-R, welches *in vivo* und *in vitro* durch Oligodendrozyten erkannt und gebunden wird, und/oder weitere Tenascin-R-Verbindungen einschließlich Tenascin-R-Homologe und Tenascin-R-Fragmente, die ebenfalls mit hoher Sensitivität und Spezifität *in vivo* und *in vitro* durch Oligodendrozyten und/oder durch andere neurale Zellen aus Primärgewebe gebunden werden, enthalten kann, wobei "Tenascin-R-Verbindungen" die an anderer Stelle definierte Bedeutung hat. Dies schließt auch Fusionsproteine aus mehreren solcher N-terminalen Tenascin-R-Fragmente ein, bevorzugt aus zwei oder drei Tenascin-R-Verbindungen. Die Bestandteile dieser Fusionsproteine können direkt oder mittels eines (flexiblen) Linkerpeptids miteinander verknüpft sein. Besonders bevorzugt enthält die Tenascin-R-Sonde humane Tenascin-R-Fragmente, ganz besonders bevorzugt die Teilsequenz der humanen TN-R, welche die Aminosäurereste 24 bis 189 der SEQ ID NO:2 umfasst. Die Tenascin-R-Sonde kann neben ihren TN-R-Anteilen auch Nicht-TN-R-Anteile besitzen, welche die Stabilität des TN-R-Anteils gewährleisten und/oder die Immobilisierbarkeit und Kopplungsfähigkeit an andere Moleküle erhalten oder erhöhen. Insbesondere ist eine erfindungsgemäße Tenascin-R-Sonde rekombinant hergestellt.

"Tenascin-R-Verbindungen" sind erfindungsgemäß Proteine oder Peptide, welche entweder N-terminale Fragmente von nativen TN-R oder deren substantiell identische Homologe sind.

"Primärgewebe" ist ein biologisches Gewebe, das direkt aus einem Organismus entnommen und ohne weitere Veränderung seines genetischen Materials verwendet wird. "Primärzellen" sind aus Primärgewebe stammende Zellen. Primärzellen sind gegenüber Zelllinien vorteilhaft, weil sie aufgrund ihrer Herkunft den Zellen im intakten Organismus strukturell und funktionell am nächsten stehen.

"Isoliert" bedeutet im Falle eines Proteins, dass es von anderen Proteinen, mit denen es normalerweise in demjenigen Organismus assoziiert ist, in welchem es natürlicherweise vorkommt, getrennt oder gereinigt wurde. Dies umfasst biochemisch gereinigte Proteine, rekombinant hergestellte Proteine und auf chemischem Wege synthetisierte Proteine. Die Definition gilt übertragen auch für Nukleinsäuren, insbesondere DNA, und Peptide.

"Nativ" wird gleichbedeutend mit "natürlich" bzw. "natürlich vorkommend" verwendet.

Für die erfindungsgemäße "rekombinante Herstellung" werden in der Fachwelt übliche Methoden zur rekombinanten Herstellung eukaryotischer Proteine verwendet, insbesondere die Expression als Fusionsprotein in pro- und eukaryotischen Zellen, ganz besonders bevorzugt die Expression als Fusionsprotein mit Polyhistidinschwanz. Des Weiteren bevorzugt ist die Verwendung von DNA, die für das entsprechende Protein kodiert.

Nukleinsäuresequenzen, insbesondere DNA-Sequenzen, die für erfindungsgemäße Proteine oder Peptide kodieren, sind im Rahmen der vorliegenden Erfindung entweder identisch oder substantiell identisch zur nativen oder zu der ihr zugrundeliegenden künstlichen erfindungsgemäßen Sequenz. Wenn im Rahmen dieser Erfindung eine spezielle Nukleinsäuresequenz genannt wird, so sind damit diese Sequenz selbst und die zu ihr substantiell identischen Sequenzen erfasst. "Substantiell identisch" bedeutet hierbei, dass nur ein Austausch von Basen in der Sequenz im Rahmen des degenerierten Nukleinsäure-Codes erfolgt ist, dass also dadurch die Codons innerhalb kodierender Sequenzen der substantiell identischen Nukleinsäure lediglich gegenüber dem ursprünglichen Molekül in einer Weise verändert sind, welche nicht zu einer Veränderung der Aminosäuresequenz des Translationsprodukts führt (in der Regel Austausch des Codons durch ein anderes Codon seiner Codon-Familie). Bevorzugt sind im Rahmen der vorliegenden Erfindung die Sequenzen, welche im Sequenzprotokoll benannt werden, und deren erfindungsgemäße Fragmente.

Proteinsequenzen und Peptidsequenzen können im Rahmen der vorliegenden Erfindung durch Substitution von Aminosäuren modifiziert werden. Bevorzugt sind solche Substitutionen, bei denen die Funktion und/oder Konformation des Proteins oder Peptids erhalten bleibt, besonders bevorzugt jene Substitutionen, bei denen eine oder mehrere Aminosäuren durch Aminosäuren ersetzt werden, die ähnliche chemische Eigenschaften besitzen, z. B. Valin durch Alanin ("konservativer Aminosäureaustausch"). Der Anteil der substituierten Aminosäuren im Vergleich zum nativen Protein oder - falls es sich nicht um ein natives Protein handelt - zur Ausgangssequenz beträgt bevorzugt 0-30% (bezogen auf die Zahl der Aminosäuren in der Sequenz), besonders bevorzugt 0-15%, ganz besonders bevorzugt 0-5%.

Nukleinsäuresequenzen und Aminosäuresequenzen können als Volllänge-Sequenzen oder als Additions- oder Deletionsprodukte dieser Volllängesequenzen zur Durchführung der Erfindung eingesetzt werden. Bei den Aminosäuresequenzen umfassen die Additionsprodukte auch Fusionsproteine, außerdem Aminosäuresequenzen, die durch Addition von 1-100, bevorzugt 1-30, ganz besonders bevorzugt 1-10 Aminosäuren entstehen. Die addierten Aminosäuren können dabei einzeln oder in zusammenhängenden Abschnitten von 2 oder mehr miteinander verknüpften Aminosäuren ein- oder angefügt werden. Die Addition kann am N- oder C-Terminus oder innerhalb der ursprünglichen Sequenz stattfinden. Es sind mehrere Additionen in einer Sequenz zulässig, wobei eine einzelne Addition bevorzugt ist, besonders bevorzugt eine Addition am C- oder N-Terminus.

Die Deletionsprodukte der Volllängen-Aminosäuresequenzen entstehen - falls nicht für spezielle Sequenzen anders angegeben - durch Deletion von 1-100, bevorzugt 1-20, ganz besonders bevorzugt 1-10 Aminosäuren. Die deletierten Aminosäuren können dabei einzeln oder in zusammenhängenden Abschnitten von 2 oder mehr miteinander verknüpften Aminosäuren entfernt werden. Die Deletion kann am N-oder C-Terminus oder innerhalb der ursprünglichen Sequenz stattfinden. Es sind mehrere Deletionen in einer Sequenz zulässig, wobei eine einzelne Deletion bevorzugt ist, besonders bevorzugt eine Deletion am C- oder N-Terminus.

Die zulässigen Deletionen und Additionen in den erfindungsgemäßen Nukleinsäuresequenzen haben den Umfang und die Ausprägung, welche der zulässigen Aminosäuredeletionen bzw.- additionen entsprechen. Über die Deletion und Addition ganzer Codons hinaus ist auch die Addition oder Deletion von einzelnen oder paarweisen Basen möglich.

Ein Fragment einer Nukleinsäure oder eines Proteins ist ein Teil von dessen Sequenz, das kürzer als Volllänge ist, jedoch noch einen minimal zur Hybridisierung bzw. spezifischen Bindung erforderlichen Sequenzabschnitt enthält. Im Falle einer Nukleinsäure ist dieser Sequenzabschnitt noch in der Lage, mit der nativen Nukleinsäure unter stringenten Bedingungen zu hybridisieren und umfasst vorzugsweise mindestens 15 Nukleotide, besonders bevorzugt mindestens 25 Nukleotide. Im Falle eines Peptids ist dieser Sequenzabschnitt ausreichend, um eine Bindung eines für einen Abschnitt des nativen Proteins spezifischen Antikörpers oder einer Zelle, welche an TN-R oder ein TN-R-Fragment bindet, zu ermöglichen. Die Peptidlänge beträgt vorzugsweise wenigstens 5 Aminosäuren, besonders bevorzugt wenigstens 10 Aminosäuren, ganz besonders bevorzugt wenigstens 20 Aminosäuren.

Ein "Fusionsprotein" im Kontext der vorliegenden Erfindung umfasst mindestens eine erfindungsgemäße Tenascin-R-Verbindung, die mit mindestens einem zweiten Protein oder Peptid verknüpft ist. Dieses zweite Protein oder Peptid ist bevorzugt ein Selektions- oder Markerprotein, ein Protein, welches der Bindung des Fusionsproteins an eine Oberfläche dient, oder eine Tenascin-R-Verbindung. Bevorzugt sind Fusionsproteine aus nativem Tenascin-R und weiteren funktionellen Proteinen und Peptiden, und Fusionsproteine aus zwei oder mehr, besonders bevorzugt zwei oder drei Tenascin-R-Fragmenten. Die Nukleinsäuresequenzen, die in einem Vektor oder einem transformierten Wirtsorganismus für die einzelnen Teile des Fusionsproteins kodieren, sind in einer Weise miteinander verknüpft, welche die Expression unter der Kontrolle eines einzigen Promoters erlaubt. Die Aminosäuresequenzen der einzelnen funktionalen Teile des Fusionsproteins sind dabei entweder direkt oder mit einem Linker verknüpft. Der Linker ist 1-30 Aminosäuren lang, bevorzugt 10-20 Aminosäuren.

"Neurodegenerative Erkrankungen" sind Erkrankungen des Nervensystems, die mit dem Absterben von neuronalen und/oder makroglialen Zellen aufgrund von Beeinträchtigungen ihrer funktionellen Integrität assoziiert sind. Solche Zellschädigungen und -verluste führen zu Ausfall oder Störung der Funktionen der betroffenen Regionen des Nervensystems und/oder der von diesen Regionen gesteuerten Körperteilen.

Die durch das Verfahren nach Ausführungsform (1) und (2) isolierten Zellen sind bevorzugt gliale Zellen, besonders bevorzugt Oligodendrozyten.

Die Tenascin-R-Sonde zur Verwendung in Ausführungsform (1) stammt dabei bevorzugt aus Hirngewebe von Fisch (Hai, Karpfen, Goldfisch, Forelle etc.), Amphibien (Salamander, Frosch, etc.), Reptilien (griechische Landschildkröte, Ringelnatter, etc.), Vögeln (Huhn, Taube etc.) und Säugern (Igel, Kaninchen, Nager, Schwein, Rind, Mensch), besonders von Säugetieren, insbesondere aus Nager, Schwein, Rind oder Mensch, wobei die Fragmente dem vorstehend definierten humanen Fragment entsprechen. Das TN-R-Proteinfragment gemäß der Erfindung wird bevorzugt rekombinant hergestellt und/oder ist ein humanes TN-R-Fragment. Eine bevorzugte Quelle für die entsprechende DNA-Sequenz ist im letztgenannten Fall die humane Neuroblastomzelllinie SH-SY5Y. Die Expression des Fragments erfolgt bevorzugt nach Transformation von humanen Flp-In 293-Zellen (Invitrogen) oder in ausgewählten *E*. *coli* Stämmen mit den entsprechenden DNA-Sequenzen.

Die TN-R-Sonde kann in Ausführungsform (1) zudem weitere funktionelle Protein-oder Peptidsequenzen enthalten und/oder an einen Träger gekoppelt sein.

Ein bevorzugter Aspekt der Ausführungsform (1) und (2) ist die Tenascin-R-Sonde, ein Tenascin-R-Fragment (a), das dem von Nukleotiden 151-648 der SEQ ID NO:1 kodierten humanen Tenascin-R-Fragment entspricht und ist vorzugsweise ein Fragment mit Aminosäureresten 51 bis 216 von SEQ ID NO:2.

In einem weiteren bevorzugten Aspekt der Ausführungsform (1) und (2) ist die Tenascin-R-Sonde (b) ein Fragment von (a), bei dem bis zu 10, vorzugsweise bis zu 5 Aminosäurereste von N- und/oder C-Terminus abgespaltet sind, und/oder das wenigstens 150 Aminosäurereste aufweist.

In einem weiteren bevorzugten Aspekt der Ausführungsform (1) und (2) ist die Tenascin-R-Sonde (c) eine Substitutions-, Deletions- und/oder Additionsmutante von (a) oder (b).

In einem weiteren bevorzugten Aspekt der Ausführungsform (1) und (2) ist die Tenascin-R-Sonde ein Tenascin-R-Fusionsprotein (d) mit einem wie vorstehend unter (a) bis (c) definiertem Tenascin-R-Fragment und einer funktionelle Komponente, umfassend eines oder mehrere weiterer funktioneller Peptide oder Proteine, oder ein Tenascin-R-Fusionsprotein, das aus zwei oder mehreren, bevorzugt zwei oder drei der wie vorstehend unter (a) bis (c) definierten funktionellen Tenascin-R-Fragmenten zusammengesetzt ist.

Die phylogenetisch konservierte Eigenschaft von TN-R Proteinen, als adhäsives Substrat für Oligodendrozyten wirken zu können, wird auf molekularer Ebene durch eine hohe Konservierung der Aminosäuresequenz zwischen verschiedenen Vertebratenspezies reflektiert: die humane TN-R Sequenz weist Homologien von 93% (zu Ratte), 75% (zu Huhn) und 60% (zu Zebrafisch) auf (Fig. 4). Diese Eigenschaft macht sich die vorliegende Anmeldung zunutze.

Die Isolierung des TN-R aus natürlichen Quellen erfolgt bevorzugt durch bekannte chromatographische und/oder immunologische Methoden zur Proteinreinigung, insbesondere durch Affinitätschromatographie an TN-R-Antikörper (Bsp. 1). Als Quelle für das TN-R kommen Gewebe und Einzelzellsuspensionen höherer und niederer Vertebraten infrage.

Rekombinante Methoden zur Herstellung der TN-R-Sonde umfassen die bekannten gängigen Methoden zur Transformation von Pro- und Eukaryoten (beschrieben z. B. in G. Schrimpf (Hrsg.), Gentechnische Methoden, 3. Auflage, Spektrum Akademischer Verlag (2002); Smith, C., The Scientist 12(3):18 (1998); Unger, T., The Scientist 11(17):20 (1997)). Geeignete Methoden zur Herstellung rekombinanter Proteine oder Proteinfragmente umfassen Transfektions- oder Transformationsmethoden, die auf verschiedenen Expressionssystemen/-vektoren für prokaryotische (insbesondere *E*. *coli*) und eukaryotische Zellen (Hefe, Pilze, Insekten- und Säugerzellen) basieren. Um funktionell aktive rekombinante Proteine zu erzeugen (also solche, die nach ihrer Faltung/Konformation und Glykosylierung dem nativen Protein am ähnlichsten sind), werden Säugerzellen als Produzenten bevorzugt. Die verschiedenen Expressionsvektoren für Säugerzellen unterscheiden sich hauptsächlich in Promotertyp (SV40, CMV, human EF1alpha, MMTV-LTR, MSV-LTR, RSV-LTR, etc.), Art der Expression (transient, konstitutiv, induzierbar), Induktionsmechanismus, Selektionsmarker (Antibiotikum- oder Drogenresistenz und/oder Koexpression leicht nachweisbarer Proteine) und Elemente für subzelluläres Targeting des Genprodukts (Mitochondrien, Kern, Sekretion). Als Produzenten rekombinanter Proteine/Proteinfragmente werden pro- und eukaryotische Expressionssysteme, die das Fremdgen konstitutiv exprimieren, bevorzugt.

In einer bevorzugten Durchführungsform von (2) zur erfindungsgemäßen Expression humaner rekombinanter Proteinfragmente werden definierte PCR-Fragmente des humanen TN-R mit Hilfe des TOPO TA Klonierungssystems (Invitrogen) in den pcsecTag/FRT/V5-His-TOPO-Vektor (Invitrogen) kloniert, welcher die konstitutive Expression und Sekretion des gewünschten, mit einem am C-Terminus mit 6xHis-Peptid versehenen Proteinfragments in humanen Flp-In 293 Zellen (Invitrogen) erlaubt. In Flp-In-Zellen ist das Plasmid pFRT/IacZeo (Invitrogen) stabil integriert, die FRT-Region wird spezifisch von der Flp-Rekombinase erkannt. Bei gleichzeitiger Transfektion der Flp-293-Zellen mit dem pOG44-Plasmid, das die Expression der Flp-Rekombinase ermöglicht, und dem die Basensequenz des gewünschten Proteinfragments tragenden pcsecTag/FRT/V5-His-TOPO-Vektor kommt es an der FRT-Region zu einem Einbau der für die Herstellung eines sekretierten Proteinfragments notwendigen Anteile des pcsecTag/FRT/V5-His-TOPO-Vektors. Dies ermöglicht die Sekretion polyHis-tragender Proteinfragmente in den Zellkulturüberstand und ihre Aufreinigung über Nickel-Chelatchromatographie aus gesammelten Zellkulturüberständen.

Ein weiterer Aspekt der Ausführungsform (1) ist die Herstellung der TN-R-Fragmente durch chemische Synthese, durch Fragmentierung von isoliertem TN-R oder rekombinant, bevorzugt rekombinant gemäß Ausführungsform (7). Geeignete rekombinante Methoden umfassen die bekannten gängigen Methoden zur Transformation von Pro- und Eukaryoten (beschrieben z. B. in G. Schrimpf (Hrsg.), Gentechnische Methoden, 3. Auflage, Spektrum Akademischer Verlag (2002); Smith, C., The Scientist 12(3):18 (1998); Unger, T., The Scientist 11(17):20 (1997)). Hierfür kann ein Wirtsorganismus gemäß Ausführungsform (6) verwendet werden, der mit einem Vektor transformiert oder transfiziert ist, der die vorstehend definierten TN-R oder TN-R-Fusionsprotein kodierenden DNA Sequenzen umfasst. Solch ein Vektor kann, neben den genannten DNA Sequenzen noch an den Wirtsorganismus angepasste funktionelle Sequenzen wie Promotoren, Leadersequenzen usw. enthalten. Zur chemischen Herstellung von TN-R-Fragmenten sowie zur Fragmentierung von isoliertem TN-R können einschlägig bekannte Methoden verwendet werden, wie z. B. Festphasen-Peptidsynthese bzw. enzymatische oder mechanische Fragmentierungsmethoden.

Ein weiterer bevorzugter Aspekt der Ausführungsform (1) betrifft ein Fusionsprotein umfassend eine Tenascin-R-Komponente, ausgewählt aus nativem Tenascin-R oder Tenascin-R-Fragmenten und Fusionsproteinen aus zwei der mehr Tenascin-R-Fragmente, und eine funktionelle Komponente, die funktionelle Peptid- oder Proteinsequenzen umfasst. Die Komponenten können direkt oder mittels eines (flexiblen) Linkerpeptides miteinander verknüpft sein. Ein weiterer Aspekt betrifft die Kombination von zwei oder mehreren der vorstehend definierten Tenascin-R-Komponenten, insbesondere Tenascin-R-Fragmenten, in einer Art und Weise, die nicht der nativen Aminosäuresequenz entspricht, zu einer erfindungsgemäßen Tenascin-R-Sonde. Die Verknüpfung kann ebenfalls direkt oder mittels eines Linkerpeptids erfolgen. Zur Synthese können die oben genannten Vektorsysteme verwendet werden, wobei diejenigen cDNA-Sequenzen von Teilen der TN-R-Sequenz, welche räumlich nicht benachbart sind, über endständige Restriktionsenzym-Schnittstellen nach gängigen technischen verfahren miteinander verknüpft werden.

Die Tenascin-R-Sonde gemäß Ausführungsform (1) und (2) umfasst vorzugsweise die Teilsequenz der humanen TN-R, welche die Aminosäurereste 24 - 189 in SEQ ID NO:2 entspricht. Ein bevorzugter Aspekt von (1) bis (4) ist, dass die Sonde eine der Aminosäuresequenzen dieser Gruppe besitzt oder sich aus 2 oder mehreren der Aminosäuresequenzen dieser Gruppe in einem Fusionsprotein zusammensetzt, wobei auch die Wiederholung einer oder mehrerer der Sequenzen innerhalb des Fusionsproteins möglich ist. Gegenstand der Erfindung sind auch die Nukleinsäuren, die Nukleinsäurefragmente umfassen, welche für derartige Proteine kodieren, bevorzugt DNA-Sequenzen und cDNA.

In einem bevorzugten Aspekt der Ausführungsform (2) ist die Tenascin-R-Sonde ein Fragment von humanem Tenascin-R und entweder durch Präparation aus Zellen von Menschen oder durch rekombinante Herstellung erhältlich. Insbesondere ist es als natives TN-R aus Zellen neuralen Ursprungs, besonders bevorzugt aus SH-SY5Y Neuroblastomzellen erhältlich. Rekombinante Herstellung speziell der TN-R-Fragmente findet bevorzugt in entsprechend transformierten humanen Flp-In 293-Zellen oder ausgewählten *E*. *coli* Stämmen statt.

Ein bevorzugter Aspekt der Ausführungsform (1) und (2) ist die Durchführung des Verfahrens als Einstufenverfahren und/oder durch selektive Substratadhäsion an die Tenascin-R-Sonde. Hierbei werden durch enzymatische Behandlung des gewünschten zentralnervösen Gewebes gewonnene Einzelzellsuspensionen auf mit TN-R Proteinen oder Proteinfragmenten beschichteten Plastikoberflächen ausgesät. Nach Inkubation, bevorzugt für 8-20 h und bevorzugt in serumfreiem Medium (was eine Vermehrung astrozytärer und mikroglialer Zellen verhindert), finden sich auf den immobilisierten TN-R Substraten reine Oligodendrozytenpopulationen. Andere Zelltypen liegen im Zellkulturüberstand vor und können durch Mediumwechsel vollständig entfernt werden. Da nur ein einziger Selektionsschritt erfolgt und die Selektionsphase kurz ist im Vergleich zur Dauer der bislang üblichen mehrwöchigen Kultivierung gemischter glialer Kulturen und weiterer Selektionsverfahren, ist die resultierende Zellausbeute hoch. Grund dafür ist u.a., dass sämtliche Oligodendrozyten (verschiedener Differenzierungsstadien) aus einem Primärgewebe auf TN-R-Substraten selektioniert werden können. Im Gegensatz hierzu ist die Isolierung von Oligodendrozyten aus gemischten glialen Kulturen mit einem hohen Verlust an Zellen verbunden (z. B. beim Abschütteln der auf einer Astrozyten-Monoschicht haftenden Oligodendrozyten und Mikroglia und bei weiterer Selektion mikroglialer Zellen).

Das erfindungsgemäße Isolierungsverfahren nach (1) und (2) erlaubt die Isolierung vollständiger Oligodendrozytenpopulationen und ist damit auch vorteilhaft gegenüber immunologischen Selektionsverfahren wie z. B. FACS, biomagnetic cell sorting oder Antikörper-Panning. Diese erlauben nur die Anreicherung distinkter Oligodendrozytenpopulationen; oligodendrogliale Zellen, die von den Antikörpern nicht erkannt werden, gehen verloren. Insbesondere hat die in dem Verfahren nach (1) und (2) verwendete Sonde die Eigenschaft, dass sie das Überleben und Transdifferenzierung adulter humaner mesenchymaler Stammzellen in neuronale oder oligodendrogliale Zellen, insbesondere unter definierten serumfreien Kulturbedingungen unterstützt

Das erfindungsgemäße Verfahren von (1) und (2) ermöglich somit die Isolierung von oftmals für weitere Experimente ausreichenden Oligodendrozytenpopulationen aus einem einzigen Vertebraten, insbesondere einem einzigen Nager. Dies ist insbesondere von Vorteil, wenn bestimmte Effekte an Mäusen untersucht werden, die z. B. in transgenen Mäusen, Knockout-Mäusen oder mit Testsubstanzen behandelten Mäusen auftreten.

Die als Startmaterial der Ausführungsform (1) und (2) verwendeten Primärgewebe können aus unterschiedlichen ZNS-Bereichen sowie aus unterschiedlichen Entwicklungsstadien stammen. Bevorzugte ZNS-Bereiche sind das Gehirn und einzelne Gehirnregionen (insbesondere Vorderhirn, Kleinhirn, Hippocampus, Hirnstamm), der optische Nerv und das Rückenmark. Die geeigneten Entwicklungsstadien umfassen embryonale, fötale, früh-/spätpostnatale und adulte Gewebe, bevorzugt frühpostnatale und adulte Gewebe.

Wird im Verfahren nach (1) bis (2) eine Einzelzellsuspension verwendet, so enthält diese Zellen einer oder mehrerer Differenzierungsstufen, bevorzugt einer einzigen Differenzierungsstufe.

Die neuralen Primärgewebe, die zur Durchführung des Verfahrens nach (1) und (2) verwendet werden, stammen aus niederen und höheren Vertebraten, einschließlich Fischen, Amphibien, Reptilien, Vögeln und Säugetieren, besonders bevorzugt aus Hai, Karpfen, Huhn, Nagern einschließlich Maus und Ratte, Rind, Schwein und Mensch, ganz besonders bevorzugt aus Nagern und Mensch.

Die TN-R-Sonde zur Verwendung in (1) und (2) stammt vorzugsweise aus TN-R höherer und niederer Vertebraten. Bevorzugt ist sie das native TN-R oder ein Fragment des nativen TN-R.

Die Gewinnung von Einzelzellsuspensionen aus Primärgewebe zum Einsatz im Verfahren gemäß Ausführungsform (1) oder (2) erfolgt nach in der Fachwelt üblichen Methoden. So kann das Gewebe in einem oder mehreren Schritten mechanisch und/oder enzymatisch in Gewebefragmente und/oder einzelne Zellen überführt werden. Geeignete Methoden werden in "Zell- und Gewebekultur" (T. Lindl, Spektrum Akademischer Verlag, 2002) und "Current Protocols in Neuroscience" (John Wiley & Sons, Inc., 2004, Hrsg. J Crawley et al.) beschrieben. Die so gewonnenen Zellen werden in serumfreiem Medium resuspendiert und dann mit der TN-R-Sonde in Kontakt gebracht. Nach einer zur vollständigen Adsorption der selektierten Zellen ausreichenden Inkubationszeit (1-48 h, bevorzugt 8-20 h) unter geeigneten Bedingungen werden die nicht adhärierten Zellen entfernt. Die adhärierten Zellen können für eine weitere Verwendung entweder adhärent bleiben oder enzymatisch, bevorzugt durch Behandlung mit Trypsin bzw. Trypsin-EDTA, Collagenase, Dispase, Pronase, Accutase^{®} oder anderen geeigneten Proteinasen, insbesondere mit Accutase^{®}, vom Adsorbens abgelöst werden. Ihre Weiterverwendung umfasst die Kultivierung - auch auf anderen Substraten oder Plastikoberflächen bzw. in anderen definierten Medien - zur Gewinnung von beispielsweise unreifen Vorläufer-Oligodendrozyten oder myelinkompetenten Oligodendrozyten.

Das Verfahren nach (1) und (2) kann unabhängig davon eingesetzt werden, ob die TN-R-Sonde und das neurale Primärgewebe aus Organismen derselben Spezies stammen. So ist eine Isolierung von Oligodendrozyten über Speziesgrenzen hinweg möglich. TN-R aus verschiedenen Vertebraten können zur Selektion von Oligodendrozyten aus Einzelzellkulturen anderer Spezies verwendet werden. Unter anderem umfasst dies die Selektion von Oligodendrozyten aus Mensch, Schwein, Rind, Huhn, Maus, Ratte, Frosch und anderen höheren Vertebraten auf TN-R aus einer anderen Spezies (umfassend Fisch, Huhn, Maus, Ratte, Rind, Schwein, Mensch etc.).

Das Verfahren nach (1) und (2) kann des Weiteren unabhängig davon eingesetzt werden, in welcher Differenzierungsstufe (z. B. Vorläufer - unreife - reife Oligodendrozyten) die selektierten Zellen vorliegen. Es werden allen Zellen einer Zellart selektiert, unabhängig von ihrem Entwicklungsstadium.

Die Selektion von definierten Zellpopulationen aus Einzelzellsuspensionen gemäß (1) und (2) erfolgt in einem Aspekt dadurch, dass die an die TN-R-Sonde gebundenen Zellen isoliert werden und zur weiteren Verwendung vorgesehen sind. In einem anderen Aspekt dagegen ist es der Überstand, der durch die spezifische Adsorption von Zellen an die TN-R-Sonde von diesen Zellen befreit wird und zur weiteren Verwendung als definierte Zellpopulation vorgesehen ist. In wieder einem anderen Aspekt wird als TN-R-Sonde ein modifiziertes TN-R oder ein TN-R-Fragment eingesetzt, dass für andere Zellen als das Ausgangsprotein (natives TN-R) selektiv ist.

In einem Aspekt der Ausführungsform (1) oder (2) ist die Tenascin-R-Sonde durch geeignete Methoden zur Immobilisierung an ein Trägermaterial gekoppelt. Die Immobilisierung kann kovalent oder nichtkovalent erfolgen. Derartige geeignete Immobilisierungsmethoden umfassen adäquate Kupplungstechniken, welche die Spezifität der Tenascin-R-Sonde nicht verändern, wie z. B. die kovalente Vernetzung des Proteins mit dem Trägermaterial oder die Immobilisierung durch Wechselwirkung mit einem geeigneten Antikörper. Bevorzugt erfolgt die Kupplung nichtkovalent, über einen Antikörper oder durch kovalente Vernetzung.

In einer bevorzugten Ausprägung dieses Aspekts der Ausführungsform (1) oder (2) werden zur Isolierung von Zellen aus Primärgewebe neuralen Ursprungs Trägermaterialien wie z. B. Zellkulturplatten mit TN-R bzw. mit rekombinant hergestellten TN-R-Fragmenten beschichtet, indem das Trägermaterial, bevorzugt eine Plastikoberfläche, mit einer Lösung der TN-R-Sonde inkubiert und anschließend gewaschen wird. Durch selektive Substratadhäsion erfolgt dann die Isolierung reinster Zellpopulationen aus Zellsuspensionen. Unter Verwendung von TN-R konnten aus frühpostnatalen Nagerhirnen 100% reine Oligodendrozytenpräparationen gewonnen werden: 2x10⁶ Oligodendrozyten aus einem P0-P2 Nagervorderhirn, 4-6x10⁶ Oligodendrozyten aus einem P5 Nagervorderhirn, 2x10⁶ Oligodendrozyten aus einem P7-P8 Nagerkleinhirn. Ähnliche Ergebnisse sind mit rekombinant hergestellten Tenascin-R-Fragmenten möglich.

In einer weiteren Ausprägung dieses Aspekts wird die TN-R-Sonde auf einer Plastikoberfläche immobilisiert. Dies ermöglicht ebenfalls die selektive Adhäsion und Isolation von definierten Zellpopulationen, insbesondere von Oligodendrozyten, jedoch nicht von anderen neuralen Zellen (wie Astrozyten, Mikroglia oder Neuronen), aus den als Startmaterial verwendeten gemischten Zellpopulationen.

Die Immobilisierung erfolgt bevorzugt durch direkten Kontakt der TN-R-Sonde mit der Trägeroberfläche. Nach ausreichender Inkubationszeit (1-4 h) wird das nicht gebundene Protein abgewaschen. Die nicht besetzten Bindungsstellen auf der Oberfläche werden anschließend blockiert, z. B. durch Inkubation mit einem BSAhaltigen Blockierungspuffer. Die so beschichteten Oberflächen können bis zu ihrer Verwendung feucht gehalten oder nach Eintrocknen verwendet werden.

Als bevorzugte TN-R-Sonde werden in Ausführungsform (2) ein N-terminales Fragment des nativen TN-R verwendet. Auch die Verwendung einer Mischung aus mehr als einer TN-R-Sonde zur Beschichtung des Trägermaterials ist möglich.

Das Verfahren nach Ausführungsform (1) bis (2) ist zur Gewinnung von neuralen Zellen, insbesondere von Oligodendrozyten, für die Anzucht differenzierter Zellen, insbesondere neuraler Zellen, in neurobiologischen und zellphysiologischen Untersuchungen, in der biologischen und klinischen Forschung und für diagnostische und therapeutische Verfahren *in vitro* und *in vivo,* insbesondere für die Herstellung eines Medikaments zur Zelltherapie und zur Therapie von neurodegenerativen Krankheiten geeignet. Des Weiteren ist es zum Nachweis von neurodegenerativen Krankheiten nutzbar.

Ausführungsform (8) betrifft Antikörper, welche spezifisch an das N-terminale Ende von TN-R, insbesondere an das N-terminale Ende von TNR in wenigstens zwei Spezies, vorzugsweise zwei Vertebraten binden.

Die Kreuzaktivität mit verschiedenen Spezies ist bedingt durch das Herstellverfahren, nämlich dass ein geeigneter Wirtsorganismus mit Tenascin-R von wenigstens zwei unterschiedlichen Spezies, vorzugsweise von zwei unterschiedlichen Vertebraten durch übliche Verfahren immunisiert wird und nachfolgenden Screening- und Reinigungsschritten isoliert wird. Geeignete Wirtsorganismen für die Immunisierung sind insbesondere nicht-humane Säuger wie Nager (Mäuse, Ratten usw.), Kaninchen, Meerschweinchen, Ziege usw.

Die Antikörper nach Ausführungsform (8) sind in verschiedenen auf Nachweis und/oder Bindung von TN-R basierenden immunochemischen Verfahren anwendbar, insbesondere in ELISAs, Westernblots, histologischen und zytologischen Untersuchungen und Immunpräzipitationen. Auch in *in vitro*-Assays sind sie von Bedeutung, da ihre Anwesenheit die inhibitorische Wirkung des TN-R Proteins auf die neuronale Zelladhäsion und das Axonwachstum neutralisieren kann. Die Antikörper reagieren spezifisch mit TN-R in Hirn-Extrakten oder gereinigtem TN-R und zeigen keine Kreuzreaktivität mit anderen TN-Proteinen. Letzteres läßt sich daraus schließen, dass keine Reaktion mit Herz oder Niere (in Maus enthaltend TN-W, Scherbich, A. et al., J. Cell. Sci. 117:571-581 (2004)), neonataler Haut oder Hautfibroblasten-konditionertem Medium (enthaltend TN-X, Zweers, M.C. et al., Cell Tissue Res. 319:279-287 (2005)) und TN-C-Präparationen aus Maushirn stattfindet. Die Antikörper sind dabei insbesondere zur selektiven Blockierung des erfindungsgemäßen TN-R-Fragments (1) und (2) geeignet.

Die monoklonalen Antikörper gemäß Ausführungsform (8) können dabei durch Kultivieren der Zelllinie gemäß Ausführungsform (9) hergestellt werden. Die Zelllinien Ausführungsform (9) sind insbesondere sogenannte Hybridomzelllinien. Diese sind z. B. durch Immunisierung eines geeigneten Wirtsorganismus mit TN-R von wenigstens zwei verschiedenen Spezies, wie vorstehend beschrieben, Isolation von Milzzellen des Wirtsorganismus und nachfolgendes Verschmelzen mit geeigneten primären Zellen, z. B. Myelomzellen, erhältlich. In Abhängigkeit vom Wirtsorganismus und vom Ursprung der primären Zellen handelt es sich um Homo-oder Heterohybridomzellen, wobei das Erstere bevorzugt ist.

Die Antikörper der Ausführungsform (8) der Erfindung sind nicht nur zum immunochemischen Nachweis von TN-R geeignet, sondern auch zur Hemmung der Wirkung von TN-R *in vivo* und *in vitro.*

Aufgrund ihrer Wechselwirkung mit TN-R in Bezug auf die neuronale Zelladhäsion und das Axonwachstum sind die Antikörper nach Ausführungsform (8) zur gezielten Beeinflussung der Neuralentwicklung *in vivo* und *in vitro,* zur Therapie und Prophylaxe von traumatischen Nervenläsionen und zur Herstellung von Medikamenten zur gezielten Beeinflussung der Neuralentwicklung und zur Therapie und Prophylaxe von traumatischen Nervenläsionen einsetzbar. Traumatische Nervenläsionen entstehen z. B. nach mechanischer Nervenschädigung. Die Regeneration der Nervenfasern nach solchen Läsionen wird durch TN-R negativ beeinflusst (Probstmeier, R. et al., J. Neurosci. Res. 60:21-36 (2000); Zhang, Y. et al., Mol. Cell. Neurosci. 17:444-459 (2001); Becker, C.C. et al., Mol. Cell. Neurosci. 26:376-389 (2004); Xu, G. et al., J. Neurochem. 91:1018-1023 (2004)).

Die Erfindung betrifft daher in Ausführungsform (14) ebenfalls ein Verfahren zur Therapie und Prophylaxe von traumatischen Nervenläsionen und zur gezielten Beeinflussung der Neuralentwicklung umfassend das Verabreichen einer geeigneten Menge an Antikörper nach Ausführungsform (8) an einen Patienten, der einer solchen Behandlung bedarf. Die verabreichte Antikörpermenge und die notwendige Dosierung wird vom behandelnden Arzt von Fall zu Fall festzulegen sein. Sie hängt dabei unter anderem einerseits vom Alter, Körpergewicht und der Konstitution des Patienten, andererseits von der Art und Schwere der zu behandelnden Krankheit ab.

Der Kit gemäß Ausführungsform (10) enthält bevorzugt das in Ausführungsform (3) definierten Protein oder eine Stammkultur der Zelllinie zur Produktion dieses Proteins. Besonders bevorzugt enthält dieser Kit humanes Tenascin-R oder dessen erfindungsgemäße Fragmente und/oder eine Stammkultur von Zellen, die zur rekombinanten Produktion dieser Proteine geeignet sind.

Ein bevorzugter Aspekt der Ausführungsform (10) ist ein Kit, in dem die Tenascin-R-Sonde durch eine adäquate Kupplungstechnik an ein Trägermaterial gebunden ist, und/oder in dem weiterhin TN-R-Antikörper (z. B. zur Überprüfung der Immobilisierungseffizienz der TN-R-Sonde), eine oder mehrere enzymatische Lösung(en) zur Zelldissoziierung, ggf. Mittel zur Detektion der Bindung von Zellen an die Tenascin-R-Sonde, Puffer und/oder Kulturmedien enthalten sind. Zu den Puffern und Medien zählen insbesondere Blockierungspuffer und definierte serumfreie Kulturmedien. Im Kit enthaltene Antikörper sind bevorzugt Antikörper der Ausführungsform (8).

In Ausführungsform (11) ist die Verwendung eines TN-R-Fragments oder eines TN-R-Fusionsproteins wie in Ausführungsform (3) definiert, bevorzugt.

Eine bevorzugte Verwendung der erfindungsgemäßen TN-R-Sonde ist der Einsatz der Tenascin-R-Sonde zur Gewinnung von Oligodendrozyten gemäß Ausführungsform (11). Ein damit verknüpfter Aspekt der Ausführungsform (11) ist die Anzucht differenzierter Zellen aus den so gewonnenen Zellpopulationen. Die differenzierungsfördernde Wirkung des nativen TN-R wurde bereits beschrieben (Pesheva, P. et al., J. Neurosci. 17:4642-51 (1997)).

Die Diagnoseverfahren gemäß der Ausführungsform (11) können *in vivo* und *in vitro* durchgeführt werden, bevorzugt jedoch *in vitro.* Bevorzugt verwendet wird für die Verwendung gemäß Ausführungsform (11) eine Tenascin-R-Sonde gemäß Ausführungsform (4), insbesondere humanes Tenascin-R oder dessen erfindungsgemäße Fragmente. Die Tenascin-R-Sonde kann dabei aus Quellen gereinigt worden sein, in denen sie natürlich vorkommt, oder rekombinant hergestellt worden sein. Bevorzugt wird eine rekombinante Tenascin-R-Sonde verwendet.

Neurodegenerative Erkrankungen, die mit einem Verlust von Oligodendrozyten (durch Zelltod) oder Myelin assoziiert sind, wie z. B. Multiple Sklerose (MS), zeichnen sich dadurch aus, dass in den betroffenen Regionen Remyelinisierung nicht oder nur im geringen Umfang stattfinden kann. Das liegt zum Großteil daran, dass die vorhandenen "traumatischen" (also als Folge der Wirkung pathologischer Stimuli veränderten) Vorläufer- und unreifen Oligodendrozyten nicht in der Lage sind zu differenzieren/remyelinisieren. Die Erfindung bietet hier Ansätze für neue diagnostische und/oder therapeutische Verfahren:

Zur Diagnose von MS ist besonders die schnelle (innerhalb von 1-2 Tagen) Selektion "traumatischer" Zellen auf TN-R-Sonden geeignet, bevorzugt aus einem Tiermodell oder aus Biopsie-Proben von Patienten. Dies ermöglicht die Durchführung von direkten Untersuchungen ihres molekularen Profils und/oder die Entwicklung diagnostischer Marker. Für die Entwicklung eines Medikaments zur Zelltherapie können "traumatische" Oligodendrozyten mit verschiedenen Wirkstoffkandidaten behandelt werden, um den Einfluss der letzteren auf die "Genesung traumatischer Zellen", bzw. die Remyelinisierungspotenz solcher Zellen zu bestimmen.

Die erfindungsgemäße Methode ist auch geeignet zur Selektion "normaler" adulter Oligodendrozyten, welche vor der Kultivierung unter traumatischen Bedingungen *in vitro* (durch Zugabe relevanter Cytokine oder Liquorproben von Patienten/erkrankten Tieren) selektioniert und anschließend mit Wirkstoffkandidaten behandelt werden. Solch ein *in vitro-*System erlaubt Untersuchungen zu den stattfindenden traumatischen Veränderungen in Oligodendrozyten, die Rückschlüsse auf solche Veränderungen *in vivo* ermöglichen.

Des Weiteren können derartig kultivierte Oligodendrozyten zur Entwicklung diagnostischer Marker dienen, wobei auch verschiedene Stadien der traumatischen Veränderungen festgestellt werden können. Die so gewonnenen Oligodendrozyten sind auch geeignet zum Screening auf Wirkstoffkandidaten, die den Zelltod und/oder eine fehlende Myelinisierungskompetenz von Oligodendrozyten unter traumatischen Bedingungen aufheben, bzw. als Medikament zur Zelltherapie *in vivo* eingesetzt werden können.

Ein bevorzugter Aspekt der Ausführungsform (11) ist somit die Verwendung der Tenascin-R-Sonde zur Diagnose von Multipler Sklerose (MS) und zur Herstellung eines Medikaments gegen MS.

Ein weiterer bevorzugter Aspekt der Ausführungsform (11) ist somit die Verwendung der Tenascin-R-Sonde zur Herstellung eines Medikaments zur Zelltherapie und zur Therapie von neurodegenerativen Krankheiten, die mit einem Verlust von Oligodendrozyten oder Myelin einhergehen, insbesondere Multipler Sklerose und periventrikulärer Leukomalazie (PVL).

Ausführungsform (13) betrifft ein Verfahren zur Zelltherapie und zur Therapie von neurodegenerativen Krankheiten, die mit einem Verlust von Oligodendrozyten oder Myelin einhergehen, insbesondere Multipler Sklerose und periventrikulärer Leukomalazie (PVL), umfassend das Verabreichen einer pharmakologisch geeigneten Menge der TN-R-Sonde an einen Patienten, der einer solchen Behandlung bedarf. Die verabreichte Menge und die notwendige Dosierung wird vom behandelnden Arzt von Fall zu Fall festzulegen sein. Sie hängt dabei unter anderem einerseits vom Alter, Körpergewicht und der Konstitution des Patienten, andererseits von der Art und Schwere der zu behandelnden Krankheit ab.

Das Verfahren (12) zur Herstellung von Oligodendrozyten aus isolierten Stammzellen kann mit neuralen oder nicht-neuralen Stammzellen durchgeführt werden. Besonders bevorzugte isolierte Stammzellen sind Vorläuferzellen neuralen oder hämatopoetischen Ursprungs. So können humane neurale Stammzellen in Gegenwart von TN-R selektiv zu reifen Oligodendrozyten differenziert werden.

Ganz besonders bevorzugt dient das Verfahren (12) der Differenzierung unreifer Oligodendrozyten *in vitro.*

Bevorzugt wird für die Differenzierung unreifer Oligodendrozyten *in vitro* auf TN-R-beschichteten Oberflächen eine TN-R-Konzentration von mindestens 10 µg/ml eingesetzt. Besonders bevorzugt ist eine TN-R-Konzentration von mindestens 20 µg/ml, ganz besonders eine TN-R-Konzentration von 20 µg/ml.

Bevorzugt wird das Verfahren (12) mit TN-R höherer Vertebraten, besonders bevorzugt mit einer erfindungsgemäßen TN-R-Sonde, ganz besonders bevorzugt mit nativem TN-R oder einem TN-R-Fragment oder -Fusionsprotein der Ausführungsform (4) durchgeführt.

Die Hybridomzelllinien tn-R4 (Produzent des Antikörpers R4) und tn-R6 (Produzent des Antikörpers R6) wurden am 02. Dezember 2005 unter den Hinterlegungsnummern DSM ACC2754 bzw. DSM ACC2753 bei der DSMZ, Deutsche Sammlung von Mikroorganismen und Zellkulturen GmbH, Mascheroder Weg 1b, 38124 Braunschweig, Deutschland, hinterlegt.

Die Erfindung wird anhand der nachfolgenden Beispiele näher erläutert. Diese schränken den Schutzbereich der Erfindung jedoch nicht ein.

### Beispiele

### Verwendete Lösungen/Medien

1. HBSS (Hank's Balanced Salt Solution) (Sigma)
2. Enzymatische Lösungen für Zelldissoziierung:
   1% (w/v) Trypsinlösung: 1% (w/v) Trypsin (Zellkultur getestet); 0,1% (w/v) DNase I; 1 mM EDTA; 0,8 mM MgSO₄; 10 mM HEPES in HBSS (Ca/Mg-frei). DNaselösung: 0,05% (w/v) DNase I; 10 mM HEPES in BME (Basal Medium Eagle).
3. Support Medium: DMEM (Sigma) supplementiert mit 10% (v/v) FCS (fötales Kälberserum), Penicillin (100 units/ml) und Streptomycin (0,1 mg/ml).
4. Definiertes serumfreies Wachstumsmedium: DMEM (Sigma) supplementiert mit Insulin (10 µg/ml), Progesteron (0,06 µg/ml), Putrescin (16 µg/ml), Natriumselenit (0,22 µM), Transferrin (0,1 mg/ml), HEPES (25 mM), Gentamycin (25 µg/ml), Penicillin (100 units/ml) und Streptomycin (0,1 mg/ml), sowie den Wachstumsfaktoren FGF-B (human recombinant basic FGF, Peprotech: 50 ng/ml) und PDGF-BB (human recombinant PDGF, Peprotech: 10 ng/ml) (unterstützen die Proliferation von Oligodendrozyten-Vorläuferzellen).
5. Serumfreies gliales Differenzierungsmedium: serumfreies Wachstumsmedium ohne Wachstumsfaktoren (FGF-B und PDGF-BB).
6. Trypsin/EDTA Lösung für hMSC (Lonza)
7. MSCGM (Mesenchymal Stem Cell Growth Medium) für hMSC (Lonza)
8. Accutase^{®} Lösung (Sigma)
9. Definiertes serumfreies Neuromedium: DMEM/Ham's F-12 (Sigma, mit L-Glutamin und 15 mM HEPES) supplementiert mit EGF (human recombinant epidermal growth factor, 20 ng/ml) und FGF-B (basic fibroblast growth factor, 20 ng/ml).
10. Serumfreies neuronales Differenzierungsmedium: Neurobasal Medium (Invitrogen) supplementiert mit 1% Glutamax (Invitrogen), 1% N2 Supplement (Invitrogen), B-27 Supplement 1:50 (Invitrogen), Gentamycin (25 µg/ml), Penicillin (100 units/ml) und Streptomycin (0,1 mg/ml), sowie dem Wachstumsfaktor BDNF (human recombinant brain-derived neurotrophic factor, 10 ng/ml).

### Verwendete Antikörper

Die R4- und R6-Antikörper sind erhältlich aus der Hybridomzelllinie tn-R4 und tn-R6, die am 02.12.05 unter den Hinterlegungsnummern DSM ACC2754 bzw. DSM ACC2753 bei der DSMZ (Deutsche Sammlung von Mikroorganismen und Zellkulturen GmbH) hinterlegt sind (siehe auch WO 2006/067094). V-5-Antikörper (Sigma, Cat. No. V8012), 04-Antikörper (Sigma, Cat. No. 07139)

### Beispiel 1: Herstellung eines humanen TNR-Cys (H-TNR-Cys) Fragments

Für die Herstellung eines rekombinanten eukaryotisch exprimierten H-TNR-Cys Fragments wird folgender Sequenzbereich ausgewählt:

| | |
|---|---|
| H-TNR-Cys: | bp 151-648 (von SEQ ID NO:1 Cystein-reiche Region) |
| Produktlänge: | 498 bp |

Zur Gewinnung humaner TN-R-spezifischer mRNA wird die menschliche Neuroblastomzellinie SH-SY5Y verwendet (Woodworth et al., 2004). Gesamt-RNA wird aus diesen Zellen unter Verwendung von Trizol-Reagenz (Invitrogen) gereinigt. Die cDNA-Synthese aus diesen RNA-Präparationen erfolgt unter Verwendung von "Random hexamer"-Primern mit Hilfe des SuperScript II Systems (Invitrogen). Zur Herstellung von H-TNR-Cys-spezifischem cDNA-Fragment werden folgende Primer verwendet.

| | |
|---|---|
| H-TNR-Cys-UP (SEQ ID NO:3): | bp 151-173:TCCATGATCAAGCCTTCAGAGTG |
| H-TNR-Cys-DO (SEQ ID NO:4): | bp 627-648: GATGCAGCCACAGGACTCAAAG |

Das gewonnene PCR-Fragment wird mit Hilfe des TOPO/TA Klonierungssystems (Invitrogen), das sich die überstehenden A-Reste der PCR-Produkte bei Verwendung von Taq-Polymerase zunutze macht, in den pcsecTag/FRT/V5-His-TOPO-Vektor (Invitrogen) kloniert. Dieser Vektor erlaubt nach stabiler Integration in eukaryontische FlpIn-Zellinien (siehe unten) die Sekretion des gewünschten, mit einem am C-Terminus mit einem 6xHis-Peptid versehenen Fragments in den Zellkulturüberstand. PolyHis-tragende Fragmente werden über Nickel-Chelatchromatographie aus gesammelten Zellkulturüberständen aufgereinigt.

Als Produzenten des H-TNR-Cys Fragments werden FlpIn 293 Zellen (Invitrogen) verwendet, die sich von der menschlichen Nierenzellinie HEK 293 ableiten. In FlpIn-Zellen ist das Plasmid pFRT/IacZEo (Invitrogen) stabil integriert. Dieser Vektor enthält eine für die Flp-Rekombinase spezifisch erkannte FRT-Region. Bei gleichzeitiger Transfektion der Flp-293-Zellen mit dem pOG44-Plasmid, das die Expression der Flp-Rekombinase ermöglicht und dem die Basensequenz des H-TNR-Cys Fragments tragenden pcsecTag/FRT/V5-His-TOPO-Vektor kommt es an der FRT-Region zu einem Einbau des pcsecTag/FRT/V5-His-TOPO-Vektors.

Zur bakteriellen Expression kann Cys-Fragment in einen prokaryotischen Expressionsvektor, z.B. dem Vektor pIN-III-ompA2 (SEQ ID NO:5; Adresse PubMed Nucleotide: NM_013045) kloniert. Das zellfrei synthetisierte Fremdgen (Cys-Region + His-Tag) verfügt über flankierende EcoRI-bzw. BamHI-spezifische Schnittstellen, die den Einbau des Fremdgens in die EcoRI-bzw. BamHI-Klonierungsstelle des pIN-III-ompA2-Vektor nach entsprechendem Verdau mit Restriktionsenzymen ermöglichen.

Die Expression des Proteins erfolgt im E.Coli-Stamm JA221 nach Vektortransformation. Die Selection von Transformanten geschieht durch Kultivierung in Ampicillin-haltigen Kulturmedien. Das Protein wird in den periplasmatischen Raum der E.Coli-Zelle sekretiert, dieser durch osmotischen Schock eröffnet und das Protein über einen C-terminalen His-Tag affinitätschromatographisch gereinigt.

Das über das FlpIn-Expressionssystem hergestellte H-TNR-Cys Fragment weist in SDS-PAGE ein Molekulargewicht von 34 kD auf und wird in Westernblot-Analysen von Tag-spezifischen (V5) und TN-R-spezifischen (R4) monoklonalen Antikörpern erkannt (Fig. 1A). Westernblot- und Funktionsanalysen rekombinanter H-TNR-Cys und H-TNR-S1-bis-5-Fragmente (S1 = 24 bis 328, S2 = 324 bis 1134, S3 = 112 bis 1358, S4 = 24 bis 506, S5 = 498 bis 955, S6 = 949 bis 1358 von SEQ ID NO:2, siehe auch WO 06/067094) weisen nach, dass der R4 Antikörper (1) ein Epitop in der Cys-Region (H-TNR-Cys Fragment) und der FNG-Domäne (H-TNR-S3 Fragment) von TN-R erkennt (Fig. 1A), (2) die Sulfatid-vermittelte Zelladhäsion auf TN-R/TN-R Fragmente blockiert (Fig. 1B) und an die Sulfatid-Bindungsstellen von TN-R bindet bzw. H-TNR-Cys und H-TNR-S3 Fragmente Sulfatide binden (Fig. 1C).

### Beispiel 2: Selektive Aufreinigung von Oligodendrozyten aus ZNS-Gewebe von Säugern mit Hilfe von rekombinanten H-TNR Fragmenten

Als Ausgangsmaterial wurden postnatale Mausgehirne (entweder das Gesamtgehirn oder isolierte Vorderhirnbereiche bzw. Präparationen des optischen Nervs) der Altersstufen Postnataltag 1 (P1) bis 4 (P4) verwendet. Isolierte Gehirnbereiche wurden nach mechanischer Zerkleinerung entsprechend der Herkunft bzw. des Alters mit 0,5 bis 1%iger (w/v) Trypsinlösung behandelt (P1 bis P2 Gehirne mit 0,5% (w/v) Trypsinlösung für 10 min bei Raumtemperatur (RT), P4 Gehirne mit 0,5% (w/v) Trypsinlösung für 15 min bei RT. Die Gewebeteile wurden nach Zugabe eines größeren Volumens HBSS bei 600 g für 5 Minuten bei 4°C pelletiert.

Zur Gewinnung von Einzelzellen wurden die pelletierten Gewebestücke in DNaseLösung aufgenommen und in einer Pasteurpipette mit verengtem Spitzendurchmesser mehrmals auf- und abpipettiert. Die erhaltene grobe Zellsuspension wurde in einem 5-10fachem Volumen an Support Medium verdünnt und 5 Minuten auf Eis inkubiert. Der die Einzelzellsuspension enthaltende Überstand wurde bei 600 g für 5 min bei 4°C zentrifugiert und die pelletierten Einzelzellen in serumfreiem Wachstumsmedium resuspendiert. Die so gewonnenen Einzelzellsuspensionen enthielten alle in den entsprechenden Gehirnen/Gehirnbereichen vorhandenen Zelltypen (Neurone, Astrozyten, Oligodendrozyten, Mikroglia, meningiale und endotheliale Zellen).

Zur Gewinnung reiner Oligodendrozytenpopulationen wurden die im letzten Abschnitt vorgestellten Einzelzellsuspensionen (1-2 x 10⁶ Zellen/ml in serumfreiem Medium) auf mit H-TNR Fragmenten beschichteten Zellkulturschalen (siehe unten) ausplattiert und für 8-20 Stunden in einem CO₂-Inkubator (5% CO₂) kultiviert. Nicht adhärente Zellen wurden mit HBSS weggespült und adhärente Zellen in serumfreiem Wachstumsmedium weiter kultiviert. Anschließend wurden die auf H-TNR Fragmenten adhärenten Zellen mit einem Sulfatid-spezifischen monoklonalen Maus-Antikörper (04; Bansal, R. et al., J. Neurosci. Res. 24:548-557 (1989)) inkubiert (30 min bei RT). Nach Fixierung der Zellen mit 4 % (v/v) Formaldehyd in PBS für 10 min bei RT wurde die Bindung des 04-Antikörpers auf die Zellen durch Inkubation mit Cyanin3- oder FITC-gekoppelten anti-Maus-Ig-Antikörpern (20 min bei RT) fluoreszenzmikroskopisch nachgewiesen. Auf dem Substrat verblieben 99+/-1% 04-färbbare Zellen, also nur Oligodendrozyten (Fig. 2).

Die durch dieses Einstufenverfahren gewonnenen Zellen konnten für weitere Verwendungszwecke durch Behandlung mit Accutase^{®} (Sigma) vom Substrat abgelöst und auf anderen Substraten/Plastikoberflachen bzw. in anderen definierten Medien zur Gewinnung von beispielsweise unreifen oder reifen, myelinkompetenten Oligodendrozyten weiter kultiviert werden. Für die Herstellung von Substraten aus H-TNR Fragmenten wurden Plastikoberflächen (Zellkulturschalen, -platten, etc.) mit H-TNR-Cys oder H-TNR-S3 Fragmenten (10 µg/ml in 0,1 M NaHCO₃) für 2 h bei RT inkubiert, mit PBS (2-3 mal) gewaschen und anschließend als Substrat für die Oligodendrozyten-Selektion verwendet. Die aus H-TNR Fragmenten hergestellten Substrate können nach Beschichtung auch eingetrocknet und bei -20°C steril gelagert werden, ohne dass die spezifischen adhäsiven Eigenschaften der Fragmente für Oligodendrozyten hierdurch verloren gingen.

### Beispiel 3: Differenzierungsverfahren für Gliazellen aus adulten humanen mesenchymalen Stammzellen (hMSC) mit Hilfe von rekombinanten H-TNR Fragmenten

Als Ausgangsmaterial wurden adulte humane mesenchymale Stammzellen (hMSC) aus dem Knochenmark (Lonza; CD105-, CD166-, CD29- und CD44-positiv) verwendet. Die aus normalem humanem Knochenmark isolierten Zellen wurden für 3 bis 4 Passagen in MSCGM vermehrt. Zur Gewinnung von Einzelzellen wurden die als Monolayer wachsenden Zellen mit Trypsin/EDTA Lösung dissoziiert, die Einzelzellsuspension nach Zugabe eines größeren Volumens MSCGM bei 600 g für 5 Minuten bei 4°C pelletiert und die pelletierten Einzelzellen in serumfreiem Wachstumsmedium resuspendiert. Zur Gewinnung glialer Zellpopulationen wurden Einzelzellsuspensionen (1-2 x 10⁶ Zellen/ml) auf unbeschichtete Schalen ausplattiert und für 6 bis 9 Tage in serumfreiem Wachstumsmedium in einem CO₂-Inkubator (5% CO₂) kultiviert. Das Zellkulturmedium wurde alle 2-3 Tage gewechselt. Anschließend wurden adhärente Zellen trypsinisiert, Einzelzellsuspensionen (1 x 10⁶ Zellen/ml) auf mit Laminin (Kontrollsubstrat) oder H-TNR Fragmenten beschichteten Schalen (wie oben beschrieben) ausplattiert und für weitere 2 bis 5 Tage in serumfreiem Wachstumsmedium kultiviert (Fig. 3A). Danach wurde das Wachstumsmedium durch gliales Differenzierungsmedium ersetzt und die Zellen wurden darin für 3 Tage weiter kultiviert. Anschließend wurden die auf den Substraten adhärenten Zellen mit 4 % (v/v) Formaldehyd und 5% (w/v) Sucrose in PBS für 30 min bei RT fixiert und mit Hilfe indirekter Immunfluoreszenz mit Neuron- (βIII tubulin) und Glia-spezifischen Antikörpern

(gegen Nestin, Sulfatid, Galactocerebrosid (GaIC) und GFAP) für 30 min bei RT (für Sulfatid und GaIC) angefärbt. Für den Nachweis von βIII Tubulin, Nestin und GFAP wurden fixierte Zellen zuerst mit einer 0,25%igen Triton X-100-Lösung in PBS permeabilisiert (20 min bei RT) und anschließend mit Molekül-spezifischen Antikörpern (abcam) über Nacht bei 4°C inkubiert. Die Bindung der entsprechenden Antikörper wurde durch Inkubation (30 min bei RT) mit Cyanin 3- oder ALEXA 488-gekoppelten sekundären Antikörpern fluoreszenzmikroskopisch visualisiert. Auf den H-TNR-S3 und H-TNR-Cys Substraten wurden 60% (auf H-TNR-S3) bis 90% (auf H-TNR-Cys) Sulfatid/GaIC-positive Zellen, also überwiegend Oligodendrozyten nachgewiesen, wobei die Überlebensrate oligodendroglial differenzierter Zellen auf H-TNR-Cys (60% der Ausgangszellzahl) 3fach höher als diese auf H-TNR-S3 war (Fig. 3B, 3D).

### Beispiel 4: Differenzierungsverfahren für neuronale Zellen aus adulten humanen mesenchymalen Stammzellen (hMSC) mit Hilfe von rekombinanten H-TNR Fragmenten

Als Ausgangsmaterial wurden adulte humane mesenchymale Stammzellen (hMSC) aus dem Knochenmark (Lonza; CD105-, CD166-, CD29- und CD44-positiv) verwendet. Die aus normalem humanem Knochenmark isolierten Zellen wurden für 3 Passagen in MSCGM vermehrt. Zellen von Monolayern wurden mit Trypsin/EDTA Lösung dissoziiert, die Einzelzellsuspension nach Zugabe eines größeren Volumens MSCGM bei 600 g für 5 Minuten bei 4°C pelletiert und die pelletierten Einzelzellen in serumfreiem Neuromedium resuspendiert. Zur Gewinnung neuronaler Zellpopulationen wurden Einzelzellsuspensionen (1-2 x 10⁵ Zellen/cm²) auf mit 0.8% (w/v) Agarose Typ VII (Sigma) beschichtete Schalen (5 ml/95 mm Schale) ausplattiert und für 3 bis 5 Tage bis zur Ausbildung von Neurosphären in Neuromedium in einem CO₂-Inkubator (5% CO₂) kultiviert. Das Zellkulturmedium wurde alle 2-3 Tage gewechselt. Anschließend wurden Neurosphären durch Zentrifugation pelletiert, mit Accutase^{®} (Sigma) für 15 min bei RT dissoziiert und in neuronales Differenzierungsmedium resuspendiert. Einzelzellsuspensionen (0.5 x 10⁶ Zellen/ml) wurden auf mit Laminin oder poly-D-Lysin (PDL, Kontrollsubstrate) und H-TNR Fragmenten beschichteten Schalen (wie oben beschrieben) ausplattiert und für 5 bis 10 Tage in neuronalem Differenzierungsmedium kultiviert. Das Zellkulturmedium wurde alle 2-3 Tage gewechselt. Anschließend wurden die auf den Substraten adhärenten Zellen mit 4 % (v/v) Formaldehyd in PBS für 30 min bei RT fixiert und mit Hilfe indirekter Immunfluoreszenzfärbung mit Neuron- und Glia-spezifischen Antikörpern (wie oben beschrieben) analysiert. Während auf H-TNR-S3 Substraten fast keine Stammzellen im neuronalen Differenzierungsmedium überlebten, konnten 80% der Zellen mit langen Axon-ähnlichen Fortsetzten auf H-TNR-Cys Substraten als βIII Tubulin-positiv und somit als Neurone/neuronale Vorläufer identifiziert werden. Hierbei betrug die Überlebensrate transdifferenzierter Zellen über 80% der aus Neurosphären stammender Ausgangszellzahl (Fig. 3C, 3D).

## Patentansprüche

1. Verfahren zur Isolierung und Reinigung von neuralen Zellen aus neuralem Primärgewebe von Vertebraten, umfassend die Selektion der Zellen aus einer Einzelzellsuspension mittels einer Tenascin-R-haltigen Sonde ("Tenascin-R-Sonde"), welche ein N-terminales Fragment von nativem Tenascin-R (TN-R) sowie Homologen und Fragmenten desselben und Fusionsproteine derartiger Verbindungen umfasst.

2. Verfahren nach Anspruch 1, wobei das N-terminale Tenascin-R-Fragment
(a) ein Tenascin-R-Fragment ist, das dem von Nukleotiden 151-648 der SEQ ID NO:1 kodierten humanen Tenascin-R-Fragment entspricht, vorzugsweise ein Fragment mit Aminosäureresten 24 bis 189 von SEQ ID NO:2 ist,
(b) ein Fragment von (a) bei dem bis zu 10, vorzugsweise bis zu 5 Aminosäureresten von N- und/oder C-Terminus abgespalten sind und/oder das wenigstens 150 Aminosäurereste aufweist,
(c) eine Substitutions-, Deletions- und/oder Additionsmutante von (a) oder (b) ist, oder
(d) ein Tenascin-R-Fusionsprotein mit einem wie vorstehend unter (a) bis (c) definiertem Tenascin-R-Fragment und einer funktionelle Komponente, umfassend eines oder mehrere weiterer funktioneller Peptide oder Proteine, ist oder ein Tenascin-R-Fusionsprotein ist, das aus zwei oder mehreren, bevorzugt zwei oder drei der wie vorstehend unter (a) bis (c) definierten funktionellen Tenascin-R-Fragmenten zusammengesetzt ist.

3. Verfahren nach Anspruch 1 oder 2, wobei
(i) das TN-R-Fragment aus Vertebraten, bevorzugt aus Fischen, Reptilien, Amphibien, Vögeln und Säugetieren, besonders bevorzugt aus Hai, Karpfen, Huhn, Nagern einschließlich Maus und Ratte, Rind, Schwein und Mensch, ganz besonders bevorzugt aus Nagern und Mensch stammt; und/oder
(ii) die Tenascin-R-Sonde weitere funktionelle Peptid- oder Proteinsequenzen enthält und/oder an einen Träger gekoppelt ist; und/oder
(iii) das Tenascin-R-Fragment ein Peptid mit der Sequenz der Aminosäurereste 24 bis 189 von SEQ ID NO:2 ist.

4. Verfahren nach einem oder mehreren der Ansprüche 1 bis 3, wobei
(i) das Verfahren zur Isolierung und Reinigung von glialen Zellen, bevorzugt von Oligodendrozyten, geeignet ist; und/oder
(ii) das Verfahren zur Transdifferenzierung adulter humaner mesenchymaler Stammzellen in neuronale oder oligodendrogliale Zellen geeignet ist; und/oder
(ii) das Vertebraten-Primärgewebe aus niederen oder höheren Vertebraten einschließlich Fischen, Amphibien, Vögeln und Säugetieren, besonders bevorzugt aus Hai, Karpfen, Huhn, Nagern einschließlich Maus und Ratte, Rind, Schwein und Mensch, ganz besonders bevorzugt aus Nagern und Mensch stammt; und/oder
(iii) die Isolierung der Zellen durch selektive Substratadhäsion an die Tenascin-R-Sonde und/oder in einem einzigen Reinigungsschritt erfolgt.

5. Verfahren nach einem oder mehreren der Ansprüche 1 bis 4, wobei die Einzelzellsuspension
(i) aus embryonalem, fötalem, früh- oder spätpostnatalem und/oder adultem Gewebe hergestellt wird; und/oder
(ii) aus Gewebe aus verschiedenen Bereichen des Nervensystems, bevorzugt aus Nervengewebe oder Gehirn, besonders bevorzugt aus Gehirn, Rückenmark oder optischem Nerv hergestellt wird; und/oder
(iii) Zellen einer oder mehrerer Differenzierungsstufen, bevorzugt einer einzigen Differenzierungsstufe, enthält.

6. Verfahren nach einem oder mehreren der Ansprüche 1 bis 5, wobei
(i) die Tenascin-R-Sonde durch nichtkovalente Wechselwirkungen, einschließlich Wechselwirkung mit TN-R-spezifischen Antikörpern usw., oder durch eine andere adäquate Kopplungstechnik, welche die Spezifität der Tenascin-R-Sonde nicht verändert, einschließlich kovalente Vernetzung usw., an ein Trägermaterial gebunden ist; und/oder
(ii) die Einzelzellsuspension mit der Tenascin-R-Sonde in Kontakt gebracht wird, so dass in der Einzelzellsuspension vorhandene Tenascin-R-bindende Zellen an die Sonde gebunden werden; und/oder
(iii) durch spezifische Bindung neuraler Stammzellen aus der Einzelzellsuspension an die Tenascin-R-Sonde eine Isolierung dieser Zellen aus der Zellkultur erfolgt, die nicht gebundenen Zellen entfernt werden und optional anschließend die über die Tenascin-R-Sonde an das Trägermaterial gebundenen Zellen durch Trypsinierung, Inkubation mit Accutase^{®} oder ein anderes adäquates Verfahren vom Trägermaterial abgelöst werden; und/oder
(iv) die gebundenen Zellen durch immunologische Methoden nachgewiesen werden; und/oder
(v) das Verfahren *in vitro* erfolgt.

7. Verfahren nach einem oder mehreren der Ansprüche 1 bis 6, das
(i) zur Gewinnung von neuralen Zellen, insbesondere von Oligodendrozyten, für die Anzucht differenzierter Zellen, insbesondere neuraler Zellen, in neurobiologischen und zellphysiologischen Untersuchungen, in der biologischen und klinischen Forschung und für diagnostische und therapeutische Verfahren *in vitro* und *in vivo,* insbesondere für die Herstellung eines Medikaments zur Zelltherapie und zur Therapie von neurodegenerativen Krankheiten; und/oder
(ii) zum Nachweis von neurodegenerativen Krankheiten
geeignet ist.

8. Tenascin-R-Fragment oder Tenascin-R-Fusionsprotein wie in Anspruch 1 bis 3 definiert.

9. DNA, die für ein Tenascin-R-Fragment oder Tenascin-R-Fusionsprotein nach Anspruch 8 kodiert.

10. Vektor, der eine DNA nach Anspruch 9 umfasst.

11. Wirtsorganismus, der mit einem Vektor nach Anspruch 10 transformiert/transfiziert ist und/oder eine DNA nach Anspruch 9 aufweist.

12. Verfahren zur Herstellung eines Tenascin-R-Fragments oder Tenascin-R-Fusionsproteins nach Anspruch 8, umfassend das Kultivieren des Wirtsorganismus nach Anspruch 11.

13. Antikörper, der durch Immunisierung eines geeigneten Wirtsorganismus mit einem Tenascin-R-Fragment nach Anspruch 8 erhältlich ist.

14. Antikörper nach Anspruch 13,
(i) der durch Immunisierung mit Tenascin-R-Fragmenten nach Anspruch 9 von wenigstens zwei unterschiedlichen Spezies, insbesondere mit solchen Tenascin-R-Fragmenten von wenigstens zwei unterschiedlichen Vertebraten erhältlich ist und/oder welcher an das N-terminales Tenascin-R von wenigstens zwei unterschiedlichen Spezies, insbesondere von wenigstens zwei unterschiedlichen Vertebraten bindet; und/oder
(ii) der monoklonal ist.

15. Zelllinie oder Hybridomzelllinie, die einen monoklonalen Antikörper nach Anspruch 14 produziert.

16. Verwendung des Antikörpers nach Anspruch 13 oder 14
(i) zum immunochemischen Nachweis von TN-R;
(ii) zur Hemmung der Wirkung von TN-R;
(iii) zur Beeinflussung der Neuralentwicklung und
(iv) zur Herstellung von Medikamenten zur Therapie und Prophylaxe traumatischer Nervenläsionen und von Medikamenten zur gezielten Beeinflussung der Neuralentwicklung.

17. Kit zur Isolierung und Reinigung von neuralen Zellen, insbesondere von Oligodendrozyten, nach einem oder mehreren der Verfahren der Ansprüche 1 bis 8, insbesondere enthaltend
(i) eine wie in Ansprüchen 1 bis 3 definierte Tenascin-R-Sonde, und/oder
(ii) einen Vektor, der für die in (i) definierte Tenascin-R-Sonde kodiert, und/oder
(iii) eine Stammkultur einer Zelllinie, die dazu geeignet ist, die wie in Ansprüchen 1 bis 3 definierte Tenascin-R-Sonde zu exprimieren.

18. Kit nach Anspruch 17, wobei
(i) die Sonde an ein Trägermaterial gebunden ist; und/oder
(ii) der Kit weiterhin Tenascin-R-Antikörper, insbesondere einen wie in Ansprüchen 14 bis 16 definierten Antikörper umfasst; und/oder
(iii) der Kit weiterhin enzymatische Lösungen zur Zelldissoziierung, Puffer und/oder Kulturmedien umfasst.

19. Verwendung eines Tenascin-R-Fragments oder eines Tenascin-R-Fusionsproteins wie in Anspruch 8 definiert, zur Gewinnung von neuralen Zellen, insbesondere von Oligodendrozyten, für die Anzucht differenzierter Zellen, insbesondere neuraler Zellen, in neurobiologischen und zellphysiologischen Untersuchungen, in der biologischen und klinischen Forschung und für diagnostische und therapeutische Verfahren *in vitro* und *in vivo,* insbesondere für die Herstellung eines Medikaments zur Zelltherapie und zur Therapie von neurodegenerativen Krankheiten, die mit einem Verlust von Oligodendrozyten oder Myelin einhergehen, insbesondere Multipler Sklerose und periventrikulärer Leukomalazie (PVL).

20. Verfahren zur Herstellung von Oligodendrozyten aus isolierten Stammzellen *in vitro* durch Inkubation der Stammzellen in Anwesenheit eines Tenascin-R-Fragments oder eines Tenascin-R-Fusionsproteins wie in Anspruch 8 definiert.

21. Verfahren nach Anspruch 20, wobei die isolierten Stammzellen neurale oder nicht-neurale Stammzellen sind, welche das Potential zur Sulfatid-Expression besitzen.

22. Verfahren zur Zelltherapie oder zur Therapie von neurodegenerativen Krankheiten, die mit einem Verlust von Oligodendrozyten oder Myelin einhergehen, insbesondere von Multipler Sklerose und periventrikulärer Leukomalazie (PVL), umfassend die Gabe eines Tenascin-R-Fragments oder eines Tenascin-R-Fusionsproteins wie in Anspruch 8 definiert, an einen menschlichen oder tierischen Patienten.

23. Verfahren zur Therapie und Prophylaxe traumatischer Nervenläsionen oder zur gezielten Beeinflussung der Neuralentwicklung, umfassend die Verabreichung einer pharmakologisch ausreichenden Menge des Antikörpers nach Anspruch 14 oder 15 an einen menschlichen oder tierischen Patienten, der einer solchen Behandlung bedarf.


SEQUENCE LISTING

<110>  Rheinische-Friedrich-Wilhelms-Univerrsitaet Bonn

<120>  Verfahren  zur Isolierung neuraler Zellen mit
       Tenascin-R-Verbindungen

<130>  072523ep

<160>  10

<170>  PatentIn version 3.3

<210>  1
<211>  4716
<212>  DNA
<213>  Homo sapiens


<220>
<221>  CDS
<222>  (82)..(4158)

<220>
<221>  sig_peptide
<222>  (82)..(150)

<400>  1
ccttggtttc cgttgcagat tcccacaact ccatgctgtg tgctgcaggc tggtcctgaa      60

cccagatctc tggctgagag g atg ggg gca gat ggg gaa aca gtg gtt ctg      111
                        Met Gly Ala Asp Gly Glu Thr Val Val Leu
                        1               5                   10

aag aac atg ctc att ggc gtc aac ctg atc ctt ctg ggc tcc atg atc      159
Lys Asn Met Leu Ile Gly Val Asn Leu Ile Leu Leu Gly Ser Met Ile
                15                  20                  25

aag cct tca gag tgt cag ctg gag gtc acc aca gaa agg gtc cag aga      207
Lys Pro Ser Glu Cys Gln Leu Glu Val Thr Thr Glu Arg Val Gln Arg
            30                  35                  40

cag tca gtg gag gag gag gga ggc att gcc aac tac aac acg tcc agc      255
Gln Ser Val Glu Glu Glu Gly Gly Ile Ala Asn Tyr Asn Thr Ser Ser
        45                  50                  55

aaa gag cag cct gtg gtc ttc aac cac gtg tac aac att aac gtg ccc      303
Lys Glu Gln Pro Val Val Phe Asn His Val Tyr Asn Ile Asn Val Pro
    60                  65                  70

ttg gac aac ctc tgc tcc tca ggg cta gag gcc tct gct gag cag gag      351
Leu Asp Asn Leu Cys Ser Ser Gly Leu Glu Ala Ser Ala Glu Gln Glu
75                  80                  85                  90

gtg agt gca gaa gac gag act ctg gca gag tac atg ggc cag acc tca      399
Val Ser Ala Glu Asp Glu Thr Leu Ala Glu Tyr Met Gly Gln Thr Ser
                95                  100                 105

gac cac gag agc cag gtc acc ttt aca cac agg atc aac ttc ccc aaa      447
Asp His Glu Ser Gln Val Thr Phe Thr His Arg Ile Asn Phe Pro Lys
            110                 115                 120

aag gcc tgt cca tgt gcc agt tca gcc cag gtg ctg cag gag ctg ctg      495

Lys Ala Cys Pro Cys Ala Ser Ser Ala Gln Val Leu Gln Glu Leu Leu
        125                 130                 135

agc cgg atc gag atg ctg gag agg gag gtg tcg gtg ctg cga gac cag       543
Ser Arg Ile Glu Met Leu Glu Arg Glu Val Ser Val Leu Arg Asp Gln
    140                 145                 150

tgc aac gcc aac tgc tgc caa gaa agt gct gcc aca gga caa ctg gac       591
Cys Asn Ala Asn Cys Cys Gln Glu Ser Ala Ala Thr Gly Gln Leu Asp
155                 160                 165                 170

tat atc cct cac tgc agt ggc cac ggc aac ttt agc ttt gag tcc tgt       639
Tyr Ile Pro His Cys Ser Gly His Gly Asn Phe Ser Phe Glu Ser Cys
                175                 180                 185

ggc tgc atc tgc aac gaa ggc tgg ttt ggc aag aat tgc tcg gag ccc       687
Gly Cys Ile Cys Asn Glu Gly Trp Phe Gly Lys Asn Cys Ser Glu Pro
            190                 195                 200

tac tgc ccg ctg ggt tgc tcc agc cgg ggg gtg tgt gtg gat ggc cag       735
Tyr Cys Pro Leu Gly Cys Ser Ser Arg Gly Val Cys Val Asp Gly Gln
        205                 210                 215

tgc atc tgt gac agc gaa tac agc ggg gat gac tgt tcc gaa ctc cgg       783
Cys Ile Cys Asp Ser Glu Tyr Ser Gly Asp Asp Cys Ser Glu Leu Arg
    220                 225                 230

tgc cca aca gac tgc agc tcc cgg ggg ctc tgc gtg gac ggg gag tgt       831
Cys Pro Thr Asp Cys Ser Ser Arg Gly Leu Cys Val Asp Gly Glu Cys
235                 240                 245                 250

gtc tgt gaa gag ccc tac act ggc gag gac tgc agg gaa ctg agg tgc       879
Val Cys Glu Glu Pro Tyr Thr Gly Glu Asp Cys Arg Glu Leu Arg Cys
                255                 260                 265

cct ggg gac tgt tcg ggg aag ggg aga tgt gcc aac ggt acc tgt tta       927
Pro Gly Asp Cys Ser Gly Lys Gly Arg Cys Ala Asn Gly Thr Cys Leu
            270                 275                 280

tgc gag gag ggc tac gtt ggt gag gac tgc ggc cag cgg cag tgt ctg       975
Cys Glu Glu Gly Tyr Val Gly Glu Asp Cys Gly Gln Arg Gln Cys Leu
        285                 290                 295

aat gcc tgc agt ggg cga gga caa tgt gag gag ggg ctc tgc gtc tgt      1023
Asn Ala Cys Ser Gly Arg Gly Gln Cys Glu Glu Gly Leu Cys Val Cys
    300                 305                 310

gaa gag ggc tac cag ggc cct gac tgc tca gca gtt gcc cct cca gag      1071
Glu Glu Gly Tyr Gln Gly Pro Asp Cys Ser Ala Val Ala Pro Pro Glu
315                 320                 325                 330

gac ttg cga gtg gct ggt atc agc gac agg tcc att gag ctg gaa tgg      1119
Asp Leu Arg Val Ala Gly Ile Ser Asp Arg Ser Ile Glu Leu Glu Trp
                335                 340                 345

gac ggg ccg atg gca gtg acg gaa tat gtg atc tct tac cag ccg acg      1167
Asp Gly Pro Met Ala Val Thr Glu Tyr Val Ile Ser Tyr Gln Pro Thr
            350                 355                 360

gcc ctg ggg ggc ctc cag ctc cag cag cgg gtg cct gga gat tgg agt      1215
Ala Leu Gly Gly Leu Gln Leu Gln Gln Arg Val Pro Gly Asp Trp Ser
        365                 370                 375

ggt gtc acc atc acg gag ctg gag cca ggt ctc acc tac aac atc agc      1263
Gly Val Thr Ile Thr Glu Leu Glu Pro Gly Leu Thr Tyr Asn Ile Ser
    380                 385                 390

gtc tac gct gtc att agc aac atc ctc agc ctt ccc atc act gcc aag      1311
Val Tyr Ala Val Ile Ser Asn Ile Leu Ser Leu Pro Ile Thr Ala Lys
395                 400                 405                 410

gtg gcc acc cat ctc tcc act cct caa ggg cta caa ttt aag acg atc      1359
Val Ala Thr His Leu Ser Thr Pro Gln Gly Leu Gln Phe Lys Thr Ile
                415                 420                 425

aca gag acc acc gtg gag gtg cag tgg gag ccc ttc tca ttt tcc ttc      1407
Thr Glu Thr Thr Val Glu Val Gln Trp Glu Pro Phe Ser Phe Ser Phe
            430                 435                 440

gat ggg tgg gaa atc agc ttc att cca aag aac aat gaa ggg gga gtg      1455
Asp Gly Trp Glu Ile Ser Phe Ile Pro Lys Asn Asn Glu Gly Gly Val
        445                 450                 455

att gct cag gtc ccc agc gat gtt acg tcc ttt aac cag aca gga cta      1503
Ile Ala Gln Val Pro Ser Asp Val Thr Ser Phe Asn Gln Thr Gly Leu
    460                 465                 470

aag cct ggg gag gaa tac att gtc aat gtg gtg gct ctg aaa gaa cag      1551
Lys Pro Gly Glu Glu Tyr Ile Val Asn Val Val Ala Leu Lys Glu Gln
475                 480                 485                 490

gcc cgc agc ccc cct acc tcg gcc agc gtc tcc aca gtc att gac ggc      1599
Ala Arg Ser Pro Pro Thr Ser Ala Ser Val Ser Thr Val Ile Asp Gly
                495                 500                 505

ccc acg cag atc ctg gtt cgc gat gtc tcg gac acc gtg gct ttt gtg      1647
Pro Thr Gln Ile Leu Val Arg Asp Val Ser Asp Thr Val Ala Phe Val
            510                 515                 520

gag tgg att ccc cct cga gcc aaa gtc gat ttc att ctt ttg aaa tat      1695
Glu Trp Ile Pro Pro Arg Ala Lys Val Asp Phe Ile Leu Leu Lys Tyr
        525                 530                 535

ggc ctg gtg ggc ggg gaa ggt ggg agg acc acc ttc cgg ctg cag cct      1743
Gly Leu Val Gly Gly Glu Gly Gly Arg Thr Thr Phe Arg Leu Gln Pro
    540                 545                 550

ccc ctg agc caa tac tca gtg cag gcc ctg cgg cct ggc tcc cga tac      1791
Pro Leu Ser Gln Tyr Ser Val Gln Ala Leu Arg Pro Gly Ser Arg Tyr
555                 560                 565                 570

gag gtg tca gtc agt gcc gtc cga ggg acc aac gag agc gat tct gcc      1839
Glu Val Ser Val Ser Ala Val Arg Gly Thr Asn Glu Ser Asp Ser Ala
                575                 580                 585

acc act cag ttc aca aca gag atc gat gcc ccc aag aac ttg cga gtt      1887
Thr Thr Gln Phe Thr Thr Glu Ile Asp Ala Pro Lys Asn Leu Arg Val
            590                 595                 600

ggt tct cgc aca gca acc agc ctt gac ctc gag tgg gat aac agt gaa      1935
Gly Ser Arg Thr Ala Thr Ser Leu Asp Leu Glu Trp Asp Asn Ser Glu
        605                 610                 615

gcc gaa gtt cag gag tac aag gtt gtg tac agc acc ctg gcg ggt gag      1983
Ala Glu Val Gln Glu Tyr Lys Val Val Tyr Ser Thr Leu Ala Gly Glu
    620                 625                 630

caa tat cat gag gta ctg gtc ccc agg ggc att ggt cca acc acc agg      2031
Gln Tyr His Glu Val Leu Val Pro Arg Gly Ile Gly Pro Thr Thr Arg
635                 640                 645                 650

gcc acc ctg aca gat ctg gta cct ggc act gag tat gga gtt gga ata      2079
Ala Thr Leu Thr Asp Leu Val Pro Gly Thr Glu Tyr Gly Val Gly Ile
                655                 660                 665

tct gcc gtc atg aac tca cag caa agc gtg cca gcc acc atg aat gcc      2127
Ser Ala Val Met Asn Ser Gln Gln Ser Val Pro Ala Thr Met Asn Ala
            670                 675                 680

agg act gaa ctt gac agt ccc cga gac ctc atg gtg aca gcc tcc tcg      2175
Arg Thr Glu Leu Asp Ser Pro Arg Asp Leu Met Val Thr Ala Ser Ser
        685                 690                 695

gag acc tcc atc tcc ctc atc tgg acc aag gcc agt ggc ccc att gac      2223
Glu Thr Ser Ile Ser Leu Ile Trp Thr Lys Ala Ser Gly Pro Ile Asp
    700                 705                 710

cac tac cga att acc ttt acc cca tcc tct ggg att gcc tca gaa gtc      2271
His Tyr Arg Ile Thr Phe Thr Pro Ser Ser Gly Ile Ala Ser Glu Val
715                 720                 725                 730

acc gta ccc aag gac agg acc tca tac aca cta aca gat cta gag cct      2319
Thr Val Pro Lys Asp Arg Thr Ser Tyr Thr Leu Thr Asp Leu Glu Pro
                735                 740                 745

ggg gca gag tac atc att tcc gtc act gct gag agg ggt cgg cag cag      2367
Gly Ala Glu Tyr Ile Ile Ser Val Thr Ala Glu Arg Gly Arg Gln Gln
            750                 755                 760

agc ttg gag tcc act gtg gat gct ttc aca ggc ttc cgt ccc atc tct      2415
Ser Leu Glu Ser Thr Val Asp Ala Phe Thr Gly Phe Arg Pro Ile Ser
        765                 770                 775

cat ctg cac ttt tct cat gtg acc tcc tcc agt gtg aac atc act tgg      2463
His Leu His Phe Ser His Val Thr Ser Ser Ser Val Asn Ile Thr Trp
    780                 785                 790

agt gat cca tct ccc cca gca gac aga ctc att ctt aac tac agc ccc      2511
Ser Asp Pro Ser Pro Pro Ala Asp Arg Leu Ile Leu Asn Tyr Ser Pro
795                 800                 805                 810

agg gat gag gag gaa gag atg atg gag gtc tcc ctg gat gcc acc aag      2559
Arg Asp Glu Glu Glu Glu Met Met Glu Val Ser Leu Asp Ala Thr Lys
                815                 820                 825

agg cat gct gtc ctg atg ggc ctg caa cca gcc aca gag tat att gtg      2607
Arg His Ala Val Leu Met Gly Leu Gln Pro Ala Thr Glu Tyr Ile Val
            830                 835                 840

aac ctt gtg gct gtc cat ggc aca gtg acc tct gag ccc att gtg ggc      2655
Asn Leu Val Ala Val His Gly Thr Val Thr Ser Glu Pro Ile Val Gly
        845                 850                 855

tcc atc acc aca gga att gat ccc cca aaa gac atc aca att agc aat      2703
Ser Ile Thr Thr Gly Ile Asp Pro Pro Lys Asp Ile Thr Ile Ser Asn
    860                 865                 870

gtg acc aag gac tca gtg atg gtc tcc tgg agc cct cct gtt gca tct      2751
Val Thr Lys Asp Ser Val Met Val Ser Trp Ser Pro Pro Val Ala Ser

875                 880                 885                 890

ttc gat tac tac cga gta tca tat cga ccc acc caa gtg gga cga cta     2799
Phe Asp Tyr Tyr Arg Val Ser Tyr Arg Pro Thr Gln Val Gly Arg Leu
                895                 900                 905

gac agc tca gtg gtg ccc aac act gtg aca gaa ttc acc atc acc aga     2847
Asp Ser Ser Val Val Pro Asn Thr Val Thr Glu Phe Thr Ile Thr Arg
            910                 915                 920

ctg aac cca gct acc gaa tac gaa atc agc ctc aac agc gtg cgg ggc     2895
Leu Asn Pro Ala Thr Glu Tyr Glu Ile Ser Leu Asn Ser Val Arg Gly
        925                 930                 935

agg gag gaa agc gag cgc atc tgt act ctt gtg cac aca gcc atg gac     2943
Arg Glu Glu Ser Glu Arg Ile Cys Thr Leu Val His Thr Ala Met Asp
    940                 945                 950

aac cct gtg gat ctg att gct acc aat atc act cca aca gaa gcc ctg     2991
Asn Pro Val Asp Leu Ile Ala Thr Asn Ile Thr Pro Thr Glu Ala Leu
955                 960                 965                 970

ctg cag tgg aag gca cca gtg ggt gag gtg gag aac tac gtc att gtt     3039
Leu Gln Trp Lys Ala Pro Val Gly Glu Val Glu Asn Tyr Val Ile Val
                975                 980                 985

ctt aca cac ttt gca gtc gct gga gag acc atc ctt gtt gac  gga gtc     3087
Leu Thr His Phe Ala Val Ala Gly Glu Thr Ile Leu Val Asp  Gly Val
            990                 995                 1000

agt gag gaa  ttt cgg ctt gtt gac  ctg ctt cct agc acc  cac tat     3132
Ser Glu Glu  Phe Arg Leu Val Asp  Leu Leu Pro Ser Thr  His Tyr
    1005                 1010                 1015

act gcc acc  atg tat gcc acc aat  gga cct ctc acc agt  ggc acc     3177
Thr Ala Thr  Met Tyr Ala Thr Asn  Gly Pro Leu Thr Ser  Gly Thr
    1020                 1025                 1030

atc agc acc  aac ttt tct act ctc  ctg gac cct ccg gca  aac ctg     3222
Ile Ser Thr  Asn Phe Ser Thr Leu  Leu Asp Pro Pro Ala  Asn Leu
    1035                 1040                 1045

aca gcc agt  gaa gtc acc aga caa  agt gcc ctg atc tcc  tgg cag     3267
Thr Ala Ser  Glu Val Thr Arg Gln  Ser Ala Leu Ile Ser  Trp Gln
    1050                 1055                 1060

cct ccc agg  gca gag att gaa aat  tat gtc ttg acc tac  aaa tcc     3312
Pro Pro Arg  Ala Glu Ile Glu Asn  Tyr Val Leu Thr Tyr  Lys Ser
    1065                 1070                 1075

acc gac gga  agc cgc aag gag ctg  att gtg gat gca gaa  gac acc     3357
Thr Asp Gly  Ser Arg Lys Glu Leu  Ile Val Asp Ala Glu  Asp Thr
    1080                 1085                 1090

tgg att cga  ctg gag ggc ctg ttg  gag aac aca gac tac  acg gtg     3402
Trp Ile Arg  Leu Glu Gly Leu Leu  Glu Asn Thr Asp Tyr  Thr Val
    1095                 1100                 1105

ctc ctg cag  gca gca cag gac acc  acg tgg agc agc atc  acc tcc     3447
Leu Leu Gln  Ala Ala Gln Asp Thr  Thr Trp Ser Ser Ile  Thr Ser
    1110                 1115                 1120

acc gct ttc  acc aca gga ggc cgg  gtg ttc cct cat ccc  caa gac     3492

Thr Ala Phe Thr Thr Gly Gly Arg Val Phe Pro His Pro Gln Asp
        1125                1130                1135

tgt gcc cag cat ttg atg aat gga gac act ttg agt ggg gtt tac      3537
Cys Ala Gln His Leu Met Asn Gly Asp Thr Leu Ser Gly Val Tyr
        1140                1145                1150

ccc atc ttc ctc aat ggg gag ctg agc cag aaa tta caa gtg tac      3582
Pro Ile Phe Leu Asn Gly Glu Leu Ser Gln Lys Leu Gln Val Tyr
        1155                1160                1165

tgt gat atg acc acc gac ggg ggc ggc tgg att gta ttc cag agg      3627
Cys Asp Met Thr Thr Asp Gly Gly Gly Trp Ile Val Phe Gln Arg
        1170                1175                1180

cgg cag aat ggc caa act gat ttt ttc cgg aaa tgg gct gat tac      3672
Arg Gln Asn Gly Gln Thr Asp Phe Phe Arg Lys Trp Ala Asp Tyr
        1185                1190                1195

cgt gtt ggc ttc ggg aac gtg gag gat gag ttc tgg ctg ggg ctg      3717
Arg Val Gly Phe Gly Asn Val Glu Asp Glu Phe Trp Leu Gly Leu
        1200                1205                1210

gac aat ata cac agg atc aca tcc cag ggc cgc tat gag ctg cgc      3762
Asp Asn Ile His Arg Ile Thr Ser Gln Gly Arg Tyr Glu Leu Arg
        1215                1220                1225

gtg gac atg cgg gat ggc cag gag gcc gcc ttc gcc tcc tac gac      3807
Val Asp Met Arg Asp Gly Gln Glu Ala Ala Phe Ala Ser Tyr Asp
        1230                1235                1240

agg ttc tct gtc gag gac agc aga aac ctg tac aaa ctc cgc ata      3852
Arg Phe Ser Val Glu Asp Ser Arg Asn Leu Tyr Lys Leu Arg Ile
        1245                1250                1255

gga agc tac aac ggc act gcg ggg gac tcc ctc agc tat cat caa      3897
Gly Ser Tyr Asn Gly Thr Ala Gly Asp Ser Leu Ser Tyr His Gln
        1260                1265                1270

gga cgc cct ttc tcc aca gag gat aga gac aat gat gtt gca gtg      3942
Gly Arg Pro Phe Ser Thr Glu Asp Arg Asp Asn Asp Val Ala Val
        1275                1280                1285

act aac tgt gcc atg tcg tac aag gga gca tgg tgg tat aag aac      3987
Thr Asn Cys Ala Met Ser Tyr Lys Gly Ala Trp Trp Tyr Lys Asn
        1290                1295                1300

tgc cac cgg acc aac ctc aat ggg aag tac ggg gag tcc agg cac      4032
Cys His Arg Thr Asn Leu Asn Gly Lys Tyr Gly Glu Ser Arg His
        1305                1310                1315

agt cag ggc atc aac tgg tac cat tgg aaa ggc cat gag ttc tcc      4077
Ser Gln Gly Ile Asn Trp Tyr His Trp Lys Gly His Glu Phe Ser
        1320                1325                1330

atc ccc ttt gtg gaa atg aag atg cgc ccc tac aac cac cgt ctc      4122
Ile Pro Phe Val Glu Met Lys Met Arg Pro Tyr Asn His Arg Leu
        1335                1340                1345

atg gca ggg aga aaa cgg cag tcc tta cag ttc tga gcagtgggcg      4168
Met Ala Gly Arg Lys Arg Gln Ser Leu Gln Phe
        1350                1355

gctgcaagcc aaccaatatt ttctgtcatt tgtttgtatt ttataatatg aaacaagggg 4228

ggagggtaat agcaatgtgt tttgcaacat attaagagta tgtgaaggaa gcagggatgt 4288

cgcaggaatc cgctggctaa catctgctct tggtttctgc tgccctggag cctgaccctc 4348

agtctccatt ctccctccta cccaggcctc ctcaaccttc acctcctttc ccaccaagga 4408

ggagaagtag gaagttttct taaagggcca attcaaagcc aagtcgtggg gtgcagattg 4468

ttatggtgac aggcacacac atttttctac ccttcttctg agatgtcctc tgccttccag 4528

gtatttgtga ttttgtcaca gcctgacatg gccaggttct cacactggcc cagagaaaag 4588

agcctcagca agagagtttt gccaacaatt ccccttaaaa ggaaacagat caactacacc 4648

gcatcccaac aacccaggtt cttttccttc cttccttcct tcctcccttc cttctttcct 4708

gccttccc 4716

<210> 2
<211> 1358
<212> PRT
<213> Homo sapiens

<400> 2

Met Gly Ala Asp Gly Glu Thr Val Val Leu Lys Asn Met Leu Ile Gly
1               5                   10                  15

Val Asn Leu Ile Leu Leu Gly Ser Met Ile Lys Pro Ser Glu Cys Gln
            20                  25                  30

Leu Glu Val Thr Thr Glu Arg Val Gln Arg Gln Ser Val Glu Glu Glu
        35                  40                  45

Gly Gly Ile Ala Asn Tyr Asn Thr Ser Ser Lys Glu Gln Pro Val Val
    50                  55                  60

Phe Asn His Val Tyr Asn Ile Asn Val Pro Leu Asp Asn Leu Cys Ser
65                  70                  75                  80

Ser Gly Leu Glu Ala Ser Ala Glu Gln Glu Val Ser Ala Glu Asp Glu
                85                  90                  95

Thr Leu Ala Glu Tyr Met Gly Gln Thr Ser Asp His Glu Ser Gln Val
            100                 105                 110

Thr Phe Thr His Arg Ile Asn Phe Pro Lys Lys Ala Cys Pro Cys Ala
        115                 120                 125

Ser Ser Ala Gln Val Leu Gln Glu Leu Leu Ser Arg Ile Glu Met Leu
    130                 135                 140

Glu Arg Glu Val Ser Val Leu Arg Asp Gln Cys Asn Ala Asn Cys Cys
145                 150                 155                 160

Gln Glu Ser Ala Ala Thr Gly Gln Leu Asp Tyr Ile Pro His Cys Ser
                165                 170                 175

Gly His Gly Asn Phe Ser Phe Glu Ser Cys Gly Cys Ile Cys Asn Glu
            180                 185                 190

Gly Trp Phe Gly Lys Asn Cys Ser Glu Pro Tyr Cys Pro Leu Gly Cys
        195                 200                 205

Ser Ser Arg Gly Val Cys Val Asp Gly Gln Cys Ile Cys Asp Ser Glu
    210                 215                 220

Tyr Ser Gly Asp Asp Cys Ser Glu Leu Arg Cys Pro Thr Asp Cys Ser
225                 230                 235                 240

Ser Arg Gly Leu Cys Val Asp Gly Glu Cys Val Cys Glu Glu Pro Tyr
                245                 250                 255

Thr Gly Glu Asp Cys Arg Glu Leu Arg Cys Pro Gly Asp Cys Ser Gly
            260                 265                 270

Lys Gly Arg Cys Ala Asn Gly Thr Cys Leu Cys Glu Glu Gly Tyr Val
        275                 280                 285

Gly Glu Asp Cys Gly Gln Arg Gln Cys Leu Asn Ala Cys Ser Gly Arg
    290                 295                 300

Gly Gln Cys Glu Glu Gly Leu Cys Val Cys Glu Glu Gly Tyr Gln Gly
305                 310                 315                 320

Pro Asp Cys Ser Ala Val Ala Pro Pro Glu Asp Leu Arg Val Ala Gly
                325                 330                 335

Ile Ser Asp Arg Ser Ile Glu Leu Glu Trp Asp Gly Pro Met Ala Val
            340                 345                 350

Thr Glu Tyr Val Ile Ser Tyr Gln Pro Thr Ala Leu Gly Gly Leu Gln
        355                 360                 365

Leu Gln Gln Arg Val Pro Gly Asp Trp Ser Gly Val Thr Ile Thr Glu
    370                 375                 380

Leu Glu Pro Gly Leu Thr Tyr Asn Ile Ser Val Tyr Ala Val Ile Ser
385                 390                 395                 400

Asn Ile Leu Ser Leu Pro Ile Thr Ala Lys Val Ala Thr His Leu Ser
                405                 410                 415

Thr Pro Gln Gly Leu Gln Phe Lys Thr Ile Thr Glu Thr Thr Val Glu
            420                 425                 430

Val Gln Trp Glu Pro Phe Ser Phe Ser Phe Asp Gly Trp Glu Ile Ser
        435                 440                 445

Phe Ile Pro Lys Asn Asn Glu Gly Gly Val Ile Ala Gln Val Pro Ser
    450                 455                 460

Asp Val Thr Ser Phe Asn Gln Thr Gly Leu Lys Pro Gly Glu Glu Tyr
465                 470                 475                 480

Ile Val Asn Val Val Ala Leu Lys Glu Gln Ala Arg Ser Pro Pro Thr
                485                 490                 495

Ser Ala Ser Val Ser Thr Val Ile Asp Gly Pro Thr Gln Ile Leu Val
            500                 505                 510

Arg Asp Val Ser Asp Thr Val Ala Phe Val Glu Trp Ile Pro Pro Arg
        515                 520                 525

Ala Lys Val Asp Phe Ile Leu Leu Lys Tyr Gly Leu Val Gly Gly Glu
    530                 535                 540

Gly Gly Arg Thr Thr Phe Arg Leu Gln Pro Pro Leu Ser Gln Tyr Ser
545                 550                 555                 560

Val Gln Ala Leu Arg Pro Gly Ser Arg Tyr Glu Val Ser Val Ser Ala
                565                 570                 575

Val Arg Gly Thr Asn Glu Ser Asp Ser Ala Thr Thr Gln Phe Thr Thr
            580                 585                 590

Glu Ile Asp Ala Pro Lys Asn Leu Arg Val Gly Ser Arg Thr Ala Thr
        595                 600                 605

Ser Leu Asp Leu Glu Trp Asp Asn Ser Glu Ala Glu Val Gln Glu Tyr
    610                 615                 620

Lys Val Val Tyr Ser Thr Leu Ala Gly Glu Gln Tyr His Glu Val Leu
625                 630                 635                 640

Val Pro Arg Gly Ile Gly Pro Thr Thr Arg Ala Thr Leu Thr Asp Leu

645                 650                 655

Val Pro Gly Thr Glu Tyr Gly Val Gly Ile Ser Ala Val Met Asn Ser
            660                 665                 670

Gln Gln Ser Val Pro Ala Thr Met Asn Ala Arg Thr Glu Leu Asp Ser
        675                 680                 685

Pro Arg Asp Leu Met Val Thr Ala Ser Ser Glu Thr Ser Ile Ser Leu
    690                 695                 700

Ile Trp Thr Lys Ala Ser Gly Pro Ile Asp His Tyr Arg Ile Thr Phe
705                 710                 715                 720

Thr Pro Ser Ser Gly Ile Ala Ser Glu Val Thr Val Pro Lys Asp Arg
                725                 730                 735

Thr Ser Tyr Thr Leu Thr Asp Leu Glu Pro Gly Ala Glu Tyr Ile Ile
            740                 745                 750

Ser Val Thr Ala Glu Arg Gly Arg Gln Gln Ser Leu Glu Ser Thr Val
        755                 760                 765

Asp Ala Phe Thr Gly Phe Arg Pro Ile Ser His Leu His Phe Ser His
    770                 775                 780

Val Thr Ser Ser Ser Val Asn Ile Thr Trp Ser Asp Pro Ser Pro Pro
785                 790                 795                 800

Ala Asp Arg Leu Ile Leu Asn Tyr Ser Pro Arg Asp Glu Glu Glu Glu
                805                 810                 815

Met Met Glu Val Ser Leu Asp Ala Thr Lys Arg His Ala Val Leu Met
            820                 825                 830

Gly Leu Gln Pro Ala Thr Glu Tyr Ile Val Asn Leu Val Ala Val His
        835                 840                 845

Gly Thr Val Thr Ser Glu Pro Ile Val Gly Ser Ile Thr Thr Gly Ile
    850                 855                 860

Asp Pro Pro Lys Asp Ile Thr Ile Ser Asn Val Thr Lys Asp Ser Val
865                 870                 875                 880

Met Val Ser Trp Ser Pro Pro Val Ala Ser Phe Asp Tyr Tyr Arg Val
                885                 890                 895

Ser Tyr Arg Pro Thr Gln Val Gly Arg Leu Asp Ser Ser Val Val Pro
            900                 905                 910

Asn Thr Val Thr Glu Phe Thr Ile Thr Arg Leu Asn Pro Ala Thr Glu
        915                 920                 925

Tyr Glu Ile Ser Leu Asn Ser Val Arg Gly Arg Glu Glu Ser Glu Arg
    930                 935                 940

Ile Cys Thr Leu Val His Thr Ala Met Asp Asn Pro Val Asp Leu Ile
945                 950                 955                 960

Ala Thr Asn Ile Thr Pro Thr Glu Ala Leu Leu Gln Trp Lys Ala Pro
                965                 970                 975

Val Gly Glu Val Glu Asn Tyr Val Ile Val Leu Thr His Phe Ala Val
            980                 985                 990

Ala Gly Glu Thr Ile Leu Val Asp  Gly Val Ser Glu Glu  Phe Arg Leu
        995                 1000                 1005

Val Asp  Leu Leu Pro Ser Thr  His Tyr Thr Ala Thr  Met Tyr Ala
    1010                 1015                 1020

Thr Asn  Gly Pro Leu Thr Ser  Gly Thr Ile Ser Thr  Asn Phe Ser
    1025                 1030                 1035

Thr Leu  Leu Asp Pro Pro Ala  Asn Leu Thr Ala Ser  Glu Val Thr
    1040                 1045                 1050

Arg Gln  Ser Ala Leu Ile Ser  Trp Gln Pro Pro Arg  Ala Glu Ile
    1055                 1060                 1065

Glu Asn  Tyr Val Leu Thr Tyr  Lys Ser Thr Asp Gly  Ser Arg Lys
    1070                 1075                 1080

Glu Leu  Ile Val Asp Ala Glu  Asp Thr Trp Ile Arg  Leu Glu Gly
    1085                 1090                 1095

Leu Leu  Glu Asn Thr Asp Tyr  Thr Val Leu Leu Gln  Ala Ala Gln
    1100                 1105                 1110

Asp Thr  Thr Trp Ser Ser Ile  Thr Ser Thr Ala Phe  Thr Thr Gly
    1115                 1120                 1125

Gly Arg  Val Phe Pro His Pro  Gln Asp Cys Ala Gln  His Leu Met
    1130                 1135                 1140

Asn Gly Asp Thr Leu Ser Gly Val Tyr Pro Ile Phe Leu Asn Gly
    1145                1150                1155

Glu Leu Ser Gln Lys Leu Gln Val Tyr Cys Asp Met Thr Thr Asp
    1160                1165                1170

Gly Gly Gly Trp Ile Val Phe Gln Arg Arg Gln Asn Gly Gln Thr
    1175                1180                1185

Asp Phe Phe Arg Lys Trp Ala Asp Tyr Arg Val Gly Phe Gly Asn
    1190                1195                1200

Val Glu Asp Glu Phe Trp Leu Gly Leu Asp Asn Ile His Arg Ile
    1205                1210                1215

Thr Ser Gln Gly Arg Tyr Glu Leu Arg Val Asp Met Arg Asp Gly
    1220                1225                1230

Gln Glu Ala Ala Phe Ala Ser Tyr Asp Arg Phe Ser Val Glu Asp
    1235                1240                1245

Ser Arg Asn Leu Tyr Lys Leu Arg Ile Gly Ser Tyr Asn Gly Thr
    1250                1255                1260

Ala Gly Asp Ser Leu Ser Tyr His Gln Gly Arg Pro Phe Ser Thr
    1265                1270                1275

Glu Asp Arg Asp Asn Asp Val Ala Val Thr Asn Cys Ala Met Ser
    1280                1285                1290

Tyr Lys Gly Ala Trp Trp Tyr Lys Asn Cys His Arg Thr Asn Leu
    1295                1300                1305

Asn Gly Lys Tyr Gly Glu Ser Arg His Ser Gln Gly Ile Asn Trp
    1310                1315                1320

Tyr His Trp Lys Gly His Glu Phe Ser Ile Pro Phe Val Glu Met
    1325                1330                1335

Lys Met Arg Pro Tyr Asn His Arg Leu Met Ala Gly Arg Lys Arg
    1340                1345                1350

Gln Ser Leu Gln Phe
    1355

<210>  3
<211>  23

<212>  DNA
<213>  Artificial

<220>
<223>  primer

<400>  3
tccatgatca agccttcaga gtg                                              23


<210>  4
<211>  22
<212>  DNA
<213>  Artificial

<220>
<223>  primer

<400>  4
gatgcagcca caggactcaa ag                                               22


<210>  5
<211>  7500
<212>  DNA
<213>  Artificial

<220>
<223>  vector pIN-III-ompA2

<400>  5
ggataaccag aagcaataaa aaatcaaatc ggatttcact atataatctc actttatcta      60

agatgaatcc gatggaagca tcctgttttc tctcaatttt tttatctaaa acccagcgtt     120

cgatgcttct ttgagcgaac gatcaaaaat aagtgccttc ccatcaaaaa aatattctca     180

acataaaaaa ctttgtgtaa tacttgtaac gctacatgga gattaactca atctagctag     240

agaggcttta cactttatgc ttccggctcg tataatgtgt ggaattgtga gcggataaca     300

atttcacaca ggaaacagct atgaccatga ttacggattc actggaactc tagataacga     360

ggcgcaaaaa atgaaaaaga cagctatcgc gattgcagtg gcactggctg gtttcgctac     420

cgtagcgcag gccgctgaat tccaagcttg gatccggctg agcaacgacg tgaacgcaat     480

gcgttccgac gttcaggctg ctaaagatga cgcagctcgt gctaaccagc gtctggacaa     540

catggctact aaataccgca agtaatagta cctgtgaagt gaaaaatggc gcacattgtg     600

cgacattttt tttgtctgcc gtttaccgct actgcgtcac gcgtaacata ttcccttgct     660

ctggttcacc attctgcgct gactctactg aaggcgcatt gctggctgcg ggagttgctc     720

cactgctcac cgaaaccgga taccctgccc gacgatacaa cgctttatcg actaacttct     780

gatctacagc cttattgtct ttaaattgcg taaagcctgc tggcagtgtg tatggcattg     840

tctgaacgtt ctgctgttct tctgccgata gtggtcgatg tacttcaaca taacgcatcc     900

cgttaggctc cacggaatat ttcaccggtt cgttgatcac tttcaccggc gttcccgtcc     960

gcacgctgga gaacaaggct ttaatatccg gtgcattcat gcgaatacac cctgaactga 1020
cgcgcaaacc gacgctgtcc ggcgcactgg taccatgaat gaggtattcg ccattaccat 1080
gcgcgaggcg cagtgcgtaa cgtcctagcg ggttatttgg tccggcagga acgactggcg 1140
gtaatttaat gccacgctcc agcgaacgct gacgaatgcc tgccgtaggc gtccaggttg 1200
ggttagggat tttctgccca acacgcgttt ccatcaccgg cgtttccagc ccctgcaatc 1260
caatacctat tggataaacc tgcacaatat tttctcccgg cggataataa taaaggcgca 1320
gctctgcaag gttggtcgac ggtcgaccgg tctcacttca acataacgca tcccgttagg 1380
ctccacggaa tatttcaccg gttcgttgat cactttcacc ggcgttcccg tccgcacgct 1440
ggagaacaag gctttaatat ccggtgcatt catgcgaata caccctgaac tgacgcgcaa 1500
accgacgctg tccggcgcac tggtaccatg aatgaggtat tcgccattac catgcgcgag 1560
gcgcagtgcg taacgtccta gcgggttatt tggtccggca ggaacgactg gcggtaattt 1620
aatgccacgc tccagcgaac gctgacgaat gcctgccgta ggcgtccagg ttgggttagg 1680
gattttctgc ccaacacgcg tttccatcac cggcgtttcc agcccctgca atccaatacc 1740
tattggataa acctgcacaa tattttctcc cggcggataa taataaaggc gcagctctgc 1800
aaggttgaca ccatcgaatg gcgcaaaacc tttcgcggta tggcatgata gcgcccggaa 1860
gagagtcaat tcagggtggt gaatgtgaaa ccagtaacgt tatacgatgt cgcagagtat 1920
gccggtgtct cttatcagac cgtttcccgc gtggtgaacc aggccagcca cgtttctgcg 1980
aaaacgcggg aaaaagtgga agcggcgatg gcggagctga attacattcc caaccgcgtg 2040
gcacaacaac tggcgggcaa acagtcgttg ctgattggcg ttgccacctc cagtctggcc 2100
ctgcacgcgc cgtcgcaaat tgtcgcggcg attaaatctc gcgccgatca actgggtgcc 2160
agcgtggtgg tgtcgatggt agaacgaagc ggcgtcgaag cctgtaaagc ggcggtgcac 2220
aatcttctcg cgcaacgcgt cagtgggctg atcattaact atccgctgga tgaccaggat 2280
gccattgctg tggaagctgc ctgcactaat gttccggcgt tatttcttga tgtctctgac 2340
cagacaccca tcaacagtat tattttctcc catgaagacg gtacgcgact gggcgtggag 2400
catctggtcg cattgggtca ccagcaaatc gcgctgttag cgggcccatt aagttctgtc 2460
tcggcgcgtc tgcgtctggc tggctggcat aaatatctca ctcgcaatca aattcagccg 2520
atagcggaac gggaaggcga ctggagtgcc atgtccggtt ttcaacaaac catgcaaatg 2580
ctgaatgagg gcatcgttcc cactgcgatg ctggttgcca acgatcagat ggcgctgggc 2640
gcaatgcgcg ccattaccga gtccgggctg cgcgttggtg cggatatctc ggtagtggga 2700
tacgacgata ccgaagacag ctcatgttat atcccgccgt taaccaccat caaacaggat 2760
tttcgcctgc tggggcaaac cagcgtggac cgcttgctgc aactctctca gggccaggcg 2820
gtgaagggca atcagctgtt gcccgtctca ctggtgaaaa gaaaaaccac cctggcgccc 2880

aatacgcaaa ccgcctctcc ccgcgcgttg gccgattcat taatgcagct ggcacgacag 2940

gtttcccgac tggaaagcgg gcagtgagcg caacgcaatt aatgtgagtt agctcactca 3000

ttaggcaccc caggctttac actttatgct aacgataatc cctgacgcgg tgcatcaggt 3060

aataacagtt gtgaaggaat agttatcgtc gtaccaggtt ttggcaccgg ggcgatagtg 3120

ttattggctt caaggatcaa cattgccgca gtatcaaaac gtcgggcaat agcctgaagg 3180

tttttatccc cttcttgcac cgtatacgtt tgattttgcc caaccagtcg gcttccggtt 3240

ggtggtagcg gataatcaac cgcccaggca gcctggatgg cgctaaaagc gccgataagc 3300

gtgagtgtaa gcaaagacgc gcgtttcatt gtaaacctcc tgtatttgcc ggagactcac 3360

gctgaaacgt cggatggcgc ttatgttcac ctgaaaccaa aacactcctg tgcaggtcag 3420

tgtaaacatt gaccatccgg caatgtgagc caaccggatg aaagctgtcc ttttagttta 3480

gctaagtgca gcggctttgg cgcgaattgc gcgaatcatc gcttccagac cttgtgaacg 3540

agatggggtg agatgttggg tgagcgccat tttttcaaac cacggacgca catcgaaatt 3600

gacaatatcc tgcggcgtca tctgatcgta gagaataaag acgaccgcaa taagcccttt 3660

cacaatcgcc gcatcgctgt cgccctgtaa ttcaataatt ccctgggcat tctggcgcat 3720

gacaatccac acctgactct gacagccctg aatgctattt tgtggacttc tgtcttcgtc 3780

gcgtaattct ggcagacgct gggggaccga tgcccttgag agccttcaac ccagtcagct 3840

ccttccggtg ggcgcggggc atgactatcg tcgccgcact tatgactgtc ttctttatca 3900

tgcaactcgt aggacaggtg ccggcagcgc tctgggtcat tttcggcgag gaccgctttc 3960

gctggagcgc gacgatgatc ggcctgtcgc ttgcggtatt cggaatcttg cacgccctcg 4020

ctcaagcctt cgtcactggt cccgccacca aacgtttcgg cgagaagcag gccattatcg 4080

ccggcatggc ggccgacgcg ctgggctacg tcttgctggc gttcgcgacg cgaggctgga 4140

tggccttccc cattatgatt cttctcgctt ccggcggcat cgggatgccc gcgttgcagg 4200

ccatgctgtc caggcaggta gatgacgacc atcagggaca gcttcaagga tcgctcgcgg 4260

ctcttaccag cctaacttcg atcactggac cgctgatcgt cacggcgatt tatgccgcct 4320

cggcgagcac atggaacggg ttggcatgga ttgtaggcgc cgccctatac cttgtctgcc 4380

tccccgcgtt gcgtcgcggt gcatggagcc gggccacctc gacctgaatg gaagccggcg 4440

gcacctcgct aacggattca ccactccaag aattggagcc aatcaattct tgcggagaac 4500

tgtgaatgcg caaaccaacc cttggcagaa catatccatc gcgtccgcca tctccagcag 4560

ccgcacgcgg cgcatctcgg gcagcgttgg gtcctggcca cgggtgcgca tgatcgtgct 4620

cctgtcgttg aggacccggc taggctggcg gggttgcctt actggttagc agaatgaatc 4680

accgatacgc gagcgaacgt gaagcgactg ctgctgcaaa acgtctgcga cctgagcaac 4740

aacatgaatg gtcttcggtt tccgtgtttc gtaaagtctg gaaacgcgga agtcagcgcc 4800
ctgcaccatt atgttccgga tctgcatcgc aggatgctgc tggctaccct gtggaacacc 4860
tacatctgta ttaacgaagc gctggcattg accctgagtg atttttctct ggtcccgccg 4920
catccatacc gccagttgtt taccctcaca acgttccagt aaccgggcat gttcatcatc 4980
agtaacccgt atcgtgagca tcctctctcg tttcatcggt atcattaccc ccatgaacag 5040
aaatccccct tacacggagg catcagtgac caaacaggaa aaaaccgccc ttaacatggc 5100
ccgctttatc agaagccaga cattaacgct tctggagaaa ctcaacgagc tggacgcgga 5160
tgaacaggca gacatctgtg aatcgcttca cgaccacgct gatgagcttt accgcagctg 5220
cctcgcgcgt ttcggtgatg acggtgaaaa cctctgacac atgcagctcc cggagacggt 5280
cacagcttgt ctgtaagcgg atgccgggag cagacaagcc cgtcagggcg cgtcagcggg 5340
tgttggcggg tgtcggggcg cagccatgac ccagtcacgt agcgatagcg gagtgtatac 5400
tggcttaact atgcggcatc agagcagatt gtactgagag tgcaccatat gcggtgtgaa 5460
ataccgcaca gatgcgtaag gagaaaatac cgcatcaggc gctcttccgc ttcctcgctc 5520
actgactcgc tgcgctcggt cgttcggctg cggcgagcgg tatcagctca ctcaaaggcg 5580
gtaatacggt tatccacaga atcaggggat aacgcaggaa agaacatgtg agcaaaaggc 5640
cagcaaaagg ccaggaaccg taaaaaggcc gcgttgctgg cgtttttcca taggctccgc 5700
ccccctgacg agcatcacaa aaatcgacgc tcaagtcaga ggtggcgaaa cccgacagga 5760
ctataaagat accaggcgtt tccccctgga agctccctcg tgcgctctcc tgttccgacc 5820
ctgccgctta ccggatacct gtccgccttt ctcccttcgg gaagcgtggc gctttctcat 5880
agctcacgct gtaggtatct cagttcggtg taggtcgttc gctccaagct gggctgtgtg 5940
cacgaacccc ccgttcagcc cgaccgctgc gccttatccg gtaactatcg tcttgagtcc 6000
aacccggtaa gacacgactt atcgccactg gcagcagcca ctggtaacag gattagcaga 6060
gcgaggtatg taggcggtgc tacagagttc ttgaagtggt ggcctaacta cggctacact 6120
agaaggacag tatttggtat ctgcgctctg ctgaagccag ttaccttcgg aaaaagagtt 6180
ggtagctctt gatccggcaa acaaaccacc gctggtagcg gtggtttttt tgtttgcaag 6240
cagcagatta cgcgcagaaa aaaaggatct caagaagatc ctttgatctt ttctacgggg 6300
tctgacgctc agtggaacga aaactcacgt taagggattt tggtcatgag attatcaaaa 6360
aggatcttca cctagatcct tttaaattaa aaatgaagtt ttaaatcaat ctaaagtata 6420
tatgagtaaa cttggtctga cagttaccaa tgcttaatca gtgaggcacc tatctcagcg 6480
atctgtctat ttcgttcatc catagttgcc tgactccccg tcgtgtagat aactacgata 6540
cgggagggct taccatctgg ccccagtgct gcaatgatac cgcgagaccc acgctcaccg 6600
gctccagatt tatcagcaat aaaccagcca gccggaaggg ccgagcgcag aagtggtcct 6660

gcaactttat ccgcctccat ccagtctatt aattgttgcc gggaagctag agtaagtagt 6720

tcgccagtta atagtttgcg caacgttgtt gccattgctg caggcatcgt ggtgtcacgc 6780

tcgtcgtttg gtatggcttc attcagctcc ggttcccaac gatcaaggcg agttacatga 6840

tcccccatgt tgtgcaaaaa agcggttagc tccttcggtc ctccgatcgt tgtcagaagt 6900

aagttggccg cagtgttatc actcatggtt atggcagcac tgcataattc tcttactgtc 6960

atgccatccg taagatgctt ttctgtgact ggtgagtact caaccaagtc attctgagaa 7020

tagtgtatgc ggcgaccgag ttgctcttgc ccggcgtcaa cacgggataa taccgcgcca 7080

catagcagaa ctttaaaagt gctcatcatt ggaaaacgtt cttcggggcg aaaactctca 7140

aggatcttac cgctgttgag atccagttcg atgtaaccca ctcgtgcacc caactgatct 7200

tcagcatctt ttactttcac cagcgtttct gggtgagcaa aaacaggaag gcaaaatgcc 7260

gcaaaaaagg gaataagggc gacacggaaa tgttgaatac tcatactctt cctttttcaa 7320

tattattgaa gcatttatca gggttattgt ctcatgagcg gatacatatt tgaatgtatt 7380

tagaaaaata aacaaatagg ggttccgcgc acatttcccc gaaaagtgcc acctgacgtc 7440

taagaaacca ttattatcat gacattaacc tataaaaata ggcgtatcac gaggcccttt 7500

<210> 6
<211> 1358
<212> PRT
<213> Mus musculus

<400> 6

Met Gly Ile Asp Gly Glu Thr Val Val Leu Lys Asn Met Leu Ile Gly
1               5                   10                  15

Val Asn Leu Ile Leu Leu Gly Ser Met Leu Lys Pro Ser Glu Cys Arg
            20                  25                  30

Leu Glu Val Thr Thr Glu Arg Ala Gln Arg Gln Thr Val Glu Glu Glu
        35                  40                  45

Gly Gly Ala Ser Ser His Asn Thr Ser Ser Lys Glu Gln Pro Met Val
    50                  55                  60

Phe Asn His Val Tyr Asn Ile Asn Val Pro Leu Glu Ser Leu Cys Ser
65                  70                  75                  80

Ser Gly Leu Glu Ala Ser Ala Glu Gln Asp Met Ser Ala Glu Asp Asp
                85                  90                  95

Thr Leu Ala Glu Tyr Ile Gly Gln Thr Ser Asp His Glu Ser Gln Val
            100                 105                 110

Thr Phe Thr His Lys Ile Asn Leu Pro Lys Lys Ala Cys Pro Cys Ala
        115                 120                 125

Ser Ser Ser Gln Val Leu Gln Glu Leu Leu Ser Arg Ile Glu Met Leu
    130                 135                 140

Glu Arg Glu Val Ser Leu Leu Arg Asp Gln Cys Asn Thr Asn Cys Cys
145                 150                 155                 160

Gln Glu Ser Ala Ala Thr Gly Gln Leu Asp Tyr Val Pro His Cys Ser
                165                 170                 175

Gly His Gly Asn Phe Ser Phe Glu Ser Cys Gly Cys Ile Cys Asn Glu
            180                 185                 190

Gly Trp Phe Gly Lys Asn Cys Ser Glu Pro Tyr Cys Pro Leu Gly Cys
        195                 200                 205

Ser Ser Arg Gly Val Cys Val Asp Gly Gln Cys Ile Cys Asp Ser Glu
    210                 215                 220

Tyr Ser Gly Asp Asp Cys Ser Glu Leu Arg Cys Pro Thr Asp Cys Ser
225                 230                 235                 240

Ser Arg Gly Leu Cys Val Asp Gly Glu Cys Val Cys Glu Glu Pro Tyr
                245                 250                 255

Thr Gly Glu Asp Cys Arg Glu Leu Arg Cys Pro Gly Asp Cys Ser Gly
            260                 265                 270

Lys Gly Gln Cys Ala Asn Gly Thr Cys Leu Cys Gln Glu Gly Tyr Ala
        275                 280                 285

Gly Glu Asp Cys Ser Gln Arg Arg Cys Leu Asn Ala Cys Ser Gly Arg
    290                 295                 300

Gly His Cys Gln Glu Gly Leu Cys Ile Cys Glu Glu Gly Tyr Gln Gly
305                 310                 315                 320

Pro Asp Cys Ser Ala Val Ala Pro Pro Glu Asp Leu Arg Val Ala Gly
                325                 330                 335

Ile Ser Asp Arg Ser Ile Glu Leu Glu Trp Asp Gly Pro Met Ala Val
            340                 345                 350

Thr Glu Tyr Val Ile Ser Tyr Gln Pro Thr Ala Leu Gly Gly Leu Gln

355                 360                 365

Leu Gln Gln Arg Val Pro Gly Asp Trp Ser Gly Val Thr Ile Met Glu
    370                 375                 380

Leu Glu Pro Gly Leu Thr Tyr Asn Ile Ser Val Tyr Ala Val Ile Ser
385                 390                 395                 400

Asn Ile Leu Ser Leu Pro Ile Thr Ala Lys Val Ala Thr His Leu Ser
                405                 410                 415

Thr Pro Gln Gly Leu Gln Phe Lys Thr Ile Thr Glu Thr Thr Val Glu
            420                 425                 430

Val Gln Trp Glu Pro Phe Ser Phe Ser Phe Asp Gly Trp Glu Ile Ser
        435                 440                 445

Phe Ile Pro Lys Asn Asn Glu Gly Gly Val Ile Ala Gln Leu Pro Ser
    450                 455                 460

Asp Val Thr Ser Phe Asn Gln Thr Gly Leu Lys Pro Gly Glu Glu Tyr
465                 470                 475                 480

Ile Val Asn Val Val Ala Leu Lys Glu Gln Ala Arg Ser Pro Pro Thr
                485                 490                 495

Ser Ala Ser Val Ser Thr Val Ile Asp Gly Pro Thr Gln Ile Leu Val
            500                 505                 510

Arg Asp Val Ser Asp Thr Val Ala Phe Val Glu Trp Thr Pro Pro Arg
        515                 520                 525

Ala Lys Val Asp Phe Ile Leu Leu Lys Tyr Gly Leu Val Gly Gly Glu
    530                 535                 540

Gly Gly Lys Thr Thr Phe Arg Leu Gln Pro Pro Leu Ser Gln Tyr Ser
545                 550                 555                 560

Val Gln Ala Leu Arg Pro Gly Ser Arg Tyr Glu Val Ser Ile Ser Ala
                565                 570                 575

Val Arg Gly Thr Asn Glu Ser Glu Ala Ser Ser Thr Gln Phe Thr Thr
            580                 585                 590

Glu Ile Asp Ala Pro Lys Asn Leu Arg Val Gly Ser Arg Thr Ala Thr
        595                 600                 605

Ser Leu Asp Leu Glu Trp Asp Asn Ser Glu Ala Glu Ala Gln Glu Tyr
    610                 615                 620

Lys Val Val Tyr Ser Thr Leu Ala Gly Glu Gln Tyr His Glu Val Leu
625                 630                 635                 640

Val Pro Lys Gly Ile Gly Pro Thr Thr Lys Thr Thr Leu Thr Asp Leu
                645                 650                 655

Val Pro Gly Thr Glu Tyr Gly Val Gly Ile Ser Ala Val Met Asn Ser
            660                 665                 670

Lys Gln Ser Ile Pro Ala Thr Met Asn Ala Arg Thr Glu Leu Asp Ser
        675                 680                 685

Pro Arg Asp Leu Met Val Thr Ala Ser Ser Glu Thr Ser Ile Ser Leu
    690                 695                 700

Ile Trp Thr Lys Ala Ser Gly Pro Ile Asp His Tyr Arg Ile Thr Phe
705                 710                 715                 720

Thr Pro Ser Ser Gly Ile Ser Ser Glu Val Thr Val Pro Arg Asp Arg
                725                 730                 735

Thr Ser Tyr Thr Leu Thr Asp Leu Glu Pro Gly Ala Glu Tyr Ile Ile
            740                 745                 750

Ser Ile Thr Ala Glu Arg Gly Arg Gln Gln Ser Leu Glu Ser Thr Val
        755                 760                 765

Asp Ala Phe Thr Gly Phe Arg Pro Ile Ser His Leu His Phe Ser His
    770                 775                 780

Val Thr Ser Ser Ser Val Asn Ile Thr Trp Ser Asp Pro Ser Pro Pro
785                 790                 795                 800

Ala Asp Arg Leu Ile Leu Asn Tyr Ser Pro Arg Asp Lys Glu Glu Asp
                805                 810                 815

Met Leu Glu Val Leu Leu Asp Ala Thr Lys Arg His Ala Val Leu Met
            820                 825                 830

Gly Leu Gln Pro Ala Thr Glu Tyr Ile Val Asn Leu Val Ala Val His
        835                 840                 845

Gly Thr Val Thr Ser Glu Pro Ile Val Gly Ser Ile Thr Thr Gly Ile
    850                 855                 860

Asp Pro Pro Lys Asn Ile Thr Ile Ser Asn Val Thr Lys Asp Ser Leu
865                 870                 875                 880

Thr Val Ser Trp Ser Ser Pro Val Ala Pro Phe Asp Tyr Tyr Arg Val
                885                 890                 895

Ser Tyr Arg Pro Thr Gln Val Gly Arg Leu Asp Ser Ser Val Val Pro
            900                 905                 910

Asn Thr Val Thr Glu Phe Ala Ile Thr Arg Leu Tyr Pro Ala Thr Glu
        915                 920                 925

Tyr Glu Ile Ser Leu Asn Ser Val Arg Gly Arg Glu Glu Ser Glu Arg
    930                 935                 940

Ile Cys Thr Leu Val His Thr Ala Met Asp Ser Pro Met Asp Leu Ile
945                 950                 955                 960

Ala Thr Asn Ile Thr Pro Thr Glu Ala Leu Leu Gln Trp Lys Ala Pro
                965                 970                 975

Met Gly Glu Val Glu Asn Tyr Val Ile Val Leu Thr His Phe Ala Ile
            980                 985                 990

Ala Gly Glu Thr Ile Leu Val Asp  Gly Val Ser Glu Glu  Phe Gln Leu
        995                 1000                1005

Val Asp  Leu Leu Pro Ser Thr  His Tyr Thr Val Thr  Met Tyr Ala
    1010                1015                1020

Thr Ser  Gly Pro Leu Met Ser  Gly Thr Ile Ala Thr  Asn Phe Ser
    1025                1030                1035

Thr Leu  Leu Asp Pro Pro Asp  Asn Leu Thr Ala Ser  Glu Val Thr
    1040                1045                1050

Arg Gln  Ser Ala Leu Ile Ser  Trp Gln Pro Pro Arg  Ala Ala Ile
    1055                1060                1065

Glu Asn  Tyr Val Leu Thr Tyr  Lys Ser Thr Asp Gly  Ser Arg Lys
    1070                1075                1080

Glu Leu  Ile Val Asp Ala Glu  Asp Thr Trp Ile Arg  Leu Glu Gly
    1085                1090                1095

Leu Ser  Glu Asn Thr Asp Tyr  Thr Val Leu Leu Gln  Ala Ala Gln
    1100                1105                1110

Glu Ala Thr Arg Ser Ser Leu Thr Ser Thr Val Phe Thr Thr Gly
    1115                1120                1125

Gly Arg Val Phe Ser His Pro Gln Asp Cys Ala Gln His Leu Met
    1130                1135                1140

Asn Gly Asp Thr Leu Ser Gly Val Tyr Thr Ile Phe Leu Asn Gly
    1145                1150                1155

Glu Leu Ser His Lys Leu Gln Val Tyr Cys Asp Met Thr Thr Asp
    1160                1165                1170

Gly Gly Gly Trp Ile Val Phe Gln Arg Arg Gln Asn Gly Gln Thr
    1175                1180                1185

Asp Phe Phe Arg Lys Trp Ala Asp Tyr Arg Val Gly Phe Gly Asn
    1190                1195                1200

Leu Glu Asp Glu Phe Trp Leu Gly Leu Asp Asn Ile His Arg Ile
    1205                1210                1215

Thr Ala Gln Gly Arg Tyr Glu Leu Arg Val Asp Met Arg Asp Gly
    1220                1225                1230

Gln Glu Ala Val Phe Ala Tyr Tyr Asp Lys Phe Ala Val Glu Asp
    1235                1240                1245

Ser Arg Ser Leu Tyr Lys Ile Arg Ile Gly Ser Tyr Asn Gly Thr
    1250                1255                1260

Ala Gly Asp Ser Leu Ser Tyr His Gln Gly Arg Pro Phe Ser Thr
    1265                1270                1275

Glu Asp Arg Asp Asn Asp Val Ala Val Thr Asn Cys Ala Met Ser
    1280                1285                1290

Tyr Lys Gly Ala Trp Trp Tyr Lys Asn Cys His Arg Thr Asn Leu
    1295                1300                1305

Asn Gly Lys Tyr Gly Glu Ser Arg His Ser Gln Gly Ile Asn Trp
    1310                1315                1320

Tyr His Trp Lys Gly His Glu Phe Ser Ile Pro Phe Val Glu Met
    1325                1330                1335

Lys Met Arg Pro Tyr Ile His Arg Leu Thr Ala Gly Arg Lys Arg

1340                 1345                 1350

Arg Ala  Leu Lys Phe
    1355

<210>  7
<211>  1356
<212>  PRT
<213>  Rattus norvegicus

<400>  7

Met Gly Ile Glu Gly Glu Thr Val Val Leu Lys Asn Met Leu Ile Gly
1               5                   10                  15

Val Asn Leu Ile Leu Leu Gly Ser Met Leu Lys Pro Ser Glu Cys Arg
            20                  25                  30

Leu Glu Val Thr Thr Glu Arg Val Gln Arg Gln Thr Val Glu Glu Glu
        35                  40                  45

Gly Gly Ala Ser Ser Tyr Asn Thr Ser Ser Lys Glu Gln Pro Met Val
    50                  55                  60

Phe Asn His Val Tyr Asn Ile Asn Val Pro Leu Glu Ser Leu Cys Ser
65                  70                  75                  80

Ser Gly Leu Glu Ala Ser Ala Glu Gln Asp Val Ser Ala Glu Asp Asp
                85                  90                  95

Thr Leu Ala Glu Tyr Thr Gly Gln Thr Ser Asp His Glu Ser Gln Val
            100                 105                 110

Thr Phe Thr His Lys Ile Asn Leu Pro Lys Lys Ala Cys Pro Cys Ala
        115                 120                 125

Ser Ser Ala Gln Val Leu Gln Glu Leu Leu Ser Arg Ile Glu Met Leu
    130                 135                 140

Glu Arg Glu Val Ser Val Leu Arg Asp Gln Cys Asn Thr Asn Cys Cys
145                 150                 155                 160

Gln Glu Ser Ala Ala Thr Gly Gln Leu Asp Tyr Val Pro His Cys Ser
                165                 170                 175

Gly His Gly Asn Phe Ser Phe Glu Ser Cys Gly Cys Ile Cys Asn Glu
            180                 185                 190

Gly Trp Phe Gly Lys Asn Cys Ser Glu Pro Tyr Cys Pro Leu Gly Cys

195                 200                 205

Ser Ser Arg Gly Val Cys Val Asp Gly Gln Cys Ile Cys Asp Ser Glu
    210                 215                 220

Tyr Ser Gly Asp Asp Cys Ser Glu Leu Arg Cys Pro Thr Asp Cys Ser
225                 230                 235                 240

Ser Arg Gly Leu Cys Val Asp Gly Glu Cys Val Cys Glu Glu Pro Tyr
                245                 250                 255

Thr Gly Glu Asp Cys Arg Glu Leu Arg Cys Pro Gly Asp Cys Ser Gly
            260                 265                 270

Lys Gly Gln Cys Ala Asn Gly Thr Cys Leu Cys Gln Glu Gly Tyr Ala
        275                 280                 285

Gly Glu Asp Cys Ser Gln Arg Arg Cys Leu Asn Ala Cys Ser Gly Arg
    290                 295                 300

Gly His Cys Gln Glu Gly Leu Cys Ile Cys Glu Glu Gly Tyr Gln Gly
305                 310                 315                 320

Pro Asp Cys Ser Ala Val Thr Pro Pro Glu Asp Leu Arg Val Ala Gly
                325                 330                 335

Ile Ser Asp Arg Ser Ile Glu Leu Glu Trp Asp Gly Pro Met Ala Val
            340                 345                 350

Thr Glu Tyr Val Ile Ser Tyr Gln Pro Ser Leu Gly Gly Leu Gln Leu
        355                 360                 365

Gln Gln Arg Val Pro Gly Asp Trp Ser Gly Val Thr Ile Thr Glu Leu
    370                 375                 380

Glu Pro Gly Leu Thr Tyr Asn Ile Ser Val Tyr Ala Val Ile Ser Asn
385                 390                 395                 400

Ile Leu Ser Leu Pro Ile Thr Ala Lys Val Ala Thr His Leu Ser Thr
                405                 410                 415

Pro Gln Gly Leu Gln Phe Lys Thr Ile Thr Glu Thr Thr Val Glu Val
            420                 425                 430

Gln Trp Glu Pro Phe Ser Phe Ser Phe Asp Gly Trp Glu Ile Ser Phe
        435                 440                 445

Thr Pro Lys Asn Asn Glu Gly Gly Val Ile Ala Gln Leu Pro Ser Asp
    450                 455                 460

Val Thr Ser Phe Asn Gln Thr Gly Leu Lys Pro Gly Glu Glu Tyr Ile
465                 470                 475                 480

Val Asn Val Val Ala Leu Lys Glu Gln Ala Arg Gly Pro Pro Thr Ser
                485                 490                 495

Ala Ser Val Ser Thr Val Ile Asp Gly Pro Thr Gln Ile Leu Val Arg
            500                 505                 510

Asp Val Ser Asp Thr Val Ala Phe Val Glu Trp Thr Pro Pro Arg Ala
        515                 520                 525

Lys Val Asp Phe Ile Leu Leu Lys Tyr Gly Leu Val Gly Gly Glu Gly
    530                 535                 540

Gly Lys Thr Thr Phe Arg Leu Gln Pro Pro Leu Ser Gln Tyr Ser Val
545                 550                 555                 560

Gln Ala Leu Arg Pro Gly Ser Arg Tyr Glu Val Ser Ile Ser Ala Val
                565                 570                 575

Arg Gly Thr Asn Glu Ser Asp Ala Ser Ser Thr Gln Phe Thr Thr Glu
            580                 585                 590

Ile Asp Ala Pro Lys Asn Leu Arg Val Gly Ser Arg Thr Ala Thr Ser
        595                 600                 605

Leu Asp Leu Glu Trp Asp Asn Ser Glu Ala Glu Ala Gln Glu Tyr Lys
    610                 615                 620

Val Val Tyr Ser Thr Leu Ala Gly Glu Gln Tyr His Glu Val Leu Val
625                 630                 635                 640

Pro Lys Gly Ile Gly Pro Thr Thr Lys Thr Thr Leu Thr Asp Leu Val
                645                 650                 655

Pro Gly Thr Glu Tyr Gly Val Gly Ile Ser Ala Val Met Asn Ser Lys
            660                 665                 670

Gln Ser Ile Pro Ala Thr Met Asn Ala Arg Thr Glu Leu Asp Ser Pro
        675                 680                 685

Arg Asp Leu Met Val Thr Ala Ser Ser Glu Thr Ser Ile Ser Leu Ile
    690                 695                 700

Trp Thr Lys Ala Ser Gly Pro Ile Asp His Tyr Arg Ile Thr Phe Thr
705                 710                 715                 720

Pro Ser Ser Gly Ile Ser Ser Glu Val Thr Val Pro Arg Asp Arg Thr
                725                 730                 735

Ser Tyr Thr Leu Thr Asp Leu Glu Pro Gly Ala Glu Tyr Ile Ile Ser
            740                 745                 750

Ile Thr Ala Glu Arg Gly Arg Gln Gln Ser Leu Glu Ser Thr Val Asp
        755                 760                 765

Ala Phe Thr Gly Phe Arg Pro Ile Ser His Leu His Phe Ser His Val
    770                 775                 780

Thr Ser Ser Ser Val Asn Ile Thr Trp Ser Asp Pro Ser Pro Pro Ala
785                 790                 795                 800

Asp Arg Leu Ile Leu Asn Tyr Ser Pro Arg Asp Glu Glu Glu Glu Met
                805                 810                 815

Met Glu Val Leu Leu Asp Ala Thr Lys Arg His Ala Val Leu Met Gly
            820                 825                 830

Leu Gln Pro Ala Thr Glu Tyr Ile Val Asn Leu Val Ala Val His Gly
        835                 840                 845

Thr Val Thr Ser Glu Pro Ile Val Gly Ser Ile Thr Thr Gly Ile Asp
    850                 855                 860

Pro Pro Lys Asn Ile Thr Ile Ser Asn Val Thr Lys Asp Ser Leu Thr
865                 870                 875                 880

Val Ser Trp Ser Pro Pro Val Ala Pro Phe Asp Tyr Tyr Glu Tyr Pro
                885                 890                 895

Ile Asp His Pro Ser Gly Arg Leu Asp Ser Ser Val Val Pro Asn Thr
            900                 905                 910

Val Thr Glu Phe Thr Ile Thr Arg Leu Tyr Pro Ala Ser Gln Tyr Glu
        915                 920                 925

Ile Ser Leu Asn Ser Val Arg Gly Arg Glu Glu Ser Glu Arg Ile Cys
    930                 935                 940

Thr Leu Val His Thr Ala Met Asp Ser Pro Met Asp Leu Ile Ala Thr
945                 950                 955                 960

Asn Ile Thr Pro Thr Glu Ala Leu Leu Gln Trp Lys Ala Pro Met Gly
                965                 970                 975

Glu Val Glu Asn Tyr Val Ile Val Leu Thr His Phe Ala Met Ala Gly
            980                 985                 990

Glu Thr Ile Leu Val Asp Gly Val  Ser Glu Glu Phe Gln  Leu Val Asp
        995                 1000                 1005

Leu Leu  Pro Arg Thr His Tyr  Thr Val Thr Met Tyr  Ala Thr Ser
    1010                 1015                 1020

Gly Pro  Leu Val Ser Gly Thr  Ile Ala Thr Asn Phe  Ser Thr Leu
    1025                 1030                 1035

Leu Asp  Pro Pro Ala Asn Leu  Thr Ala Ser Glu Val  Thr Arg Gln
    1040                 1045                 1050

Ser Ala  Leu Ile Ser Trp Gln  Pro Pro Arg Ala Ala  Ile Glu Asn
    1055                 1060                 1065

Tyr Val  Leu Thr Tyr Lys Ser  Thr Asp Gly Ser Arg  Lys Glu Leu
    1070                 1075                 1080

Ile Val  Asp Ala Glu Asp Thr  Trp Ile Arg Leu Glu  Gly Leu Ser
    1085                 1090                 1095

Glu Asn  Thr Asp Tyr Thr Val  Leu Leu Gln Ala Ala  Gln Glu Ala
    1100                 1105                 1110

Thr Arg  Ser Ser Leu Thr Ser  Thr Ile Phe Thr Thr  Gly Gly Arg
    1115                 1120                 1125

Val Phe  Ser His Pro Gln Asp  Cys Ala Gln His Leu  Met Asn Gly
    1130                 1135                 1140

Asp Thr  Leu Ser Gly Val Tyr  Thr Ile Phe Leu Asn  Gly Glu Leu
    1145                 1150                 1155

Ser His  Lys Leu Gln Val Tyr  Cys Asp Met Thr Thr  Asp Gly Gly
    1160                 1165                 1170

Gly Trp  Ile Val Phe Gln Arg  Arg Gln Asn Gly Gln  Thr Asp Phe
    1175                 1180                 1185

Phe Arg  Lys Trp Ala Asp Tyr  Arg Val Gly Phe Gly  Asn Leu Glu

1190                 1195                 1200

Asp Glu  Phe Trp Leu Gly Leu  Asp Asn Tyr His Arg  Ile Thr Ala
    1205                 1210                 1215

Gln Gly  Arg Tyr Glu Leu Arg  Val Asp Met Arg Asp  Gly Gln Glu
    1220                 1225                 1230

Ala Val  Phe Ala Tyr Tyr Asp  Lys Phe Ala Val Glu  Asp Ser Arg
    1235                 1240                 1245

Ser Leu  Tyr Lys Leu Arg Ile  Gly Gly Tyr Asn Gly  Thr Ala Gly
    1250                 1255                 1260

Asp Ser  Leu Ser Tyr His Gln  Gly Arg Pro Phe Ser  Thr Glu Asp
    1265                 1270                 1275

Arg Asp  Asn Asp Val Ala Val  Thr Asn Cys Ala Met  Ser Tyr Lys
    1280                 1285                 1290

Gly Ala  Trp Trp Tyr Lys Asn  Cys His Arg Thr Asn  Leu Asn Gly
    1295                 1300                 1305

Lys Tyr  Gly Glu Ser Arg His  Ser Gln Gly Ile Asn  Trp Tyr His
    1310                 1315                 1320

Trp Lys  Gly His Glu Phe Ser  Ile Pro Phe Val Glu  Met Lys Met
    1325                 1330                 1335

Arg Pro  Tyr Ile His Arg Leu  Thr Ala Gly Arg Lys  Arg Arg Ala
    1340                 1345                 1350

Leu Lys  Phe
    1355

<210>  8
<211>  1334
<212>  PRT
<213>  Homo sapiens

<400>  8

Met Ile Lys Pro Ser Glu Cys Gln Leu Glu Val Thr Thr Glu Arg Val
1               5                   10                  15

Gln Arg Gln Ser Val Glu Glu Glu Gly Gly Ile Ala Asn Tyr Asn Thr
            20                  25                  30

Ser Ser Lys Glu Gln Pro Val Val Phe Asn His Val Tyr Asn Ile Asn

35                  40                  45

Val Pro Leu Asp Asn Leu Cys Ser Ser Gly Leu Glu Ala Ser Ala Glu
    50                  55                  60

Gln Glu Val Ser Ala Glu Asp Glu Thr Leu Ala Glu Tyr Met Gly Gln
65                  70                  75                  80

Thr Ser Asp His Glu Ser Gln Val Thr Phe Thr His Arg Ile Asn Phe
                85                  90                  95

Pro Lys Lys Ala Cys Pro Cys Ala Ser Ser Ala Gln Val Leu Gln Glu
            100                 105                 110

Leu Leu Ser Arg Ile Glu Met Leu Glu Arg Glu Val Ser Val Leu Arg
        115                 120                 125

Asp Gln Cys Asn Ala Asn Cys Cys Gln Glu Ser Ala Ala Thr Gly Gln
    130                 135                 140

Leu Asp Tyr Ile Pro His Cys Ser Gly His Gly Asn Phe Ser Phe Glu
145                 150                 155                 160

Ser Cys Gly Cys Ile Cys Asn Glu Gly Trp Phe Gly Lys Asn Cys Ser
                165                 170                 175

Glu Pro Tyr Cys Pro Leu Gly Cys Ser Ser Arg Gly Val Cys Val Asp
            180                 185                 190

Gly Gln Cys Ile Cys Asp Ser Glu Tyr Ser Gly Asp Asp Cys Ser Glu
        195                 200                 205

Leu Arg Cys Pro Thr Asp Cys Ser Ser Arg Gly Leu Cys Val Asp Gly
    210                 215                 220

Glu Cys Val Cys Glu Glu Pro Tyr Thr Gly Glu Asp Cys Arg Glu Leu
225                 230                 235                 240

Arg Cys Pro Gly Asp Cys Ser Gly Lys Gly Arg Cys Ala Asn Gly Thr
                245                 250                 255

Cys Leu Cys Glu Glu Gly Tyr Val Gly Glu Asp Cys Gly Gln Arg Gln
            260                 265                 270

Cys Leu Asn Ala Cys Ser Gly Arg Gly Gln Cys Glu Glu Gly Leu Cys
        275                 280                 285

Val Cys Glu Glu Gly Tyr Gln Gly Pro Asp Cys Ser Ala Val Ala Pro
    290                 295                 300

Pro Glu Asp Leu Arg Val Ala Gly Ile Ser Asp Arg Ser Ile Glu Leu
305                 310                 315                 320

Glu Trp Asp Gly Pro Met Ala Val Thr Glu Tyr Val Ile Ser Tyr Gln
                325                 330                 335

Pro Thr Ala Leu Gly Gly Leu Gln Leu Gln Gln Arg Val Pro Gly Asp
            340                 345                 350

Trp Ser Gly Val Thr Ile Thr Glu Leu Glu Pro Gly Leu Thr Tyr Asn
        355                 360                 365

Ile Ser Val Tyr Ala Val Ile Ser Asn Ile Leu Ser Leu Pro Ile Thr
    370                 375                 380

Ala Lys Val Ala Thr His Leu Ser Thr Pro Gln Gly Leu Gln Phe Lys
385                 390                 395                 400

Thr Ile Thr Glu Thr Thr Val Glu Val Gln Trp Glu Pro Phe Ser Phe
                405                 410                 415

Ser Phe Asp Gly Trp Glu Ile Ser Phe Ile Pro Lys Asn Asn Glu Gly
            420                 425                 430

Gly Val Ile Ala Gln Val Pro Ser Asp Val Thr Ser Phe Asn Gln Thr
        435                 440                 445

Gly Leu Lys Pro Gly Glu Glu Tyr Ile Val Asn Val Val Ala Leu Lys
    450                 455                 460

Glu Gln Ala Arg Ser Pro Pro Thr Ser Ala Ser Val Ser Thr Val Ile
465                 470                 475                 480

Asp Gly Pro Thr Gln Ile Leu Val Arg Asp Val Ser Asp Thr Val Ala
                485                 490                 495

Phe Val Glu Trp Ile Pro Pro Arg Ala Lys Val Asp Phe Ile Leu Leu
            500                 505                 510

Lys Tyr Gly Leu Val Gly Gly Glu Gly Gly Arg Thr Thr Phe Arg Leu
        515                 520                 525

Gln Pro Pro Leu Ser Gln Tyr Ser Val Gln Ala Leu Arg Pro Gly Ser
    530                 535                 540

Arg Tyr Glu Val Ser Val Ser Ala Val Arg Gly Thr Asn Glu Ser Asp
545                 550                 555                 560

Ser Ala Thr Thr Gln Phe Thr Thr Glu Ile Asp Ala Pro Lys Asn Leu
                565                 570                 575

Arg Val Gly Ser Arg Thr Ala Thr Ser Leu Asp Leu Glu Trp Asp Asn
            580                 585                 590

Ser Glu Ala Glu Val Gln Glu Tyr Lys Val Val Tyr Ser Thr Leu Ala
        595                 600                 605

Gly Glu Gln Tyr His Glu Val Leu Val Pro Arg Gly Ile Gly Pro Thr
    610                 615                 620

Thr Arg Ala Thr Leu Thr Asp Leu Val Pro Gly Thr Glu Tyr Gly Val
625                 630                 635                 640

Gly Ile Ser Ala Val Met Asn Ser Gln Gln Ser Val Pro Ala Thr Met
                645                 650                 655

Asn Ala Arg Thr Glu Leu Asp Ser Pro Arg Asp Leu Met Val Thr Ala
            660                 665                 670

Ser Ser Glu Thr Ser Ile Ser Leu Ile Trp Thr Lys Ala Ser Gly Pro
        675                 680                 685

Ile Asp His Tyr Arg Ile Thr Phe Thr Pro Ser Ser Gly Ile Ala Ser
    690                 695                 700

Glu Val Thr Val Pro Lys Asp Arg Thr Ser Tyr Thr Leu Thr Asp Leu
705                 710                 715                 720

Glu Pro Gly Ala Glu Tyr Ile Ile Ser Val Thr Ala Glu Arg Gly Arg
                725                 730                 735

Gln Gln Ser Leu Glu Ser Thr Val Asp Ala Phe Thr Gly Phe Arg Pro
            740                 745                 750

Ile Ser His Leu His Phe Ser His Val Thr Ser Ser Ser Val Asn Ile
        755                 760                 765

Thr Trp Ser Asp Pro Ser Pro Pro Ala Asp Arg Leu Ile Leu Asn Tyr
    770                 775                 780

Ser Pro Arg Asp Glu Glu Glu Glu Met Met Glu Val Ser Leu Asp Ala
785                 790                 795                 800

Thr Lys Arg His Ala Val Leu Met Gly Leu Gln Pro Ala Thr Glu Tyr
                805                 810                 815

Ile Val Asn Leu Val Ala Val His Gly Thr Val Thr Ser Glu Pro Ile
            820                 825                 830

Val Gly Ser Ile Thr Thr Gly Ile Asp Pro Pro Lys Asp Ile Thr Ile
        835                 840                 845

Ser Asn Val Thr Lys Asp Ser Val Met Val Ser Trp Ser Pro Pro Val
    850                 855                 860

Ala Ser Phe Asp Tyr Tyr Arg Val Ser Tyr Arg Pro Thr Gln Val Gly
865                 870                 875                 880

Arg Leu Asp Ser Ser Val Val Pro Asn Thr Val Thr Glu Phe Thr Ile
                885                 890                 895

Thr Arg Leu Asn Pro Ala Thr Glu Tyr Glu Ile Ser Leu Asn Ser Val
            900                 905                 910

Arg Gly Arg Glu Glu Ser Glu Arg Ile Cys Thr Leu Val His Thr Ala
        915                 920                 925

Met Asp Asn Pro Val Asp Leu Ile Ala Thr Asn Ile Thr Pro Thr Glu
    930                 935                 940

Ala Leu Leu Gln Trp Lys Ala Pro Val Gly Glu Val Glu Asn Tyr Val
945                 950                 955                 960

Ile Val Leu Thr His Phe Ala Val Ala Gly Glu Thr Ile Leu Val Asp
                965                 970                 975

Gly Val Ser Glu Glu Phe Arg Leu Val Asp Leu Leu Pro Ser Thr His
            980                 985                 990

Tyr Thr Ala Thr Met Tyr Ala Thr  Asn Gly Pro Leu Thr  Ser Gly Thr
        995                 1000                 1005

Ile Ser  Thr Asn Phe Ser Thr  Leu Leu Asp Pro Pro  Ala Asn Leu
    1010                 1015                 1020

Thr Ala  Ser Glu Val Thr Arg  Gln Ser Ala Leu Ile  Ser Trp Gln
    1025                 1030                 1035

Pro Pro  Arg Ala Glu Ile Glu  Asn Tyr Val Leu Thr  Tyr Lys Ser

1040                 1045                 1050

Thr Asp Gly Ser Arg Lys Glu Leu Ile Val Asp Ala Glu Asp Thr
    1055                 1060                 1065

Trp Ile Arg Leu Glu Gly Leu Leu Glu Asn Thr Asp Tyr Thr Val
    1070                 1075                 1080

Leu Leu Gln Ala Ala Gln Asp Thr Thr Trp Ser Ser Ile Thr Ser
    1085                 1090                 1095

Thr Ala Phe Thr Thr Gly Gly Arg Val Phe Pro His Pro Gln Asp
    1100                 1105                 1110

Cys Ala Gln His Leu Met Asn Gly Asp Thr Leu Ser Gly Val Tyr
    1115                 1120                 1125

Pro Ile Phe Leu Asn Gly Glu Leu Ser Gln Lys Leu Gln Val Tyr
    1130                 1135                 1140

Cys Asp Met Thr Thr Asp Gly Gly Gly Trp Ile Val Phe Gln Arg
    1145                 1150                 1155

Arg Gln Asn Gly Gln Thr Asp Phe Phe Arg Lys Trp Ala Asp Tyr
    1160                 1165                 1170

Arg Val Gly Phe Gly Asn Val Glu Asp Glu Phe Trp Leu Gly Leu
    1175                 1180                 1185

Asp Asn Ile His Arg Ile Thr Ser Gln Gly Arg Tyr Glu Leu Arg
    1190                 1195                 1200

Val Asp Met Arg Asp Gly Gln Glu Ala Ala Phe Ala Ser Tyr Asp
    1205                 1210                 1215

Arg Phe Ser Val Glu Asp Ser Arg Asn Leu Tyr Lys Leu Arg Ile
    1220                 1225                 1230

Gly Ser Tyr Asn Gly Thr Ala Gly Asp Ser Leu Ser Tyr His Gln
    1235                 1240                 1245

Gly Arg Pro Phe Ser Thr Glu Asp Arg Asp Asn Asp Val Ala Val
    1250                 1255                 1260

Thr Asn Cys Ala Met Ser Tyr Lys Gly Ala Trp Trp Tyr Lys Asn
    1265                 1270                 1275

Cys His  Arg Thr Asn Leu Asn  Gly Lys Tyr Gly Glu  Ser Arg His
    1280                 1285                 1290

Ser Gln  Gly Ile Asn Trp Tyr  His Trp Lys Gly His  Glu Phe Ser
    1295                 1300                 1305

Ile Pro  Phe Val Glu Met Lys  Met Arg Pro Tyr Asn  His Arg Leu
    1310                 1315                 1320

Met Ala  Gly Arg Lys Arg Gln  Ser Leu Gln Phe
    1325                 1330

<210>  9
<211>  1353
<212>  PRT
<213>  Gallus gallus

<400>  9

Met Gly Thr Asp Ser Glu Asn Pro Val Leu Arg Asn Val Leu Ile Ser
1               5                   10                  15

Phe Asn Leu Leu Leu Leu Gly Ala Val Leu Lys Pro Phe Glu Cys Arg
            20                  25                  30

Leu Glu Val Thr Thr Glu Pro Ala Glu Arg Pro Ala Val Asp Glu Glu
        35                  40                  45

Gly Gly Leu Ala Asn Cys Ser Pro Pro Val Lys Glu Gln Pro Met Val
    50                  55                  60

Phe His His Ile Tyr Asn Ile Asn Val Pro Val Asp Ser Cys Cys Ser
65                  70                  75                  80

Ser Met Leu Arg Ser Ser Ala Glu Glu Val Ser Ser Glu Asp Asp Arg
                85                  90                  95

Leu Ala Glu Tyr Thr Glu Gln Thr Ser Asp Ser Glu Ser Gln Val Thr
            100                 105                 110

Phe Thr His Arg Ile Asn Leu Pro Lys Gln Ala Cys Lys Cys Ser Thr
        115                 120                 125

Ser Leu Pro Ser Leu Gln Glu Leu Leu Ser Arg Ile Glu Met Leu Glu
    130                 135                 140

Arg Glu Val Ser Met Leu Arg Asp Gln Cys Asn Ser Asn Cys Cys Gln
145                 150                 155                 160

Glu Asn Ala Ala Thr Gly Arg Leu Asp Tyr Thr Leu Pro Cys Ser Gly
                165                 170                 175

His Gly Asn Phe Ser Leu Glu Ser Cys Arg Cys Ile Cys Ser Glu Gly
            180                 185                 190

Trp Ala Gly Ser Asn Cys Ser Glu Pro Arg Cys Pro Arg Gly Cys Ser
        195                 200                 205

Ser Arg Gly Val Cys Leu Glu Gly Gln Cys Val Cys Asp Asn Asp Tyr
    210                 215                 220

Gly Gly Glu Asp Cys Ser Gln Leu Arg Cys Pro Ala Gly Cys Gly Ser
225                 230                 235                 240

Arg Gly Leu Cys Val Asp Gly Glu Cys Ile Cys Glu Glu Gly Phe Gly
                245                 250                 255

Gly Glu Asp Cys Ser Gln Pro Arg Cys Pro Arg Asp Cys Ser Gly Arg
            260                 265                 270

Gly His Cys Asp Asn Gly Thr Cys Val Cys Ala Glu Gly Tyr Ala Gly
        275                 280                 285

Glu Asp Cys Gly Trp Leu Arg Cys Pro Asn Ala Cys Ser Gly Arg Gly
    290                 295                 300

Val Cys Gln Asp Gly Leu Cys Ile Cys Glu Asp Gly Tyr Gly Gly Gln
305                 310                 315                 320

Asp Cys Ser Ala Val Ala Pro Pro Glu Asn Leu Arg Val Thr Gly Ile
                325                 330                 335

Ser Asp Gly Ser Ile Glu Leu Ala Trp Asp Ser Leu Gly Ala Ala Thr
            340                 345                 350

Glu Tyr Val Val Ser Tyr Gln Pro Ala Gly Pro Gly Gly Ser Gln Leu
        355                 360                 365

Gln Gln Arg Val Pro Gly Asp Trp Ser Thr Ile Thr Ile Thr Glu Leu
    370                 375                 380

Glu Pro Gly Val Ala Tyr Asn Val Ser Ile Tyr Ala Val Ile Ser Asp
385                 390                 395                 400

Val Leu Ser Ser Pro Val Thr Thr Lys Val Thr Thr Asn Leu Ala Thr
                405                 410                 415

Pro Gln Gly Leu Lys Phe Lys Thr Ile Thr Glu Thr Thr Val Glu Val
            420                 425                 430

Gln Trp Glu Pro Phe Ser Phe Pro Phe Asp Gly Trp Glu Ile Ser Phe
        435                 440                 445

Ile Pro Lys Asn Asn Glu Gly Gly Val Ile Ala Gln Leu Pro Ser Thr
    450                 455                 460

Val Thr Thr Phe Asn Gln Thr Gly Leu Lys Pro Gly Glu Glu Tyr Thr
465                 470                 475                 480

Val Thr Val Val Ala Leu Lys Asp Gln Ala Arg Ser Pro Pro Ala Ser
                485                 490                 495

Asp Ser Ile Ser Thr Leu Ile Asp Gly Pro Thr Gln Ile Leu Val Arg
            500                 505                 510

Asp Val Ser Asp Thr Val Ala Phe Val Glu Trp Thr Pro Pro Arg Ala
        515                 520                 525

Arg Val Asp Ala Ile Leu Leu Lys Tyr Gly Leu Ala Asp Gly Glu Gly
    530                 535                 540

Gly Arg Thr Thr Phe Arg Leu Gln Pro Pro Leu Ser Gln Tyr Ser Leu
545                 550                 555                 560

Gln Ala Leu Arg Pro Gly Ala Arg Tyr His Leu Ala Val Ser Ala Leu
                565                 570                 575

Arg Gly Ala Asn Glu Ser Gln Pro Ala Leu Ala Gln Phe Thr Thr Glu
            580                 585                 590

Ile Asp Ala Pro Lys Asn Leu Arg Val Gly Ser Arg Thr Pro Ala Ser
        595                 600                 605

Leu Glu Leu Thr Trp Asp Asn Ser Glu Ala Glu Ala His Ser Tyr Arg
    610                 615                 620

Val Val Tyr Ser Thr Leu Ala Gly Glu His Tyr His Glu Val Leu Val
625                 630                 635                 640

Pro Arg Asp Thr Gly Pro Thr Thr Arg Ala Thr Leu Ala Asp Leu Val
                645                 650                 655

Pro Gly Thr Glu Tyr Gly Ile Gly Ile Ser Ala Val Met Asp Ser Gln
            660                 665                 670

Gln Ser Val Pro Ala Thr Met Asn Ala Arg Thr Glu Leu Asp Ser Pro
        675                 680                 685

Arg Asp Leu Leu Val Thr Ala Ser Thr Glu Thr Ser Ile Ser Leu Ser
    690                 695                 700

Trp Thr Lys Ala Met Gly Pro Ile Asp His Tyr Arg Val Thr Phe Thr
705                 710                 715                 720

Pro Ala Ser Gly Met Ala Ser Glu Val Thr Val Ser Arg Asn Glu Ser
                725                 730                 735

Gln Leu Thr Leu Ser Glu Leu Glu Pro Gly Thr Glu Tyr Thr Ile Ser
            740                 745                 750

Ile Ile Ala Glu Arg Gly Arg Gln Gln Ser Leu Glu Ala Thr Val Asp
        755                 760                 765

Ala Phe Thr Gly Val Arg Pro Ile Thr Gln Leu His Phe Ser Gln Leu
    770                 775                 780

Thr Ser Ser Ser Val Asn Ile Thr Trp Ser Asp Pro Ser Pro Pro Ala
785                 790                 795                 800

Asp Arg Leu Val Leu Thr Tyr Ser Pro Arg Asp Glu Glu Ala Pro Gln
                805                 810                 815

Gln Leu Ala Leu Asp Gly Thr Arg Arg His Ala Ser Leu Thr Gly Leu
            820                 825                 830

Arg Pro Ser Thr Glu Tyr Leu Val Ser Leu Val Ala Val His Gly Ala
        835                 840                 845

Val Ser Ser Glu Pro Val Thr Gly Ser Ile Thr Thr Gly Met Asp Ala
    850                 855                 860

Pro Lys Asp Leu Arg Val Gly Asn Ile Thr Gln Asp Ser Met Val Ile
865                 870                 875                 880

Tyr Trp Ser Pro Pro Val Ala Pro Phe Asp His Tyr Arg Ile Ser Tyr
                885                 890                 895

Arg Ala Ala Glu Gly Arg Thr Asp Ser Thr Ala Ile Gly Asn Asp Ala
            900                 905                 910

Thr Glu Tyr Ile Met Arg Leu Leu Gln Pro Ala Thr Lys Tyr Glu Ile

        915                 920                 925

Gly Val Lys Ser Val Arg Gly Arg Glu Glu Ser Glu Val Ala Ser Ile
    930                 935                 940

Thr Thr Tyr Thr Ala Met Asp Ala Pro Leu Gly Val Thr Ala Thr Asn
945                 950                 955                 960

Ile Thr Pro Thr Glu Ala Leu Leu Gln Trp Asn Pro Pro Leu Met Asp
                965                 970                 975

Val Glu Ser Tyr Val Leu Val Leu Thr Arg His Thr Gly Glu Thr Ile
            980                 985                 990

Leu Val Asp Gly Ile Asn Gln Glu Tyr Gln Leu Thr Asn Leu Gln Pro
        995                 1000                1005

Ser Thr Thr Tyr Thr Val Ala Met Tyr Ala Thr Asn Gly Pro Leu
    1010                1015                1020

Thr Ser Gln Thr Ile Ser Thr Asn Phe Thr Thr Leu Leu Asp Pro
    1025                1030                1035

Pro Thr Asn Leu Thr Ala Ser Glu Val Thr Arg Arg Ser Ala Leu
    1040                1045                1050

Leu Ser Trp Val Pro Pro Val Gly Asp Ile Glu Asn Tyr Ile Leu
    1055                1060                1065

Thr Tyr Arg Ser Thr Asp Gly Ser Arg Lys Glu Leu Ile Val Asp
    1070                1075                1080

Ala Glu Asp Thr Trp Ile Arg Leu Glu Gly Leu Ser Glu Thr Thr
    1085                1090                1095

Gln Tyr Thr Val Arg Leu Gln Ala Ala Gln Asn Ala Met Arg Ser
    1100                1105                1110

Gly Phe Ile Ser Thr Thr Phe Thr Thr Gly Gly Arg Val Phe Ala
    1115                1120                1125

Asn Pro Gln Asp Cys Ala Gln His Leu Met Asn Gly Asp Thr Leu
    1130                1135                1140

Ser Gly Val Tyr Thr Ile Ser Ile Asn Gly Asp Leu Ser Gln Arg
    1145                1150                1155

Val Gln Val Phe Cys Asp Met Ser Thr Asp Gly Gly Gly Trp Ile
    1160                1165                1170

Val Phe Gln Arg Arg Gln Asn Gly Leu Thr Asp Phe Phe Arg Lys
    1175                1180                1185

Trp Ala Asp Tyr Arg Val Gly Phe Gly Asn Leu Glu Asp Glu Phe
    1190                1195                1200

Trp Leu Gly Leu Asp Asn Ile His Lys Ile Thr Ser Gln Gly Arg
    1205                1210                1215

Tyr Glu Leu Arg Ile Asp Met Arg Asp Gly Gln Glu Ala Ala Tyr
    1220                1225                1230

Ala Tyr Tyr Asp Lys Phe Ser Val Gly Asp Ser Arg Ser Leu Tyr
    1235                1240                1245

Lys Leu Arg Ile Gly Asp Tyr Asn Gly Thr Ser Gly Asp Ser Leu
    1250                1255                1260

Thr Tyr His Gln Gly Arg Pro Phe Ser Thr Lys Asp Arg Asp Asn
    1265                1270                1275

Asp Val Ala Val Thr Asn Cys Ala Met Ser Tyr Lys Gly Ala Trp
    1280                1285                1290

Trp Tyr Lys Asn Cys His Arg Thr Asn Leu Asn Gly Lys Tyr Gly
    1295                1300                1305

Glu Ser Arg His Ser Gln Gly Ile Asn Trp Tyr His Trp Lys Gly
    1310                1315                1320

His Glu Phe Ser Ile Pro Phe Val Glu Met Lys Met Arg Pro Tyr
    1325                1330                1335

Asn His Arg Asn Ile Ser Gly Arg Lys Arg Arg Ser Leu Gln Leu
    1340                1345                1350

<210>  10
<211>  1350
<212>  PRT
<213>  Danio rerio

<400>  10

Met Trp Gly Cys Ala Met Ala Ile Gln Gly Ser Val Val Ser Leu Ala
1               5                   10                  15

Leu Leu Phe Phe Gly Ile Gln Ala Val Pro Ser Pro Thr Ser Lys Leu
            20                  25                  30

Val Arg Thr Thr Arg Val Arg Arg Gln Val Pro Glu Gly Gly Asp Pro
        35                  40                  45

Ala Asp Asn Gln Met Gln Pro Met Val Phe Asn His Val Tyr Asn Ile
    50                  55                  60

Asn Val Pro Val Glu Ser Leu Cys Ser Val Asp Leu Asp Ala Val Thr
65                  70                  75                  80

Gly Pro Glu Thr Asn Lys Thr Ala Ser Gly Ser Asp Lys Met Pro Thr
                85                  90                  95

Glu Tyr Thr Glu Glu Thr Val Asp Ser Asp Ser Gln Val Thr Phe Thr
            100                 105                 110

His Arg Ile Asn Ile Pro Lys Gln Ala Cys Ala Cys Pro Ser Ala Thr
        115                 120                 125

Thr Ile Glu Gln Leu Ala Ser Arg Ile Glu Met Leu Glu Arg Glu Val
    130                 135                 140

Ser Leu Leu Arg Thr Gln Cys Ser Ser Ser Cys Cys Gly Glu Ser Ser
145                 150                 155                 160

Val Met Gly Arg Leu Asp Phe Val Pro Gln Cys Gly His Gly Thr Phe
                165                 170                 175

Ser Met Glu Val Cys Gly Cys Val Cys Glu Glu Gly Trp Ile Gly Lys
            180                 185                 190

Asn Cys Thr Glu Pro Arg Cys Pro Asp Asp Cys Ser Gly Gln Gly Ile
        195                 200                 205

Cys Ile Glu Gly Asp Cys Val Cys Asp Arg Asn Phe Gly Gly Glu Asn
    210                 215                 220

Cys Ser Glu Pro Arg Cys Pro Ser Asp Cys Ser Asp Arg Gly Leu Cys
225                 230                 235                 240

Ile Asp Gly Glu Cys Val Cys Glu Glu Ala Phe Ala Gly Glu Asp Cys
                245                 250                 255

Ser Leu Gly Arg Cys Leu Asn Asp Cys Ser Asp Gln Gly Ala Cys Val
            260                 265                 270

Asn Gly Ser Cys Gln Cys Arg Ser Gly Phe Leu Gly Glu Asp Cys Ser
        275                 280                 285

Leu Ile Phe Cys Ala Asn Asn Cys Ser Gln Arg Gly Val Cys Lys Glu
    290                 295                 300

Gly Phe Cys Val Cys Gln Glu Gly Tyr Thr Gly Asp Asp Cys Thr Ser
305                 310                 315                 320

Val Leu Pro Pro Met Asn Leu Arg Val Arg Gly Val Ser Glu Asn Thr
                325                 330                 335

Ile Asp Leu Gln Trp Glu Gly Pro Ala Ile Leu Thr Asp Tyr Leu Ile
            340                 345                 350

Thr Tyr Ala Pro Thr Thr Pro Gly Gly Val Gln Leu Glu Met Arg Val
        355                 360                 365

Pro Gly Asn Val Thr Asn Ile Thr Ile Lys Gly Leu Asn Pro Gly Leu
    370                 375                 380

Glu Tyr Asn Val Asn Val Tyr Ala Val Ile Asp Asn Ile Ile Ser Ser
385                 390                 395                 400

Pro Met Asn Thr Leu Val Ser Thr Tyr Leu Ser Asn Pro Asp Gly Leu
                405                 410                 415

Leu Phe Lys Ser Ile Met Glu Thr Ser Val Glu Val Gln Trp Gln Pro
            420                 425                 430

Leu Asp Tyr Leu Phe Asp Gly Trp Glu Ile Ser Phe Ile Pro Lys Asp
        435                 440                 445

Asn Asp Gly Gly Met Thr Ala Gln Leu Pro Ser Thr Ile Thr Ser Phe
    450                 455                 460

His Gln Thr Gly Leu Lys Pro Gly Glu Glu Tyr Thr Val Asn Leu Val
465                 470                 475                 480

Ala Leu Lys Asp Gln Gly Arg Ser Leu Pro Val Thr Ala Thr Val Thr
                485                 490                 495

Thr Leu Ile Asp Gly Pro Ile Gln Leu Ile Val Arg Asp Ile Ser Asp
            500                 505                 510

Thr Val Ala Phe Val Glu Trp Ser Pro Pro Lys Ala Lys Val Asp Gln
        515                 520                 525

Ile Ile Leu Arg Tyr Gly Leu Val Gly Val Glu Gly Pro Lys Thr Thr
    530                 535                 540

Phe Arg Leu Gln Pro Thr Leu Ser Gln Tyr Ser Leu Gln Val Leu Arg
545                 550                 555                 560

Pro Gly Ser Thr Tyr Glu Val Ser Val Ser Gly Val Arg Asn Gly Thr
                565                 570                 575

Glu Ser Gly Val Ile Ser Thr Glu Phe Thr Thr Glu Ile Asp Ala Pro
            580                 585                 590

Lys Asn Leu Arg Val Leu Ser Lys Thr Ser Ile Ser Leu Glu Leu Glu
        595                 600                 605

Trp Asp Asn Ser Glu Ala Asp Val Glu Gly Tyr Ser Val Val Tyr Ser
    610                 615                 620

Thr Leu Ala Gly Asp Gln Tyr Asp Lys Val Ile Val Pro Arg Asn Asp
625                 630                 635                 640

Gly Pro Ser Thr Lys Thr Thr Leu Asn Glu Leu Met Pro Gly Thr Glu
                645                 650                 655

Tyr Gly Ile Gly Ile Ser Ala Val Leu Gly Ala Asn Gln Ser Thr Pro
            660                 665                 670

Ala Thr Met Asn Ala Arg Thr Gly Leu Asp Val Pro Leu Asp Leu Thr
        675                 680                 685

Val Thr Ala Ser Thr Asp Asn Thr Ile Thr Leu Leu Trp Gly Thr Val
    690                 695                 700

Gln Gly Pro Ile Asp His Tyr Arg Val Thr Tyr Thr Ser Ser Ser Gly
705                 710                 715                 720

Val Thr Thr Glu Leu Thr Val Pro Lys Asp Val Thr Thr Thr Thr Leu
                725                 730                 735

Thr Ser Leu Glu Pro Gly Thr Lys Tyr Thr Ile Thr Val Ala Ala Gln
            740                 745                 750

Arg Gly Arg Gln Gln Ser Thr Ala Ala Thr Ile Asp Ala Phe Thr Gly
        755                 760                 765

Phe Arg Pro Ile Thr Gln Leu Phe Phe Ser Glu Val Ser Ser Asp Ser

770                 775                 780

Leu Thr Val Ala Trp Ser Ser Pro Ala Pro Pro Ala Asp Ala Phe Ile
785                 790                 795                 800

Leu Asn Tyr Ser Ala Gln Asp Ser Ser Glu Asp Ser Glu Ile Ala Leu
                805                 810                 815

Asp Gly Ser Lys Thr Arg Ile Thr Leu Thr Gly Leu Met Pro Ser Arg
            820                 825                 830

Arg Tyr Thr Val Thr Leu Val Thr Met His Gly Asn Val Thr Ser Lys
        835                 840                 845

Pro Val Val Gly Ser Val Asn Thr Gly Met Asp Pro Pro Arg Asp Ile
    850                 855                 860

Thr Val Leu Tyr Val Thr Glu Glu Ser Val Thr Ile Thr Trp Ile Gln
865                 870                 875                 880

Pro Leu Ala Leu Phe Asp Tyr Tyr Arg Met Ser Tyr Gln Ser Ser Lys
                885                 890                 895

Gly Arg Met Asp Ser Val Val Ile Asp Asn Asp Ile Asn Asn Tyr Thr
            900                 905                 910

Leu Thr Ser Leu His Pro Ala Thr Glu Tyr Glu Ile Lys Leu Asn Ala
        915                 920                 925

Val Arg Gly Ser Gln Glu Ser Lys Val Ile Thr Thr Thr Val Phe Thr
    930                 935                 940

Ala Met Asp Met Pro Ala Glu Leu Thr Ala Leu Asn Val Thr Pro Gln
945                 950                 955                 960

Gly Ala Leu Leu Arg Trp Asn Pro Pro Ala Ser Ser Val Asp Asn Tyr
                965                 970                 975

Val Leu Thr Val Thr Arg Asn Gln Val Thr Ala Asp Thr Phe Leu Val
            980                 985                 990

Glu Gly Asn Lys Gln Glu Tyr Gln  Leu Ser Gln Leu Ile  Pro Ser Thr
        995                 1000                1005

Thr Tyr  Ser Val Ala Leu Tyr  Ala Thr Lys Gly Pro  Leu Thr Ser
    1010                1015                1020

Gly Thr Val Ile Ser Asn Phe Val Thr Pro Met Asp Ala Pro Gln
    1025                1030                1035

Asn Leu Thr Ala Ser Glu Ile Asn His Arg Ser Ala Leu Ile Ser
    1040                1045                1050

Trp Gln Pro Pro Ile Ala Asp Ile Asp Asn Tyr Met Leu Thr Tyr
    1055                1060                1065

Lys Ala Ala Asp Gly Ser Arg Lys Glu Leu Ile Leu Asp Ala Glu
    1070                1075                1080

Asp Thr Trp Ile Arg Leu Glu Gly Leu Ala Glu Thr Thr Glu Tyr
    1085                1090                1095

Thr Val Arg Leu Gln Ala Ala Arg Gly Leu Glu Thr Ser Ala Ile
    1100                1105                1110

Val Ser Thr Ser Phe Thr Thr Gly Asn Arg Leu Phe Pro Thr Pro
    1115                1120                1125

Gln Asn Cys Ala Gln His Leu Leu Asn Gly Glu Thr Leu Gly Gly
    1130                1135                1140

Ile Tyr Thr Ile Tyr Val Asn Arg Asp Leu Ser Gln Gly Val Gln
    1145                1150                1155

Val Tyr Cys Asp Met Thr Thr Asp Gly Gly Gly Trp Ile Val Phe
    1160                1165                1170

Gln Arg Arg Gln Asn Gly Leu Thr Asp Phe Ser Arg Lys Trp Thr
    1175                1180                1185

Asp Tyr Lys Ile Gly Phe Gly Asn Leu Glu Asp Glu Phe Trp Leu
    1190                1195                1200

Gly Leu Asp Asn Ile His Lys Ile Ala Ala Gln Gly Arg Tyr Glu
    1205                1210                1215

Leu Arg Ile Asp Met Lys Asp Gly Gln Glu Ser Val Tyr Ala Asn
    1220                1225                1230

Tyr Asp Arg Phe Ser Ile Gly Asp Ser Lys Ser Leu Tyr Lys Leu
    1235                1240                1245

Arg Ile Gly Glu Tyr Asn Gly Thr Ala Gly Asp Ser Leu Ser Tyr
    1250                1255                1260

His Gln Ser Arg Pro Phe Ser Thr Lys Asp Lys Asp Asn Asp Ile
    1265                1270                1275

Ala Val Thr Asn Cys Ala Leu Ser Tyr Lys Gly Ala Trp Trp Tyr
    1280                1285                1290

Lys Asn Cys His Arg Ala Asn Leu Asn Gly Lys Tyr Gly Glu Ser
    1295                1300                1305

Arg His Ser Gln Gly Ile Asn Trp Tyr His Trp Lys Gly His Glu
    1310                1315                1320

Phe Ser Ile Pro Phe Val Glu Met Lys Met Arg Pro Phe Asn Tyr
    1325                1330                1335

Arg Ser Ile Ser Gly Lys Arg Arg Arg Ser Thr His
    1340                1345                1350